# FASCICULE DE BREVET EUROPEEN

(11) **EP 2 142 644 B1**
(45) Date de publication et mention de la délivrance du brevet: **20.11.2013**
(21) Numéro de dépôt: 08805511.6
(22) Date de dépôt: 25.04.2008
(51) Int. Cl.: C12N 9/14, C07K 14/195, A61K 38/46

(54) **PHOSPHOTRIESTERASES HYPERTHERMOPHILES MUTEES ET LEURS UTILISATIONS**
MUTIERTE HYPERTHERMOPHILE PHOSPHOTRIESTERASEN UND ANWENDUNGEN DAVON
MUTATED HYPERTHERMOPHILIC PHOSPHOTRIESTERASES AND USES THEREOF

(30) Priorité: 27.04.2007 FR 0703104
(43) Date de publication de la demande: 13.01.2010
(73) Titulaire: Université de Lorraine, 54052 Nancy Cedex (FR); Centre National de la Recherche Scientifique, 75794 Paris Cedex 16 (FR)
(72) Inventeur: CHABRIERE, Eric, F-13009 Marseilles (FR); ELIAS, Mickael, F-13009 Marseille (FR)
(74) Mandataire: Grosset-Fournier, Chantal Catherine
(86) Numéro de dépôt international: PCT/FR2008/000596
(87) Numéro de publication internationale: WO 2008/145865

(56) Documents cités:
- WO-A-2005/059125
- ELIAS MIKAEL ET AL: "Crystallization and preliminary X-ray diffraction analysis of the hyperthermophilic Sulfolobus solfataricus phosphotriesterase." ACTA CRYSTALLOGRAPHICA. SECTION F, STRUCTURAL BIOLOGY AND CRYSTALLIZATION COMMUNICATIONS 1 JUL 2007, vol. 63, no. Pt 7, 1 juillet 2007 (2007-07-01), pages 553-555, XP009089597 ISSN: 1744-3091
- PORZIO ET AL: "A new phosphotriesterase from Sulfolobus acidocaldarius and its comparison with the homologue from Sulfolobus solfataricus" BIOCHIMIE, MASSON, PARIS, FR, vol. 89, no. 5, 27 janvier 2007 (2007-01-27), pages 625-636, XP022081069 ISSN: 0300-9084
- MERONE LUIGIA ET AL: "A thermostable phosphotriesterase from the archaeon Sulfolobus solfataricus: cloning, overexpression and properties" EXTREMOPHILES, vol. 9, no. 4, août 2005 (2005-08), pages 297-305, XP002451459 ISSN: 1431-0651

## Description

La présente description a pour objet des phosphotriestérases (PTE) hyperthermophiles mutées, et leurs utilisations en tant que bio-épurateurs dans le cadre de la décontamination des surfaces des matériaux, de la peau, ou des muqueuses, contaminés par des composés organophosphorés, ou dans le cadre de la préparation de vêtements, gants, cartouches, capteurs d'organophosphorés, ou dans le cadre de la préparation de médicaments utilisables dans le cadre de la prévention ou du traitement d'une contamination externe ou d'une intoxication interne par ingestion ou inhalation par des composés organophosphorés, ou dans le cadre de la dépollution des eaux polluées par des composés organophosphorés, ainsi que les séquences nucléotidiques codant pour ces PTE hyperthermophiles mutées et leur utilisation dans le cadre de la préparation de bactéries exprimant ces PTE, notamment à leur surface.

### ORGANOPHOSPHORES

Les organophosphorés (OPs) sont des molécules très toxiques, qui composent certains agents chimiques de guerre et pesticides. Certains de ces composés comme le paraoxon ou le parathion sont utilisés pour leur pouvoir insecticide. En effet, ils sont faciles à fabriquer et sont largement répandus dans les cultures des pays en voie de développement. Malheureusement, ces épandages massifs sont responsables de très nombreuses intoxications dans le monde (200000 décès par an selon l'OMS).

Les OPs sont pour la plupart des produits instables car ils s'hydrolysent rapidement. Ils ne persistent donc pas dans l'environnement sous leur forme toxique. Par contre, certains produits développés par les armées sont beaucoup plus stables et dangereux, comme le sarin, le soman, le tabun ou le VX. Ces agents chimiques de guerre intéressent maintenant de plus en plus les terroristes. Le sarin notamment a déjà été utilisé lors d'attentats perpétrés par la secte Aum, en 1994 à Matsumoto et en 1995 dans le métro de Tokyo. Face à ces menaces grandissantes, l'étude et surtout la mise au point de moyens performants de décontamination est plus que jamais d'actualité.

Les organophosphorés agissent par absorption percutanée et par inhalation. Ce sont des liquides très souvent incolores et inodores. L'intoxication par l'un de ces agents se manifeste rapidement (moins de 1 minute à 60 minutes) par des symptômes caractéristiques et extrêmement graves (jusqu'à la mort du sujet intoxiqué). Ces molécules, une fois ingérés dans l'organisme humain, jouent le rôle de neurotoxique. Elles s'attaquent à une enzyme très importante pour le bon fonctionnement du système nerveux : l'acétylcholinestérase. Cette enzyme est essentielle dans la transmission du message nerveux. En effet, lors du passage de l'influx de neurone à neurone, l'information électrique est transformée en message chimique dans la fente synaptique. Les molécules ainsi déversées sont appelées neurotransmetteur (exemple : l'acétylcholine). Une fois libérée dans la fente, l'acétylcholine se fixe majoritairement sur les récepteurs du neurone post-synaptique afin d'assurer la continuité du message nerveux. Les molécules fixées et non fixées doivent ensuite être recaptées ou dégradées, permettant ainsi la régulation de l'intensité et de la durée de l'influx. Le rôle des acétylcholinestérases est donc d'assurer l'arrêt du message nerveux, en dégradant l'acétylcholine dans la fente synaptique.

Les OPs réagissent rapidement avec la sérine du site actif des acétylcholinestérases, ce qui forme une phosphoenzyme inactive. L'intermédiaire covalent ainsi formé, l'enzyme a perdu toute activité. Ces composés constituent donc des inhibiteurs irréversibles de ces enzymes. L'acétylcholine n'est alors plus dégradée dans la fente synaptique et s'accumule.

Pour se prémunir de ces dangers, des protocoles de prévention et de décontamination sont prévus. Le matériel est, à l'heure actuelle, décontaminé à l'aide de soude (NaOH) très concentrée. Des tenues de protection et des masques ont été conçus pour éviter tout contact avec ces agents. En cas d'intoxication humaine, un traitement à la soude est bien évidemment inenvisageable. La victime est simplement décontaminée à l'aide d'une solution d'hypochlorite (eau de Javel) et lavée abondamment au savon et à l'eau. Des gants de Foulon permettent aussi d'absorber le liquide sur la peau de la victime. Pour les cas d'inhalation (percutanée ou non) de neurotoxiques, il existe un prétraitement à la pyrostigmine, pouvant être pris en cachet. Cette molécule bloque réversiblement les acétylcholinestérases et empêche les OPs de s'y fixer. La vie de l'individu est ainsi préservée. Par ailleurs, il existe aussi un traitement symptomatique d'urgence sous forme de seringues auto-injectantes contenant de l'atropine (anticholinergique), du diazépam (anticonvulsifiant) et de la pralidoxime (réactivateur des acétylcholinestérases inhibées). L'injection doit cependant être faite immédiatement après l'intoxication pour être efficace. Ceci n'empêche toutefois pas l'apparition de séquelles incapacitantes.

Si des progrès ont été accomplis depuis vingt ans dans la prophylaxie avec les techniques citées précédemment, les traitements de ces intoxications et les protections existantes restent néanmoins insatisfaisants. Toutes les pistes pharmacologiques explorées semblent malheureusement conduire à une impasse. Cependant, l'émergence du concept d'épurateur biocatalytique ou « bioscavenger » a ranimé l'espoir d'un arsenal thérapeutique plus performant. En effet, l'idée d'utiliser des enzymes capables de piéger et/ou de dégrader les OPs sur la peau et dans le sang avant qu'ils n'atteignent leurs cibles biologiques neuromusculaires et centrales est particulièrement séduisante.

La ButyrylCholinestérase (BuChe) humaine est une enzyme semblable à l'acétylcholinestérase dont le rôle physiologique n'est pas clairement établi. Malgré cela, elle représente un grand espoir car elle piège dans la voie sanguine les organophosphorés avant qu'ils n'atteignent leurs cibles (Raveh et al.,1993). De plus, l'enzyme naturelle injectée à l'homme est particulièrement stable, avec une demi-vie de 11 jours. Toutefois, ce piégeur naturel est en quantité bien trop faible dans le sang pour nous protéger naturellement des dangers des OPs. Elle agit en fait comme un fixateur stoechiométrique des OPs : une enzyme ne peut neutraliser qu'une seule molécule. Un rapide calcul permet de montrer qu'il faut des quantités énormes d'enzyme pour obtenir un traitement efficace. Les moyens à employer semblent alors disproportionnés, et correspondraient à une dose de 200mg de protéine par injection et par soldat. Toutefois, et à défaut de mieux, la BuChE représente un projet concret notamment pour l'armée américaine qui a prévue la mise à disposition fin 2006 d'un million de doses pour ses soldats. La production de l'enzyme est assurée par génie génétique grâce à des chèvres transgéniques. La nécessité d'une telle quantité de protéine coûte néanmoins très cher, et malgré les moyens mis en oeuvre, cette entreprise constitue un challenge technologique majeur. Il existe bien quelques variants de BuChE ayant une activité OPhydrolasique mais leur catalyse est très lente en comparaison des enzymes capables d'hydrolyser naturellement les OPs.

La Paraoxonase humaine (HPON1) est une OPhydrolase qui présente de nombreux avantages. Son rôle protecteur contre l'intoxication OP a été établi chez la souris. De plus, son origine humaine devrait éviter que les injections multiples des protocoles thérapeutiques n'induisent de réponse immune. HPON1 est une protéine du plasma principalement associée aux HDL. La structure tridimensionnelle de l'enzyme naturelle n'est pas résolue, seulement la structure d'une chimère entre PON1 humaine, de souris, de rat et de lapin, (Harel et al., 2004). Néanmoins, cette structure n'a pas permis d'obtenir des mutants plus actifs. De plus, une utilisation pharmacologique est dans l'immédiat impossible. En effet, toutes les tentatives pour obtenir de grande quantité de paraoxonase humaine active ont échouées pour des raisons techniques.

D'autres OP-hydrolases prometteuses ont été isolées. Il s'agit des enzymes de la famille des phosphotriestérases (PTEs). Ces enzymes constituent de véritables épurateurs catalytiques découvert dans les bactéries du sol : notamment *Pseudomonas diminuta* et *flavobacterium sp.*(Munnecke, 1976) pour le gène opd, et *Agrobacterium radiobacter* pour le gène opdA (Jackson et al., 2005). Les PTEs sont des enzymes extrêmement prometteuses pour la mise au point d'un bioscavenger d'agents neurotoxiques. Mais ces enzymes possèdent aussi des intérêts fondamentaux : en effet, l'(les) implication(s) biologique(s) de ces dernières reste(nt) parfaitement inconnue(s). De plus, le mécanisme catalytique de ces enzymes extrêmement efficace possède quelques zones d'ombres.

Les PTEs sont les plus actives des enzymes connues dégradant les OPs. Leurs études pourraient permettre de réaliser des traitements pour des décontaminations thérapeutiques (cutanées et ophtalmologiques) qui remplaceraient avantageusement le seul moyen efficace actuel qui est la soude. Cette dernière n'est bien évidemment pas utilisable sur des êtres vivants. Par ailleurs, les PTEs seraient aussi efficaces pour décontaminer des sols pollués par les pesticides. On pourrait également les utiliser pour détecter des pollutions aux OPs. Ainsi, des projets tentent de fixer chimiquement ces protéines à un support et de détecter la catalyse éventuelle par divers moyens comme la détection de signaux électriques ou par spectrophotométrie. Autre atout majeur, les PTEs sont capables d'hydrolyser un large spectre d'OPs, comme le parathion, le paraoxon, le soman, le sarin et le plus toxique de tous, le VX.

Les hypothèses concernant l'origine de cette activité OP-hydrolase chez les bactéries sont multiples et controversées, même s'il semble plus vraisemblable que cette activité découle d'une proximité structurale de son substrat naturel avec ces poisons. En outre, le rôle physiologique de ces enzymes reste inconnu (Aubert et al., 2004). Il existe plusieurs gènes connus codant pour des PTEs mésophiles. Un premier gène (*opd*) a été isolé simultanément de *P. diminuta* et de *Flavobacterium sp.,* et code une protéine de 365 acides aminées. Cette protéine possède un peptide signal de 29 résidus permettant son adressage dans l'espace périplasmique. Un autre gène connu (*opda*), isolé d'*A*. *radiobacter* (Jackson et al., 2005), code une protéine de 362 acides aminés possédant un peptide signal de 33 résidus. Ces deux protéines partagent 90% d'identité de séquence. Si ces PTEs mésophiles sont très actives vis-à-vis des OPs, elles sont cependant chères à produire, et peu stables.

Récemment, une nouvelle protéine de cette famille a été isolée et purifiée (Merone et al., 2005). Cette métalloenzyme de 35,5 kDa, possède 31% d'identité de séquence avec la PTE de *P. diminuta,* a été isolée de l'*archae Sulfolobus solfataricus.* Cet organisme vit dans des conditions extrêmes (87°-93°C et pH 3.5-5). Ces dernières confèrent à cette protéine des propriétés de thermostabilité exceptionnelles. C'est une enzyme hyperthermophile dont l'activité maximale se situe vers 95°C, et qui est nettement moins active vis-à-vis du paraoxon que la PTE de *P. diminuta.* Une autre PTE hyperthermophile a été isolée de *Sulfolobus acidocaldarius* (Porzio et al., 2007). Les PTEs hyperthermophiles sont moins actives vis-à-vis des OPs que les PTEs mésophiles, mais présentent en revanche l'avantage d'être très stables et peu chères à produire.

### INFECTIONS BACTERIENNES

Les infections bactériennes constituent l'une des causes majeures des pathologies humaines. Certaines de ces infections peuvent être contractées à l'hôpital et constituent un problème majeur de santé publique. En France, selon les différentes études menées, environ 5 à 10% des personnes hospitalisées sont victimes lors de leur séjour à l'hôpital, soit 600000 à 1000000 de patients par an. En se surajoutant aux pathologies initialement responsables de l'hospitalisation, ces infections aggravent le pronostic vital des patients (environ 6000 décès par an, dixième cause de décès en France). A cette réalité s'ajoute un surcoût financier à la prolongation des durées de séjour à l'hôpital et à la mise en place de traitements onéreux. Ces problèmes sont encore amplifiés par l'apparition d'un nombre croissant de résistances aux antibiotiques. Un certain nombre de stratégies sont développées pour acquérir de nouveaux outils contre ces résistances. Une des pistes les plus prometteuses consiste à perturber les communications entre bactéries. En effet, bien que les bactéries soient des organismes unicellulaires, elles sont capables de communiquer entre elles et ainsi de répondre collectivement à un changement environnemental. Ces mécanismes de communication, appelés « quorum sensing » (QS), permettent la synchronisation et les modulations de l'expression de certains gènes (Federle and Bassler, 2003; Fuqua and Greenberg, 2002; Whitehead et al., 2001). Cette communication est modulée par de petites molécules « signal », capables de diffuser librement à travers les membranes des cellules et d'aller réguler les profils d'expression des gènes. Par ailleurs, le phénomène du QS n'est pas limité aux procaryotes, puisque certains pathogènes eucaryotes unicellulaires d'algues utilisent aussi le QS pour coordonner certaines fonctions biologiques, comme la virulence (Oh et al., 2001).

De tous les signaux utilisés pour le QS, les acylhomoserine lactones (AHLs) semblent les plus répandues (notamment chez les bactéries Gram-négatives) et sont les plus étudiées.

Leur implication est démontrée dans de nombreuses fonctions biologiques importantes, comme la symbiose, la conjugaison, la production d'antibiotiques, la sporulation, la virulence et la formation de biofilm (Fuqua and Greenberg, 2002; Whitehead et al., 2001; Zhang, 2003).

La concentration de ces molécules « signal » est très importante et régulée, en partie par des enzymes capables de dégrader ces composés. On trouve en particulier des AHL acylases et AHL lactonases qui sont capables de dégrader ces lactones, comme AiiA, issue de Bacillus thuringiensis (Dong et al., 2002). Pour lutter contre les infections bactériennes, l'idée de perturber le quorum sensing est une piste extrêmement prometteuse (Rasmussen and Givskov, 2006). En effet, étant donné que des pathogènes mutants pour le QS n'exprime plus de gènes de virulence et deviennent non virulent (Passador et al., 1993; Pirhonen et al., 1993), il semble donc possible de contrôler les infections bactériennes en atténuant le QS des pathogènes.

Ainsi, l'expression d'une enzyme atténuant le QS : une enzyme de « quorum quenching » (QQ), que ce soit une AHL lactonase ou une AHL acylase, dans des pathogènes de plante ou d'humain comme Erwinia carotovora et Pseudomonas aeruginosa, réduit de façon importante leur virulence (Dong et al., 2000; Lin et al., 2003; Reimmann et al., 2002). De plus, des plants transgéniques exprimant une QQ lactonase sont effectivement résistants aux infections de pathogènes (Dong et al., 2001).

Récemment, la protéine SsoPox, issue de l'archae hyperthermophille Sulfolobus solfataricus a été clonée et caractérisée pour son activité phosphotriesterase (Merone et al., 2005). Cette protéine est hyperthermostable avec une demi-vie de dénaturation d'environ 4 heures et 90 à 95 et 100°C, respectivement. Ceci permet une purification très efficace et à bas coût de la protéine recombinante en chauffant les lysats cellulaires, et en précipitant ainsi les protéines de l'hôte (Escherichia coli). En 2006, il a été montré que SsoPox possède une grande activité AHL lactonase (Afriat et al., 2006).

La présente invention a essentiellement pour but de fournir de nouvelles PTEs présentant l'avantage d'être à la fois :
- plus actives vis-à-vis des OPs que les PTEs hyperthermophiles sauvages susmentionnées,
- plus stables et moins chères à produire que les PTEs mésophiles sauvages susmentionnées.

L'invention a également pour but de fournir de nouveaux composés bio-épurateurs utilisables dans le cadre de la décontamination des surfaces des matériaux, de la peau, ou des muqueuses, contaminés par des composés organophosphorés, ou dans le cadre de la préparation de médicaments utilisables dans le cadre de la prévention ou du traitement d'une contamination externe ou d'une intoxication interne par ingestion ou inhalation par des composés organophosphorés, ou dans le cadre de la dépollution des eaux polluées par des composés organophosphorés.

Un autre but de la présente invention est de fournir des kits pour la décontamination des surfaces des matériaux, de la peau, ou des muqueuses, contaminés par des composés organophosphorés, ou pour la dépollution des eaux polluées par des composés organophosphorés, ou pour la destruction des stocks de neurotoxiques.

Un autre but de la présente invention est de fournir un capteur des ces composés organophosphorés, présentant l'avantage d'être extrêmement sensible grâce aux capacités susmentionnés des nouvelles PTEs.

Un autre but de la présente invention est de fournir des matériaux imprégnés de nouvelles PTEs présentant les avantages susmentionnés, sous forme liquide ou solide, tels que gants, vêtements divers (notamment immobilisation sur tissus pour tenues de protections chimiques), lingettes, mousses pulvérisables.

Un autre but de la présente invention est de fournir des compositions pharmaceutiques, notamment sous forme injectable ou de pommades, comprenant les nouvelles PTEs présentant les avantages susmentionnés, en association avec un véhicule pharmaceutiquement acceptable.

Un autre but de la présente invention est de fournir des cartouches de décontamination externe, à l'intérieur desquelles sont greffées des nouvelles PTEs, notamment pour décontaminer le sang d'une personne intoxiquée par les organophosphorés.

Un autre but de la présente invention est de fournir des bactéries transformées à l'aide de séquences nucléotidiques codant pour ces nouvelles PTEs et exprimant ces dernières dans leur cytoplasme ou à leur surface, lesdites bactéries ainsi transformées étant susceptibles d'être utilisées à leur tour dans le cadre de la décontamination des organophosphorés.

L'invention a pour objet des phosphotriestérases (PTE) hyperthermophiles mutées possédant une activité lactonase dérivées des PTE hyperthermophiles répondant à la séquence consensus SEQ ID NO : 1, et comprenant l'une au moins des quatre mutations suivantes :
- substitution de la tyrosine Y en position 98,
- substitution de la tyrosine Y en position 100,
- substitution de l'arginine R en position 224,
- substitution de la cystéine C en position 259,
de SEQ ID NO : 1 par tout autre aminoacide naturel ou non, lesdites phosphotriestérases (PTE) hyperthermophiles mutées possédant une activité lactonase possédant une activité suppérieure à celle des phosphotriestérases (PTE) hyperthermophiles non mutées dont elles dérivent.

Les PTEs hyperthermophiles mutées possédant une activité lactonase susmentionnées de l'invention ont l'avantage d'être thermostables, à savoir d'être des protéines capables de conserver leur activité enzymatique à des températures comprises jusqu'à 95°C (Merone et al., 2005). L'activité enzymatique des PTEs hyperthermophiles mutées susmentionnées de l'invention correspond notamment à l'activité d'hydrolyse des OPs telle que mesurée selon la méthode précédemment décrite (Merone et al., 2005). Cette thermostabilité leur confère l'avantage d'être peu chères à produire, d'une part car elles sont stables dans les solvants organiques ce qui leur permet de s'accommoder mieux aux procédés industriels, et, d'autre part, car elles sont très peu chères à purifier par la technique du chauffage des lysats cellulaires des cellules productrices de ces PTEs, telle que *E*. *coli* ; on obtient ainsi un grand rendement et une bonne pureté en une étape.

Les PTEs hyperthermophiles mutées possédant une activité lactonase susmentionnées de l'invention ont également l'avantage d'être plus actives dans le cadre de l'hydrolyse des OPs (notamment selon la méthode susmentionnée), que les PTEs hyperthermophiles sauvages dont elles dérivent. Les PTEs hyperthermophiles mutées possédant une activité lactonase susmentionnées ont également l'avantage d'être plus actives dans le cadre du « quorum quenching » que les PTEs hyperthermophiles sauvages dont elles dérivent, c'est-à-dire dans le cadre de la résistance aux infections de pathogènes.

L'invention a plus particulièrement pour objet les phosphotriestérases hyperthermophiles mutées susmentionnées, dérivées de la PTE hyperthermophile de *Sulfolobus solfataricus* correspondant à la séquence SEQ ID NO: 3, ou de la PTE hyperthermophile de *Sulfolobus acidocaldarius* correspondant à la séquence SEQ ID NO : 5, lesdites séquences SEQ ID NO : 3 et SEQ ID NO : 5 appartenant à la séquence consensus SEQ ID NO : 1, l'aminoacide en position 2 dans SEQ ID NO : 1 étant manquant dans SEQ ID NO :3.

L'invention concerne plus particulièrement les phosphotriestérases hyperthermophiles mutées possédant une activité lactonase susmentionnées, comprenant au moins les quatre mutations suivantes :
- substitution de la tyrosine Y en position 98,
- substitution de la tyrosine Y en position 100,
- substitution de l'arginine R en position 224,
- substitution de la cystéine C en position 259,
de SEQ ID NO : 1 par tout autre aminoacide naturel ou non.

L'invention a plus particulièrement pour objet les phosphotriestérases hyperthermophiles mutées possédant une activité lactonase susmentionnées, caractérisées en ce qu'elles comprennent également l'une au moins des mutations suivantes :
- substitution de la valine V en position 28,
- substitution de la proline P en position 68,
- substitution de la thréonine T en position 69,
- substitution de la leucine L en position 73,
- substitution de l'aspartate D en position 142,
- substitution de la glycine G en position 226,
- substitution de la leucine L en position 227,
- substitution de la phénylalanine F en position 230,
- substitution du tryptophane W en position 264,
- substitution du tryptophane W en position 279,
de SEQ ID NO : 1 par tout autre aminoacide naturel ou non.

L'invention concerne plus particulièrement les phosphotriestérases hyperthermophiles mutées possédant une activité lactonase susmentionnées, caractérisées en ce qu'elles comprennent les cinq mutations suivantes :
- substitution de la valine V en position 28,
- substitution de la leucine L en position 73,
- substitution de l'aspartate D en position 142,
- substitution de la glycine G en position 226,
- substitution de la leucine L en position 227,
de SEQ ID NO : 1 par tout autre aminoacide naturel ou non.

L'invention a plus particulièrement pour objet les phosphotriestérases hyperthermophiles mutées possédant une activité lactonase susmentionnées, caractérisées en ce qu'elles comprennent les cinq mutations suivantes :
- substitution de la proline P en position 68,
- substitution de la thréonine T en position 69,
- substitution de la phénylalanine F en position 230,
- substitution du tryptophane W en position 264,
- substitution du tryptophane W en position 279,
de SEQ ID NO : 1 par tout autre aminoacide naturel ou non.

L'invention concerne plus particulièrement les phosphotriestérases hyperthermophiles mutées possédant une activité lactonase susmentionnées, dérivées des PTE hyperthermophiles répondant à la séquence consensus SEQ ID NO : 1, caractérisées en ce qu'elles comprennent l'une au moins des quatre mutations suivantes :
- substitution de la tyrosine Y en position 98 par un tryptophane W,
- substitution de la tyrosine Y en position 100 par une phénylalanine F,
- substitution de l'arginine R en position 224 par une histidine H,
- substitution de la cystéine C en position 259 par une leucine L,
et, le cas échéant, l'une au moins des mutations suivantes :
- substitution de la valine V en position 28 par une alanine A,
- substitution de la proline P en position 68 par une valine V,
- substitution de la thréonine T en position 69 par une sérine S,
- substitution de la leucine L en position 73 par une isoleucine I,
- substitution de l'aspartate D en position 142 par une thréonine T,
- substitution de la glycine G en position 226 par une proline P,
- substitution de la leucine L en position 227 par une histidine H,
- substitution de la phénylalanine F en position 230 par une sérine S,
- substitution du tryptophane W en position 264 par une alanine A,
- substitution du tryptophane W en position 279 par une isoleucine I.

L'invention a plus particulièrement pour objet les phosphotriestérases hyperthermophiles mutées possédant une activité lactonase susmentionnées, dérivées de la PTE hyperthermophile de *Sulfolobus solfataricus* correspondant à la séquence SEQ ID NO : 3, et comprenant l'une au moins des quatre mutations suivantes :
- substitution de la tyrosine Y en position 97,
- substitution de la tyrosine Y en position 99,
- substitution de l'arginine R en position 223,
- substitution de la cystéine C en position 258,
de SEQ ID NO : 3 par tout autre aminoacide naturel ou non.

L'invention concerne plus particulièrement les phosphotriestérases hyperthermophiles mutées possédant une activité lactonase susmentionnées, dérivées de la PTE hyperthermophile de *Sulfolobus solfataricus* correspondant à la séquence SEQ ID NO : 3, et comprenant au moins les quatre mutations suivantes :
- substitution de la tyrosine Y en position 97,
- substitution de la tyrosine Y en position 99,
- substitution de l'arginine R en position 223,
- substitution de la cystéine C en position 258,
de SEQ ID NO : 3 par tout autre aminoacide naturel ou non.

L'invention a plus particulièrement pour objet les phosphotriestérases hyperthermophiles mutées possédant une activité lactonase susmentionnées, dérivées de la PTE hyperthermophile de *Sulfolobus solfataricus* correspondant à la séquence SEQ ID NO : 3, caractérisées en ce qu'elles comprennent également l'une au moins des mutations suivantes :
- substitution de la valine V en position 27,
- substitution de la proline P en position 67,
- substitution de la thréonine T en position 68,
- substitution de la leucine L en position 72,
- substitution de l'aspartate D en position 141,
- substitution de la glycine G en position 225,
- substitution de la leucine L en position 226,
- substitution de la phénylalanine F en position 229,
- substitution du tryptophane W en position 263,
- substitution du tryptophane W en position 278,
de SEQ ID NO : 3 par tout autre aminoacide naturel ou non.

L'invention concerne plus particulièrement les phosphotriestérases hyperthermophiles mutées possédant une activité lactonase susmentionnées, dérivées de la PTE hyperthermophile de *Sulfolobus solfataricus* correspondant à la séquence SEQ ID NO : 3, caractérisées en ce qu'elles comprennent les cinq mutations suivantes :
- substitution de la valine V en position 27,
- substitution de la leucine L en position 72,
- substitution de l'aspartate D en position 141,
- substitution de la glycine G en position 225,
- substitution de la leucine L en position 226,
de SEQ ID NO : 3 par tout autre aminoacide naturel ou non.

L'invention a plus particulièrement pour objet les phosphotriestérases hyperthermophiles mutées possédant une activité lactonase susmentionnées, dérivées de la PTE hyperthermophile de *Sulfolobus solfataricus* correspondant à la séquence SEQ ID NO : 3, caractérisées en ce qu'elles comprennent les cinq mutations suivantes :
- substitution de la proline P en position 67,
- substitution de la thréonine T en position 68,
- substitution de la phénylalanine F en position 229,
- substitution du tryptophane W en position 263,
- substitution du tryptophane W en position 278,
de SEQ ID NO : 3 par tout autre aminoacide naturel ou non.

L'invention concerne plus particulièrement les phosphotriestérases hyperthermophiles mutées possédant une activité lactonase susmentionnées, dérivées de la PTE hyperthermophile de *Sulfolobus solfataricus* correspondant à la séquence SEQ ID NO : 3, caractérisées en ce qu'elles comprennent l'une au moins des quatre mutations suivantes :
- substitution de la tyrosine Y en position 97 par un tryptophane W,
- substitution de la tyrosine Y en position 99 par une phénylalanine F,
- substitution de l'arginine R en position 223 par une histidine H,
- substitution de la cystéine C en position 258 par une leucine L,
et, le cas échéant, l'une au moins des mutations suivantes :
- substitution de la valine V en position 27 par une alanine A,
- substitution de la proline P en position 67 par une valine V,
- substitution de la thréonine T en position 68 par une sérine S,
- substitution de la leucine L en position 72 par une isoleucine I,
- substitution de l'aspartate D en position 141 par une thréonine T,
- substitution de la glycine G en position 225 par une proline P,
- substitution de la leucine L en position 226 par une histidine H,
- substitution de la phénylalanine F en position 229 par une sérine S,
- substitution du tryptophane W en position 263 par une alanine A,
- substitution du tryptophane W en position 278 par une isoleucine I.

L'invention a plus particulièrement pour objet les phosphotriestérases hyperthermophiles mutées possédant une activité lactonase susmentionnées, dérivées de la PTE hyperthermophile de *Sulfolobus solfataricus* correspondant à la séquence SEQ ID NO : 3, caractérisées en ce qu'elles correspondent aux séquences suivantes :
- SEQ ID NO : 7 correspondant à la séquence SEQ ID NO : 3 comprenant les quatre mutations suivantes :
   * substitution de la tyrosine Y en position 97 par un tryptophane W,
   * substitution de la tyrosine Y en position 99 par une phénylalanine F,
   * substitution de l'arginine R en position 223 par une histidine H,
   * substitution de la cystéine C en position 258 par une leucine L,
- SEQ ID NO : 9 correspondant à la séquence SEQ ID NO : 7 comprenant en plus les cinq mutations suivantes :
   * substitution de la valine V en position 27 par une alanine A,
   * substitution de la leucine L en position 72 par une isoleucine I,
   * substitution de l'aspartate D en position 141 par une thréonine T,
   * substitution de la glycine G en position 225 par une proline P,
   * substitution de la leucine L en position 226 par une histidine H,
- SEQ ID NO : 11 correspondant à la séquence SEQ ID NO : 9 comprenant en plus les cinq mutations suivantes :
   * substitution de la proline P en position 67 par une valine V,
   * substitution de la thréonine T en position 68 par une sérine S,
   * substitution de la phénylalanine F en position 229 par une sérine S,
   * substitution du tryptophane W en position 263 par une alanine A,
   * substitution du tryptophane W en position 278 par une isoleucine I.

L'invention concerne plus particulièrement les phosphotriestérases hyperthermophiles mutées possédant une activité lactonase susmentionnées, dérivées de la PTE hyperthermophile de *Sulfolobus solfataricus* correspondant à la séquence SEQ ID NO : 3, caractérisées en ce qu'elles comprennent au moins une mutation correspondant à la substitution de l'un au moins des aminoacides des couples d'aminoacides suivants dont les positions dans SEQ ID NO : 3 sont indiquées ci-après par un autre aminoacide naturel ou non: 2R/314S, 14K/12E, 26R/75D, 26R/42E, 33R/42E, 33R/45E, 55R/52E, 55R/285E, 74R/121D, 81K/42E, 81K/43D, 84K/80E, 109R/113E, 123K/162E, 147K/148D, 151K/148D, 154R/150E, 154R/187E, 154R/188E, 161K/188E, 183R/150E, 183R/187E, 183R/180E, 210K/245D, 215K/214D, 223R/256D, 223R/202D, 234K/204D, 235R/202D, 241R/245D, 245D/244K, 250K/249D, 277R/286D, 292K/298E, 310K/307E.

L'invention a plus particulièrement pour objet les phosphotriestérases hyperthermophiles mutées possédant une activité lactonase susmentionnées, dérivées de la PTE hyperthermophile de *Sulfolobus acidocaldarius* correspondant à la séquence SEQ ID NO : 5, et comprenant l'une au moins des quatre mutations suivantes :
- substitution de la tyrosine Y en position 98,
- substitution de la tyrosine Y en position 100,
- substitution de l'arginine R en position 224,
- substitution de la cystéine C en position 259,
de SEQ ID NO : 5 par tout autre aminoacide naturel ou non.

L'invention concerne plus particulièrement les phosphotriestérases hyperthermophiles mutées possédant une activité lactonase susmentionnées, dérivées de la PTE hyperthermophile de *Sulfolobus acidocaldarius* correspondant à la séquence SEQ ID NO : 5, comprenant au moins les quatre mutations suivantes :
- substitution de la tyrosine Y en position 98,
- substitution de la tyrosine Y en position 100,
- substitution de l'arginine R en position 224,
- substitution de la cystéine C en position 259,
de SEQ ID NO : 5 par tout autre aminoacide naturel ou non.

L'invention a plus particulièrement pour objet les phosphotriestérases hyperthermophiles mutées possédant une activité lactonase susmentionnées, dérivées de la PTE hyperthermophile de *Sulfolobus acidocaldarius* correspondant à la séquence SEQ ID NO : 5, caractérisées en ce qu'elles comprennent également l'une au moins des mutations suivantes :
- substitution de la valine V en position 28,
- substitution de la proline P en position 68,
- substitution de la thréonine T en position 69,
- substitution de la leucine L en position 73,
- substitution de l'aspartate D en position 142,
- substitution de la glycine G en position 226,
- substitution de la leucine L en position 227,
- substitution de la phénylalanine F en position 230,
- substitution du tryptophane W en position 264,
- substitution du tryptophane W en position 279,
de SEQ ID NO : 5 par tout autre aminoacide naturel ou non.

L'invention concerne plus particulièrement les phosphotriestérases hyperthermophiles mutées possédant une activité lactonase susmentionnées, dérivées de la PTE hyperthermophile de *Sulfolobus acidocaldarius* correspondant à la séquence SEQ ID NO : 5, caractérisées en ce qu'elles comprennent les cinq mutations suivantes :
- substitution de la valine V en position 28,
- substitution de la leucine L en position 73,
- substitution de l'aspartate D en position 142,
- substitution de la glycine G en position 226,
- substitution de la leucine L en position 227,
de SEQ ID NO : 5 par tout autre aminoacide naturel ou non.

L'invention a plus particulièrement pour objet les phosphotriestérases hyperthermophiles mutées possédant une activité lactonase susmentionnées, dérivées de la PTE hyperthermophile de *Sulfolobus acidocaldarius* correspondant à la séquence SEQ ID NO : 5, caractérisées en ce qu'elles comprennent les cinq mutations suivantes :
- substitution de la proline P en position 68,
- substitution de la thréonine T en position 69,
- substitution de la phénylalanine F en position 230,
- substitution du tryptophane W en position 264,
- substitution du tryptophane W en position 279,
de SEQ ID NO : 5 par tout autre aminoacide naturel ou non.

L'invention concerne plus particulièrement les phosphotriestérases hyperthermophiles mutées possédant une activité lactonase susmentionnées, dérivées de la PTE hyperthermophile de *Sulfolobus acidocaldarius* correspondant à la séquence SEQ ID NO : 5, caractérisées en ce qu'elles comprennent l'une au moins des quatre mutations suivantes :
- substitution de la tyrosine Y en position 98 par un tryptophane W,
- substitution de la tyrosine Y en position 100 par une phénylalanine F,
- substitution de l'arginine R en position 224 par une histidine H,
- substitution de la cystéine C en position 259 par une leucine L,
et, le cas échéant, l'une au moins des mutations suivantes :
- substitution de la valine V en position 28 par une alanine A,
- substitution de la proline P en position 68 par une valine V,
- substitution de la thréonine T en position 69 par une sérine S,
- substitution de la leucine L en position 73 par une isoleucine I,
- substitution de l'aspartate D en position 142 par une thréonine T,
- substitution de la glycine G en position 226 par une proline P,
- substitution de la leucine L en position 227 par une histidine H,
- substitution de la phénylalanine F en position 230 par une sérine S,
- substitution du tryptophane W en position 264 par une alanine A,
- substitution du tryptophane W en position 279 par une isoleucine I.

L'invention a plus particulièrement pour objet les phosphotriestérases hyperthermophiles mutées possédant une activité lactonase susmentionnées, dérivées de la PTE hyperthermophile de *Sulfolobus acidocaldarius* correspondant à la séquence SEQ ID NO : 5, caractérisées en ce qu'elles correspondent aux séquences suivantes :
- SEQ ID NO : 13 correspondant à la séquence SEQ ID NO : 5 comprenant les quatre mutations suivantes :
   * substitution de la tyrosine Y en position 98 par un tryptophane W,
   * substitution de la tyrosine Y en position 100 par une phénylalanine F,
   * substitution de l'arginine R en position 224 par une histidine H,
   * substitution de la cystéine C en position 259 par une leucine L,
- SEQ ID NO : 15 correspondant à la séquence SEQ ID NO : 13 comprenant en plus les cinq mutations suivantes :
   * substitution de la valine V en position 28 par une alanine A,
   * substitution de la leucine L en position 73 par une isoleucine I,
   * substitution de l'aspartate D en position 142 par une thréonine T,
   * substitution de la glycine G en position 226 par une proline P,
   * substitution de la leucine L en position 227 par une histidine H,
- SEQ ID NO : 17 correspondant à la séquence SEQ ID NO : 15 comprenant en plus les cinq mutations suivantes :
   * substitution de la proline P en position 68 par une valine V,
   * substitution de la thréonine T en position 69 par une sérine S,
   * substitution de la phénylalanine F en position 230 par une sérine S,
   * substitution du tryptophane W en position 264 par une alanine A,
   * substitution du tryptophane W en position 279 par une isoleucine I.

L'invention a plus particulièrement pour objet les phosphotriestérases hyperthermophiles mutées possédant une activité lactonase susmentionnées, dans lesquelles au moins l'un des acides aminés impliqués dans les ponts salins est modifié par substitution ou délétion, de telle sorte que la température d'activation des dites phosphotriestérases hyperthermophiles mutées possédant une activité lactonase soit diminuée par rapport à la température d'activation des phosphotriestérases hyperthermophiles mutées possédant une activité lactonase dans lesquels les acides aminés impliqués dans les ponts salins sont non modifiés.

Dans un mode de réalisation avantageux de l'invention, les acides aminés impliqués dans les ponts salins mentionnés précédemment peuvent être remplacés par une séquence d'au moins deux acides aminés. Il s'agit alors d'une addition.

Il est entendu dans l'invention que le terme « substitution » correspond au remplacement d'un acide aminé par un autre. Les substitutions peuvent être conservatives, c'est-à-dire que l'acide aminé substitué est remplacé par un acide aminé **de même structure**ou de même propriété physico-chimique (acides aminés polaires, hydrophobes, acides, basiques...) de telle sorte que la structure tridimensionnelle de la protéine reste inchangée, ou au contraire non conservative. Egalement les substitutions définies selon l'invention concernent des acides aminés naturels, ou des acides aminés artificiels. Ainsi, les acides aminés impliqués dans les ponts salins peuvent être remplacés par un acide aminé naturel ou un acide aminé artificiel.

Il est également entendu dans l'invention que la « délétion » correspond au retrait d'un acide aminé, de telles sorte que la séquence protéique ayant subit ladite délétion est plus courte que la séquence n'ayant pas subit ladite délétion.

L'étude de la structure tridimensionnelle des PTE hyperthermophyles a permis de mettre en évidence les acides aminés impliqués dans les ponts salins. Ces acides aminés importants sont chargés et ont une distance d'interaction inférieure à 5,5Å. Si la protéine, bien que possédant des mutations dans le site actif, citées précédemment, ne possède pas une activité suffisante comparée aux PTE mésophile, il est possible de muter les acides aminés impliqués dans les ponts salins. Ces mutations ont pour effet de rompre les interactions, et de rendre la protéine plus flexible.

Les données cristallographiques ont permis de montrer 25 interactions d'une distance de moins de 4Å, 6 interactions de distances comprises entre 4Å et 5Å, et 4 interactions de distances comprises entre 5Å et 5,5Å. Les interactions susmentionnées impliquent 2 acides aminés. Ainsi, au total, 52 acides aminés sont impliqués dans les ponts salins.

Les acides aminés impliqués dans ces interactions, ainsi que la distance de l'interaction desdits acides aminés sont indiqués dans le tableau 1 suivant.

**Tableau 1 : interaction impliquées dans les ponts salins de la protéine SsoPox**

| D<4Å | 4Å <D<5Å | 5Å <D<5,5Å |
|---|---|---|
| 2R ↔ 314S-COOH | 33R ↔ 42E | 145R ↔ 187B |
| 14K ↔ 12E | 81K ↔ 42E | 183R ↔ 150E |
| 26R ↔ 75D | 84K ↔ 80E | 215R ↔ 214D |
| 26R ↔ 42E | 147K ↔ 148D | 244K ↔ 245D |
| 33R ↔ 45E | 161K ↔ 188E | |
| 55R ↔ 52E | 310K ↔ 307E | |
| 55R ↔ 285E | | |
| 74R ↔ 121D | | |
| 81K ↔ 43D | | |
| 109R ↔ 113E | | |
| 123K ↔ 162E | | |
| 151K ↔ 148D | | |
| 154R ↔ 150E | | |
| 154R ↔ 188E | | |
| 183R ↔ 187E | | |
| 183R ↔ 180E | | |
| 210K ↔ 245D | | |
| 223R ↔ 256D | | |
| 223R ↔ 202D | | |
| 234K ↔ 204D | | |
| 235R ↔ 202D | | |
| 241R ↔ 245D | | |
| 250K ↔ 249D | | |
| 277R ↔ 286D | | |
| 292K ↔ 298E | | |

Dans un mode de réalisation avantageux de l'invention, dans les phosphotriestérases hyperthermophiles mutées possédant une activité lactonase susmentionnées de Sso, l'un au moins des acides aminés des positions suivantes 2, 12, 14, 26, 33, 42, 43, 45, 52, 55, 74, 75, 80, 81, 84, 109, 113, 121, 123, 145, 147, 148, 150, 151, 154, 161, 162, 180, 183, 187, 188, 202, 204, 210, 214, 215, 223, 234, 235, 241, 244, 245, 249, 250, 256, 277, 285, 286, 292, 298, 307 et 310 est modifié. La position des acides aminés précédents est définie par rapport au premier acide aminé de la protéine SsoPox.

Dans un mode de réalisation avantageux de l'invention, dans les phosphotriestérases hyperthermophiles mutées possédant une activité lactonase susmentionnées de Sac, l'un au moins des acides aminés des positions suivantes 3, 13, 15, 27, 34, 43, 44, 46, 53, 56, 75, 76, 81, 82, 85, 110, 114, 122, 124, 146, 148, 149, 151, 152, 155, 162, 163, 181, 184, 188, 189, 203, 205, 211, 215, 216, 224, 235, 236, 242, 245, 246, 250, 251, 257, 278, 286, 287, 293, 299, 308 et 311 est modifié. La position des acides aminés précédents est définie par rapport au premier acide aminé de la protéine SacPox.

L'invention concerne également les séquences nucléotidiques codant pour les phosphotriestérases hyperthermophiles mutées possédant une activité lactonase telles que définies ci-dessus.

L'invention a également pour objet les vecteurs, notamment plasmides, contenant des séquences nucléotidiques codant pour les phosphotriestérases hyperthermophiles mutées possédant une activité lactonase telles que définies ci-dessus.

L'invention concerne également les cellules hôtes, notamment les bactéries, transformées à l'aide d'un vecteur tel que défini ci-dessus, de manière à ce que leur génome contienne des séquences nucléotidiques codant pour les phosphotriestérases hyperthermophiles mutées possédant une activité lactonase telles que définies ci-dessus, lesdites PTE hyperthermophiles mutées possédant une activité lactonase étant produites dans le cytoplasme des cellules hôtes, ou secrétées à leur surface.

L'invention concerne également les cellules hôtes, notamment bactéries, couplées aux phosphotriestérases hyperthermophiles mutées possédant une activité lactonase telles que définies ci-dessus, ou présantant des phosphotriestérases hyperthermophiles mutées possédant une activité lactonase telles que définies ci-dessus, greffées sur leur surface.

L'invention concerne également les organismes transgéniques, notamment mammifères, transformées à l'aide d'un vecteur tel que défini ci-dessus, lesdits organismes transgéniques étant résistants aux pathogènes.

L'invention a également pour objet l'utilisation de phosphotriestérases hyperthermophiles mutées possédant une activité lactonase telles que définies ci-dessus, ou de cellules hôtes transformées susmentionnées, en tant que bio-épurateurs dans le cadre de la décontamination des surfaces des matériaux, de la peau, ou des muqueuses, contaminés par des composés organophosphorés, **ou des bactéries** ou dans le cadre de la préparation de médicaments (ou bio-médicaments) utilisables dans le cadre de la prévention ou du traitement d'une contamination externe ou d'une intoxication interne par ingestion ou inhalation par des composés organophosphorés, ou dans le cadre de la préparation de médicaments utilisables dans le cadre de la prévention ou du traitement d'une infection bactérienne, ou dans le cadre de la dépollution des eaux polluées par des composés organophosphorés, ou dans le cadre de la destruction des stocks de neurotoxiques.

L'invention a également pour objet des matériaux imprégnés de phosphotriestérases hyperthermophiles mutées possédant une activité lactonase telles que définies ci-dessus, sous forme liquide ou solide, tels que gants, vêtements divers (notamment immobilisation sur tissus pour tenues de protections chimiques), lingettes, mousses pulvérisables.

L'invention concerne également des kits pour la décontamination des surfaces des matériaux, de la peau, ou des muqueuses, contaminés par des composés organophosphorés, ou pour la dépollution des eaux polluées par des composés organophosphorés, caractérisés en ce qu'ils comprennent des phosphotriestérases hyperthermophiles mutées possédant une activité lactonase telles que définies ci-dessus (le cas échéant sous forme lyophilisée), ou des matériaux imprégnés de phosphotriestérases hyperthermophiles mutées possédant une activité lactonase susmentionnés.

L'invention a également pour objet des capteurs des composés organophosphorés susmentionnés, présentant l'avantage d'être extrêmement sensible grâce aux capacités décrites ci-dessus des phosphotriestérases hyperthermophiles mutées possédant une activité lactonase telles que définies ci-dessus.

L'invention concerne également des cartouches de décontamination externe, à l'intérieur desquelles sont greffées des phosphotriestérases hyperthermophiles mutées possédant une activité lactonase telles que définies ci-dessus selon l'invention, et utilisables notamment pour décontaminer le sang d'une personne intoxiquée par les organophosphorés.

L'invention concerne également des compositions pharmaceutiques caractérisées en ce qu'elles comprennent des phosphotriestérases hyperthermophiles mutées possédant une activité lactonase telles que définies ci-dessus, en association avec un véhicule pharmaceutiquement acceptable.

L'invention a également pour objet des compositions pharmaceutiques telles que définies ci-dessus, caractérisées en ce qu'elles se présentent sous une forme administrable par voie injectable notamment en solution ou encapsidées ou peggylées, ou par voie topique, notamment sous forme PEGylée ou encapsulée ou sous forme de pommade, aérosol ou lingettes.

L'invention concerne également l'utilisation de matériaux imprégnés susmentionnés, ou de cartouches de décontamination externes définies ci-dessus, en tant qu'antiseptiques pour la décontamination de l'infection bactérienne de surface.

L'invention concerne également l'utilisation de la composition pharmaceutique définie précédemment, pour le traitement des infections bactériennes, notamment dans le sang.

L'invention sera davantage illustrée à l'aide de la description détaillée qui suit de la phosphotriestérase de *Sulfolobus solfataricus,* et des mutations effectuées à cette dernière dans le cadre de la préparation de phosphotriestérases hyperthermophiles mutées possédant une activité lactonase telles que définies ci-dessus selon l'invention.

Les organophosphorés (OPs) sont des molécules très toxiques, qui composent certains agents chimiques de guerre et des pesticides. Ces produits représentent de nos jours un grave problème de santé publique (intoxications, pollutions des sols, pollutions des eaux). Les solutions actuellement disponibles ne sont malheureusement pas satisfaisantes. Néanmoins, les biotechnologies constituent une alternative extrêmement prometteuse. En effet, certaines enzymes utilisables comme « bioscavenger » sont capables de détruire avec une grande efficacité un large spectre de ces poisons.

### I) Etude structurale de la phosphotriestérase de Sulfolobus solfataricus

Cette enzyme, issue d'une archae, possède la caractéristique d'être hyperthermophile. Son étude permet d'approfondir les connaissances sur les protéines thermostables. Cette protéine, capable d'hydrolyser les OPs, est un candidat potentiel aux décontaminations environnementales et cutanées. Ce travail sert donc de base pour obtenir, par bio-ingénierie, des enzymes actives et thermostables. Cette dernière caractéristique permet une production importante à faible coût. La protéine recombinante, produite par un organisme mésophile tel que *Escherichia coli,* peut être purifiée efficacement en une étape. Les lysats cellulaires sont chauffés, entraînant la précipitation des protéines mésophiles de l'hôte. Ne reste soluble que la protéine hyperthermostable.

Également, les travaux permis de démontrer que SsoPox est une lactonase naturelle avec une activité de promiscuité phosphotriesterase (Elias et al., 2007b).

Grâce à son activité de « quorum quenching », sa facilité de production à bas coût, et sa grande stabilité qui la rend compatible aux contraintes des processus industriels, SsoPox est un candidat prometteur pour lutter contre les infections bactériennes en utilisant la voie du quorum quenching.

### A) Matériel et méthodes

### 1. CRISTALLISATION

Le clonage, l'expression, et la purification de la PTE hyperthermophile de *S*. *Solfataricus* chez *E*. *coli* sont décrits par Merone et al. (2005). L'enzyme a été concentrée à 5.8mg.mL-¹. Les essais de cristallisation ont été réalisés en utilisant la méthode de la diffusion de vapeur en goutte suspendue. Des volumes égaux, allant de 1 à 2 µL de solution protéique et de la solution du réservoir ont été mélangés. Les gouttes résultantes ont été équilibrées contre une solution réservoir contenant de 15 à 18% (masse/volume) de polyéthylène glycol (PEG) 8000 en tampon Tris-HCl pH 8. De très fins cristaux apparaissent après une semaine à 4°C.

### 2. COLLECTE DES DONNEES

Les cristaux ont tout d'abord été transférés dans une solution cryoprotectante composée de la solution du réservoir et de 25% (volume/volume) de glycérol. Chaque cristal a ensuite été congelé instantanément dans de l'azote liquide. Les données de diffraction aux rayons X ont été collectées à 100°K sur la ligne FIP BM 30 du synchrotron de Grenoble. (ESRF, Grenoble, France). Pour ces collectes, un détecteur MarCCD (165mm) a été utilisé. Un jeu de données a été enregistré à 2.6Å de résolution.

### 3. DETERMINATION DE LA STRUCTURE

Les données de diffraction ont été intégrées et mises à l'échelle grâce aux programmes XDS2000 et la suite de logiciels CCP4 (Collaborative Computational Project). Le premier remplacement moléculaire a été réalisé avec PHASER en utilisant un modèle polyalanine déduit de la structure de la PTE de *P. diminuta* (code PDB : 1DPM). Deux molécules de protéines ont été trouvées dans l'unité asymétrique. Le site actif constitué de deux ions métalliques, était clairement visible. La carte de densité électronique a été améliorée en utilisant un procédé d'aplatissement de solvant et la moyennation par symétrie non cristallographique des deux molécules grâce au logiciel DM. La construction manuelle du modèle a été réalisée en utilisant le programme COOT. Certaines chaînes latérales ont pu être placées et quelques boucles impliquées dans l'interface du dimère ont du être retirées. Un nouveau remplacement moléculaire a été réalisé en utilisant MOLREP, en fixant les deux premières solutions trouvées et en utilisant le modèle ainsi amélioré. Deux solutions supplémentaires ont été trouvées, pour donner un total de quatre molécules dans l'unité asymétrique (R= 46%, Rfree= 51 %). Le modèle de structure a ensuite été construit manuellement et affiné par des successions de cycles des logiciels COOT et REFMAC.

### B) Résultats

### 1. STRUCTURE TRIDIMENSIONNELLE

La PTE de *S*. *solfataricus* cristallise en PEG et les données de diffraction ont été collectées au synchrotron (ESRF, Grenoble, France). La structure de cette protéine a été déterminée par la méthode du remplacement moléculaire (voir Matériel et Méthodes), et affinée à 2.6 Å de résolution à un facteur R de 21.8%, un Rfree de 28% et une bonne géométrie. La chaîne principale est entièrement visible dans la carte de densité, et ce pour les deux homodimères de l'unité asymétrique, malgré une résolution moyenne.

La structure de cette PTE hyperthermophile est telle que la molécule est approximativement globulaire avec des dimensions d'environ 40 Å × 54 Å × 46 Å. Sa topologie est très similaire aux deux PTEs mésophiles dont les structures sont déjà connues. La première est issue de *P. diminuta* (Vanhooke et al., 1994; code pdb: IDPM) et la seconde est issue d'*A*. *radiobacter* (Jackson et al., 2005; code pdb: 2D2J). Cette structure hyperthermostable peut être décrite comme un tonneau (β/α)₈ distordu aussi appelé tonneau TIM. Elle consiste en 8 brins β parallèles formant le tonneau flanqué par 11 hélices α. La superposition de ces trois structures résolues donne des déviations moyennes (RMS) pour la position des carbones α entre la PTE de *S*. *solfataricus* et celle de *P. diminuta* (sur 268 atomes) et entre la PTE de *S*. *solfataricus* et celle de *A. radiobacter* (sur 271 atomes) de 1.05 Å 1.11 Å, respectivement.

Il y a quelques différences majeures, à savoir deux raccourcissements de la structure de la PTE de *S*. *solfataricus* comparée aux structures des PTE mésophiles. Le premier est localisé à l'entrée du site actif et consiste en la délétion d'une boucle de 15 résidus. L'autre raccourcissement concerne les deux extrémités de la chaîne polypeptidique. En effet, il y a 6 et 2 résidus en moins à l'extrémité C-terminal, 2 et 4 résidus en moins en N-terminal, par rapport aux PTEs de *P. diminuta* et d'*A*. *radiobacter,* respectivement. Une autre modification concerne la présence, dans la structure de la PTE de *S*. *solfataricus,* de boucles supplémentaires impliquées dans la dimérisation.

### 2. INTERFACE DU DIMERE

Tout comme les PTEs mésophiles (Benning et al., 1994; Jackson et al., 2005), la PTE de *S*. *solfataricus* cristallise en homodimère. Pour les deux PTEs mésophiles, l'aire de contact entre les monomères est d'environ 1350 Å², avec 62.5% de contacts hydrophobiques, et on compte environ 25 liaisons hydrogènes à l'interface. Dans la structure de la PTE de *S*. *solfataricus,* les boucles supplémentaires impliquées dans la formation du dimère augmentent la surface de contact. Les deux monomères semblent ainsi s'interpénétrer. En effet, l'aire de contact est de 1720 Å². Malgré cette augmentation, la quantité de liaisons hydrogènes à l'interface est du même ordre (environ 20). En fait, cette augmentation de la surface de contact est essentiellement due à des contacts hydrophobes supplémentaires. Ainsi, les contacts hydrophobes à l'interface représentent 68% de contacts totaux dans ce dimère.

En outre, la conformation du dimère n'est pas identique à celle des PTEs mésophiles. Il y a un mouvement évident de la position relative du second monomère qui pourrait avoir été causé par les boucles supplémentaires impliquées dans la dimérisation.

Un autre point intéressant concerne l'accessibilité globale au solvant de la protéine. En fait, toutes ces PTEs : celle de *P. diminuta*, d'*A. radiobacter* et de *S*. *solfataricus,* ont environ la même accessibilité au solvant en ce qui concerne les monomères (13076.1 Å², 12828.7 Å², 13039.4 Å², respectivement), et environ le même volume (42464.2 Å³, 44313.2 Å³, 43429.9 Å³ respectivement). Par contre, le dimère hyperthermostable est plus petit (86950 Å³) que les dimères mésophiles (88800 Å³).

### 3. POTENTIEL ELECTROSTATIQUE

Les analyses du potentiel électrostatique avec Swiss-PdbViewer révèlent que la PTE de *S. solfataricus* est une protéine très chargée. En effet, la structure montre 39 Asp et Glu, 37 Lys et Arg qui sont autant de charges localisées à la surface de la protéine. Ce nombre très élevé de résidus chargés est la cause de la présence de nombreux clusters de charges. Une face est principalement chargée négativement, l'autre plutôt positivement. Cette répartition particulière des charges doit conférer à la protéine un très fort moment dipolaire. En outre, les frontières entre les deux monomères sont uniformément chargées négativement. Ceci est très surprenant, car des charges non complémentaires auraient plutôt tendance à augmenter l'énergie de répulsion entre les deux monomères. Une seule région de la protéine est peu chargée. Il s'agit d'une poche hydrophobe, qui correspond au site actif. Ce dernier est entouré de charges négatives.

### 4. LIAISONS IONIQUES

Afin de compenser les énergies de couplage induites par ce grand nombre de charges et le fort moment dipolaire, la moitié de ces résidus chargés de surface est impliquée dans des ponts salins. La PTE de *S*. *solfataricus* compte 25 ponts salins par monomère à comparer avec les 15 unités pour les PTEs mésophiles. La majorité de ces ponts salins est uniformément localisée à la surface de la protéine. Comme décrit pour d'autres protéines hyperthermophiles, ce grand nombre de ponts salins forme des réseaux complexes de charges à la surface de la protéine.

### 5. DESCRIPTION DU SITE ACTIF

Comme les PTEs mésophiles, le site actif de la PTE de *S*. *solfataricus* consiste en un centre bimétallique, localisé en C-terminal du tonneau β. Ces deux cations métalliques sont pontés par la molécule d'eau catalytique, et par un résidu modifié, une lysine carboxylée. L'enzyme utilise une lysine carboxylée plutôt qu'un glutamate probablement car la paire d'électrons portée par l'azote peut être délocalisée, et ainsi permettre à chacun des oxygènes de porter une charge négative, et ainsi de compenser les quatre charges formelles positives des deux métaux. Pour leur coordination, quatre histidines sont également impliquées, ainsi qu'un acide aspartique (Asp 256) et une autre molécule d'eau. Le métal le plus enfoui (appelé α) adopte une géométrie en bipyramide trigonale, cordinné par His 22, His 24, Asp 256, Lys 137 et la molécule d'eau pontante. Le métal le plus exposé au solvant (appelé β) adopte une géométrie en bipyramide trigonale distordue, avec pour ligands His 170, His 199, Lys 137, la molecule d'eau pontante et une autre molécule d'eau. La molécule d'eau pontante est équidistante des deux métaux, avec une distance d'environ 2 Å, et est en liaison hydrogène avec Asp 256 (2.68 Å). Dans la PTE de *S*. *solfataricus,* le centre métallique, les quatre histidines, l'aspartate et la lysine carboxylée sont conservés et bien superposés avec les autres structures connues de PTE. La comparaison structurale avec les deux PTEs mésophiles a conduit à l'identification du site de fixation des phosphotriesters selon le site décrit (Chen-Goodspeed et al., 2001). La nature chimique des chaînes latérales dans la cavité est globalement inchangée par rapport aux PTEs mésophiles, ce qui conduit probablement à une fixation similaire des phosphotriesters dans le site actif de la PTE de *S*. *solfataricus.*

Bien que le repliement soit globalement similaire aux PTEs mésophiles, le site actif de la PTE de *S*. *solfataricus* est beaucoup plus étroit, à cause d'une légère modification du squelette peptidique et la présence de deux tyrosines (Tyr 97 et Tyr 99). En outre, à l'entrée du site actif, une boucle de neuf résidus crée un tunnel hydrophobe, qui est connecté au site actif. Ceci n'a pas été décrit dans les structures des PTEs mésophiles.

### C) Conclusion

### 1. THERMOSTABILITE

La PTE de *S*. *solfataricus* est une enzyme extrêmement stable. En effet, elle est active vis-à-vis du paraoxon jusqu'à des températures très élevées. Merone et al. (2005), n'ont, par ailleurs, pas trouvé son maximum d'activité pour des raisons techniques, mais jusqu'à 95°C, l'activité continue à augmenter. L'étude structurale de cette protéine montre quelques indices permettant d'expliquer le mécanisme de cette extraordinaire thermostabilité.

### 1.1 LE RACCOURCISSEMENT DE LA STRUCTURE

La structure de la PTE *S*. *solfataricus* montre la délétion d'une grande boucle par rapport aux PTEs mésophiles (voir résultats). Ceci est un cas classique, car les boucles des protéines thermostables sont très souvent plus courtes que celles de leurs homologues mésophiles (Vieille C., 1996), ce qui permet de stabiliser l'état natif, ou de défavoriser l'état dénaturé. Dans cette structure thermostable, les deux extrémités de la chaîne sont raccourcies et plus ancrées au coeur de la protéine. De plus, afin de renforcer ce coeur compact, ces deux extrémités sont en interaction ionique entre l'acide carboxylique terminal (Ser 314) et la chaîne latérale de Arg 2. Nous pouvons aussi noter que deux résidus prolines (Pro 4 et Pro 309) rigidifient un peu plus ces extrémités. Ce type de stabilisation mutuelle entre le N-terminal et le C-terminal est considéré comme un facteur augmentant la rigidité globale des protéines, et a également été décrit dans la phosphoribosyl anthranilate isomérase de *T*. *maritima.* En fait, la réduction de la flexibilité des boucles et des extrémités du squelette peptidique par leur stabilisation via un pont salin, ou bien leur raccourcissement ou leur délétion, contribue à l'augmentation globale de la stabilité de la protéine.

### 1.2 L'ASSOCIATION EN DIMERE

Les boucles supplémentaires impliquées dans le dimère thermostable ont des conséquences. La première concerne l'assemblage des monomères. En effet, la conformation de ce dimère est différente de la conformation du dimère classique TIM. Un fait similaire a déjà été décrit pour le dimère TIM de Ttx (Walden et al. 2004).

Une autre conséquence directe consiste en l'augmentation de la surface de contact entre les deux monomères, et conduit à une valeur extrême pour les complexes permanents (Lo Conte L. et al., 1999) (voir résultats). Le renforcement de cette interaction confirme que la dimérisation est un moyen important de thermostabilisation (Vieille et al., 2001). L'extrême hydrophobicité de cette surface de contact (Lo Conte L. et al., 1999) doit être un important facteur de stabilisation, étant donné que les contacts hydrophobes sont plus favorables à haute température.

Ces boucles supplémentaires impliquées dans le dimère induisent également une modification du volume du dimère. En effet, les monomères de cette protéine hyperthermostable possèdent environ le même volume que les monomères des PTEs mésophiles. Par contre, son dimère est bien plus petit. Ceci est cohérent avec le fait que la minimisation du rapport surface/volume, comme c'est le cas pour cette protéine, peut augmenter la stabilité des protéines simultanément en réduisant l'énergie induite par les surfaces défavorables, tout en augmentant les interactions attractives internes (Sterner et Liebl, 2001).

Le renforcement observé de ce dimère est cohérent avec la préférence des protéines hyperthermostables à s'organiser en oligomères. Ceci a aussi été décrit pour des protéines TIM (Walden et al., 2004).

### 1.3 COMPARAISON DES SEQUENCES

La séquence de cette protéine hyperthermostable montre une composition en acides aminés différente de celles des PTEs mésophiles (Merone et al., 2005). En fait, dans cette protéine de *S*. *solfataricus,* il y a une diminution du contenu en acides aminés polaires non chargés comme Gln, Asn, Thr et Ser. En effet, 50 (15.9% des résidus totaux) d'entre eux sont présents dans la PTE thermostable, alors qu'il y en a 65 et 60 (18.3% et 19.7% des résidus totaux) dans les séquences des PTEs de *P. diminuta* et d'*A. radiobacter,* respectivement. Cette différence est une valeur typique entre des protéines hyperthermophiles et mésophiles (Sterner et Liebl, 2001). Les résidus Gln et Asn sont sujets à la déamination, qui peut être catalysée par les résidus Thr et Ser (Wright, 1991). Ceci signifie que Gln et Asn sont des maillons faibles dans la structure protéiques, à cause de leur tendance à la déamination qui peut conduire au clivage de la chaîne, particulièrement à des températures approchant ou dépassant 90°C. Il semble probable que ces maillons faibles sont protégés ou éliminés dans ces protéines thermostables. En moyenne, la neutralisation de ces points faibles conduit à une réduction globale du nombre de ces résidus dans les séquences hyperthermostables, comme le montrent les analyses statistiques (Szilagyi et Zavodszky, 2000).

Par ailleurs, la séquence de la PTE de *S*. *solfataricus* montre une augmentation du contenu en acides aminés chargés. Elle contient 77 (24.5% des résidus totaux) résidus chargés Asp, Glu, Lys ou Arg, tandis que les deux PTEs mésophiles en contiennent 69 (environ 21 % des résidus totaux). La structure tridimensionnelle montre que l'essentiel de ces résidus chargés est localisé à la surface de la protéine, ce qui correspond à des observations antérieures (Szilagyi et Zavodszky, 2000) sur les protéines hyperthermostables. Ces descriptions reflètent les grandes différences entre les proportions des résidus chargés et celles des résidus non chargés dans ce type de protéines, comme cela avait déjà été déduit par les comparaisons de génome entiers d'organismes hyperthermophiles et mésophiles (Fukuchi et Nishikawa, 2001).

### 1.4 LES INTERACTIONS ELECTROSTATIQUES

Les analyses du nombre total de liaisons hydrogène en utilisant HBPLUS n'ont pas montré de différences significatives entre les PTEs mésophiles et la protéine hyperthermostable. Par contre, la différence de stabilité entre ces protéines est certainement partiellement due au grand nombre de ponts salins observés pour la PTE de *S*. *solfataricus,* comparé aux protéines mésophiles. Les ponts salins sont décrits comme des éléments dominants dans les structures de protéines hyperthermophiles, car ils contribuent à la stabilité thermodynamique des protéines (Sterner et Liebl, 2001). Ainsi, parallèlement à l'augmentation de la thermostabilité des protéines, ces ponts salins ont tendance à s'organiser en réseaux de charges, qui sont le plus souvent retrouvés à la surface de la protéine (Vieille C., 1996), comme observé dans cette structure. Un haut niveau de coopérativité entre les liaisons ioniques semble plus efficace pour la thermostabilité qu'une somme de paires d'ions isolées.

La présence de ce grand nombre de ponts salins et leur organisation en réseaux semblent insuffisante pour expliquer seule l'incroyable thermostabilité de la PTE de *S*. *solfataricus.* En effet, l'augmentation du nombre de ponts salins entre les TIMs hyperthermophiles et leurs homologues mésophiles a seulement été décrite pour le TIM bactérien de *Thermotoga maritima.* Cette stratégie n'est vraisemblablement pas la seule pour atteindre la thermostabilité, étant donné que tous les TIMs d'archae précédemment décrits n'ont pas exploité les liaisons ioniques comme un moyen de thermostabilisation. La PTE de *S*. *solfataricus* est, à notre connaissance, le premier TIM d'archae qui possède un nombre significativement plus important de ponts salins que ses homologues mésophiles.

Les protéines s'adaptent à des conditions extrêmes en conservant leur état fonctionnel, qui est caractérisé par une balance subtile entre stabilité et flexibilité. Etant donné que cette balance est basée sur quelques liaisons hydrogènes, de ponts salins, d'interactions hydrophobes ou de raccourcissement de boucles, la thermostabilisation est évidemment atteinte avec une accumulation de nombreuses et subtiles améliorations à différents endroits de la protéine (Jaenicke, R. 1996). Il est également proposé qu'à température ambiante, les enzymes thermostables se montrent moins flexibles que leurs homologues mésophiles ; par contre, à leur température optimale d'activité, les deux enzymes montrent une flexibilité équivalente.

La structure cristallographique de la PTE de *S*. *solfataricus* montre que sa rigidité accrue est causée par un certain nombre de différences structurales précises avec ses homologues mésophiles. Les déterminants possibles de sa thermostabilité sont les délétions et les stabilisations de régions flexibles ; la compaction et le renforcement du dimère ; et un nombre accru de paires d'ions qui sont en partie organisées en réseaux de charges.

### 2. LE SITE ACTIF

### 2.1 IMPLICATION BIOLOGIQUE

Plusieurs OP-hydrolases ont été isolées et caractérisées. La plus connue d'entre elles est la PTE, qui a été originellement isolée des bactéries du sol. Le substrat naturel de ces enzymes reste toutefois inconnu. Comme la synthèse du plus efficace de leur substrat déterminé jusqu'à présent, à savoir la paraoxon, a été décrite pour la première fois en 1950, il existe quelques controverses concernant l'origine de cette activité. En effet, cela pourrait être une activité due à une proximité structurale de substrat, ou ces enzymes pourraient avoir évoluées spécifiquement contre ces molécules sur cette courte période de temps.

En ce qui concerne la PTE de *S*. *solfataricus,* il semble peu probable que cette protéine ait évolué spécifiquement pour hydrolyser ces insecticides, puisque ces molécules sont très peu présentes dans le biotope de cette archae. De plus, son environnement (environ 90°C) conduirait à une dégradation spontanée extrêmement rapide de ces composés thermolabiles.

La structure de cette enzyme met également en évidence un tunnel hydrophobe qui communique avec le site actif. Ce tunnel, plutôt étroit, pourrait être un indice pour des substrats physiologiques tels que les *N-acyl L*-homoserine lactones. Ce fait serait par ailleurs cohérent avec la découverte de ce gène au milieu d'un cluster de lipases (Merone et al., 2005). La présence de ce type de tunnel hydrophobe est classique pour ce type de substrats et a déjà été décrit (Musayev et al., 2005). Malgré trois prolines, la boucle de neuf résidus à l'origine du tunnel semble plus flexible que le reste de la protéine, avec un facteur d'agitation thermique moyen de 48 Å². Ceci suggère que cette boucle est suffisamment flexible pour une adaptation à la fixation d'un substrat, et suffisamment rigide pour le bon positionnement de ce dernier.

La structure tridimensionnelle révèle aussi la présence dans la cavité du site actif d'un résidu cystéine. Ceci est intéressant, car ce type de résidu est, avec Met, Asn et Gln, considéré comme un acide aminé thermolabile pour des protéines hyperthermophiles. Généralement, il y a une diminution drastique de leur nombre dans ces protéines (Sterner et Liebl, 2001), comme cela est décrit pour Asn et Gln dans cette étude. Cys 258 est très accessible au solvant. La structure cristalline ne montre pas d'activation particulière de ce résidu. Toutefois, la connaissance de la réactivité de cette cystéine apporterait un élément intéressant pour l'établissement du rôle physiologique de cette enzyme.

### 2.2 NOUVEAU MECANISME PROPOSE

Basé sur le mécanisme suggéré par Aubert et al. (2004), nous proposons pour cette nouvelle PTE hyperthermophile un nouveau mécanisme pour la PTE hyperthermophile activée ne faisant pas intervenir le transfert de proton précédemment décrit.

Le centre bi-métallique est utilisé pour activer le substrat pour une attaque nucléophile en polarisant la liaison phosphore-oxygène. L'oxygène du phosphore se fixe sur le métal β, ce qui augmente le caractère électrophile du centre phosphore et facilite l'attaque nucléophile de l'ion hydroxyde. En fait, la fixation de l'oxygène porté par le phosphore sur le métal β pourrait avoir augmenté la réactivité de la molécule d'eau en affaiblissant l'interaction de cette dernière sur le métal β.

Quand le substrat est complexé à ce centre bi-métallique, l'ion hydroxyde attaque le centre phosphore via un mécanisme de type SN2, qui entraîne la formation d'un intermédiaire pentavalent qui ponte les deux métaux. La charge négative qui se développe sur l'oxygène du phosphore est stabilisée par l'interaction avec les métaux, particulièrement le métal β. La paire d'électrons de cet oxygène se rabat sur la liaison phopshore-oxygène, permettant le départ du groupement partant. Le produit phosphore qui ponte les deux métaux est évacué du site actif par une nouvelle molécule d'eau du solvant. Par son pKa, cette molécule phosphorée est déprotonée par le solvant. Aubert et al. (2004) suggèrent l'existence d'un transfert de proton dans le mécanisme de la PTE de *P. diminuta.* Brièvement, dans ce mécanisme, Asp 301 est supposé prendre le proton de l'intermédiaire pentavalent. Ensuite, le proton est évacué du site actif avec l'assistance de His 254 et Asp 233. Nous ne pensons pas que ceci pourrait se produire pour la PTE de *S*. *solfataricus,* car le résidu correspondant à His 254 est une arginine. Cette arginine interagit avec Asp256 via son Nε, ce qui rend plus difficile un éventuel transfert de proton dans le mécanisme de la PTE de *S*. *solfataricus.*

Ce nouveau mécanisme est en fait général pour toutes les PTEs caractérisées. Certains indices contredisent en effet le mécanisme de Aubert et al. La PTE d'*A*. *radiobacter* est une enzyme très active et possède elle aussi une arginine à cette position. De plus, des travaux de mutagenèse réalisés sur la PTE de *P. diminuta* confirment notre hypothèse. Les mutations H254R et H254G ne montrent pas d'effets drastiques sur les paramètres catalytiques de l'enzyme (Grimsley et al., 2005; Hill et al., 2003).

D'autres incohérences peuvent également être notées. Dans la PTE de *P. diminuta,* Asp 301 est en très proche interaction avec le métal α. Ceci implique, pour des considérations électrostatiques, que ce résidu chargé négativement ne doit pas être protoné. En outre, les deux azotes de l'imidazole de His 254 sont en liaisons hydrogènes avec des résidus chargés négativement (Asp 301 et Asp 233). Ceci doit avoir pour effet d'augmenter le pKa de cette histidine, ce qui favorise la forme imidazolium, et suggère que ce résidu n'est pas dans le meilleur environnement pour réaliser la catalyse acide/base proposée dans ce transfert de proton.

En conclusion, l'analyse de cette structure a permis de trouver des éléments expliquant l'incroyable thermostabilité de cette enzyme. En outre, l'analyse de cette structure ainsi qu'un important travail bibliographique a permis de proposer un nouveau mécanisme général d'action pour l'ensemble des PTEs.

Enfin, toutes ces analyses permettent de conduire à la mise au point rapide de biocatalyseurs selon le protocole décrit ci-après.

### II) Procédé de préparation de phosphotriestérases hyperthermophiles mutées possédant une activité lactonase selon l'invention, dérivées de la PTE hyperthermophile de Sulfolobus solfataricus correspondant à la séquence SEQ ID NO : 3,

Afin de préparer le gène synthétique *Ssopox* de 945 paires de bases (bp) *Ssopox* codant pour les phosphotriestérases hyperthermophiles mutées possédant une activité lactonase selon l'invention, dérivées de la PTE hyperthermophile de *Sulfolobus solfataricus* correspondant à la séquence SEQ ID NO : 3, et comprenant l'une au moins des quatre mutations suivantes :
- substitution de la tyrosine Y en position 97 par un tryptophane W,
- substitution de la tyrosine Y en position 99 par une phénylalanine F,
- substitution de l'arginine R en position 223 par une histidine H,
- substitution de la cystéine C en position 258 par une leucine L,
et, le cas échéant, l'une au moins des mutations suivantes :
- substitution de la valine V en position 27 par une alanine A,
- substitution de la proline P en position 67 par une valine V,
- substitution de la thréonine T en position 68 par une sérine S,
- substitution de la leucine L en position 72 par une isoleucine I,
- substitution de l'aspartate D en position 141 par une thréonine T,
- substitution de la glycine G en position 225 par une proline P,
- substitution de la leucine L en position 226 par une histidine H,
- substitution de la phénylalanine F en position 229 par une sérine S,
- substitution du tryptophane W en position 263 par une alanine A,
- substitution du tryptophane W en position 278 par une isoleucine 1, 14 oligonucléotides (7 sens et 7 antisens) ont été utilisés. La taille des oligonucléotides étaient d'environ 90 pb, et chaque amorce chevauche la suivante par une région chevauchante de 27 pb. Les séquences oligonucléotidiques en question dans le sens 5'-3' sont les suivantes :

Les nucléotides choisis pour effectuer les mutations dans la protéine sont indiqués en gras et les régions chevauchantes sont soulignées. Les oligonucléotides **1for** et **14rev** ont également été construits de manière à insérer les sites de restriction *Nde*I et *EcoR*I respectivement aux séquences N-terminale et C-terminale du gène.

Les oligonucléotides ont été purifiés par électrophorèse sur gel d'acrylamide (10%) selon la méthode décrite dans Sambrook (Sambrook J. and Russell D. W. 2001 "Molecular Cloning: a laboratory manual").

La méthode de reconstruction du gène comprend essentiellement 4 étapes. Dans la première étape, des couples successifs d'oligonucléotides (par exemple 1for avec 2rev) sont mélangés (à une concentration finale de 200nM) dans un tube PCR avec un tampon polymérase 1X DeepVent (NEB), 6mM MgSO₄ 200uM dNTP et 1U de Polymérase DeepVent, et placés dans un appareillage à cycle thermique afin d'obtenir des fragments d'environ 160 pb (cycle : 10 min à 95°C, 1 min à 68°C, 10 min à 72°C). Les fragments obtenus sont mélangés (à une concentration de 20nM), puis digérés par la DnaseI (0,0013U; 30s-1.5min à température ambiante. La réaction DnaseI est stoppée par inactivation thermique à 95°C pendant 10 minutes. La troisième étape, ou étape d'assemblage, est un PCR (1min 95°C, 30sec 50°C, 30sec 72°C, 55 fois) afin de combiner les fragments obtenus après digestion par la DnaseI et reconstruire le gène S*sopox* entier, sans addition d'oligos externes.

Dans la dernière étape, ou étape d'amplification, les oligonucléotides externes (5' sens et 3' antisens ssopox; Merone et al., 2005) sont additionnés au mélange, à une concentration finale de 1µM, afin d'amplifier le gène entier (cycle PCR : 1min à 95°C, 30sec à 50°C, 30sec à 72°C).

Un fragment d'environ 950 pb est détectable par passage d'un aliquote sur gel d'agarose 1%. L'ADN est purifié et cloné dans un plasmide pour séquençage.

### BIBLIOGRAPHIE

AFRIAT, L., ROODVELDT, C., MANCO, G. & TAWFIK, D.S. (2006). The latent promiscuity of newly identified microbial lactonases is linked to a recently diverged phosphotriesterase. Biochemistry 45, 1367OE-13686.
AUBERT SD., LI Y., RAUSHEL FM. Mechanism for the hydrolysis of organophosphates by the bacterial phosphotriesterase. Biochemistry. 2004, 43: 5707-15.
BENNING MM., KUO JM., RAUSHEL FM., HOLDEN HM. Three-dimensional structure of phosphotriesterase: an enzyme capable of detoxifying organophosphate nerve agents. Biochemistry. 1994, 33: 15001-7.
BENNING MM., KUO JM., RAUSHEL FM., HOLDEN HM. Three-dimensional structure of the binuclear metal center of phosphotriesterase. Biochemistry. 1995, 34: 7973-8.
BENNING MM., SHIM H., RAUSHEL FM., HOLDEN HM. High resolution X-ray structures of different metal-substituted forms of phosphotriesterase from Pseudomonas diminuta. Biochemistry. 2001, 40: 2712-22
CHEN-GOODSPEED M, SOGORB MA., WU F, HONG SB & RAUSHEL FM. Structural déterminants of the substrate and stereochemical specificity of phosphotriesterase. Biochemistry, 2001, 40, 1325-31
DONG, Y.H., GUSTI, A.R., ZHANG, Q., XU, J.L. & ZHANG, L.H. (2002). Identification of quorum-quenching N-acyl homo serine lactonases from Bacillus species. Appl Environ Microbiol68, 1754-1759.
DONG, Y.H., WANG, L.H., XU, J.L., ZHANG, H.B., ZHANG, X.F. & ZHANG, L.H. (2001). Quenching quorum-sensing-dependent bacterial infection by an N-acyl homo serine lactonase. Nature 411,813-817.
DONG, Y.H., XU, J.L., LI, X.Z. & ZHANG, L.H. (2000). AiiA, an enzyme that inactivates the acylhomoserine lactone quorum-sensing signal and attenuates the virulence of Erwinia carotovora. Proceedings of the National Academy of Sciences of the United States of America 97, 3526-3531.
ELIAS, M., DUPUY, J., MERONE, L., LECOMTE, C., ROSSI, M., MASSON, P., MANCO, G. & CHABRIERE, E. (2007a). Crystallization and preliminary X-ray diffraction analysis of the hyperthermophilic Sulfolobus solfataricus phosphotriesterase. Acta Crystallograph Sect F Struct Biol Cryst Commun 63, 553-555.
ELIAS, M., DUPUY, J., MERONE, L., MANDRICH, L., PORZIO, E., MONIOT, S., ROCHU, D., LECOMTE, C., ROSSI, M., MASSON, P., ET AL. (2007b). Structural basis for natural lactonase and promiscuous phosphotriesterase activities. submitted.
FEDERLE, M.J. & BASSLER, B.L. (2003). Interspecies communication in bacteria. The Journal of clinical investigation 112, 1291-1299.
FUKUCHI S., NISHIKAWA K. Protein surface amino acid compositions distinctively differ between thermophilic and mesophilic bacteria. J. Mol. Biol. 2001, 309: 835-43.
FUQUA, C. & GREENBERG, E.P. (2002). Listening in on bacteria: acyl-homoserine lactone signalling. Nature reviews 3,685-695.
GRIMSLEY JK., CALAMINI B., WILD JR., MESECAR AD. Structural and mutational studies of organophosphorus hydrolase reveal a cryptic and functional allosteric-binding site. Arch Biochem Biophys. 2005,442:169-79.
HAREL M., AHARONI A., GAIDUKOV L., BRUMSHTEIN B., KHERSONSKY O., MEGED R., DVIR H., RAVELLI RB., McCARTHY A., TOKER L., SILMAN I., SUSSMAN JL. & TAWFIK DS. Structure and evolution of the serum paraoxonase family of detoxifying and anti-atherosclerotic enzymes. Nat. Struct. Mol. Biol. 2004, 11:412-9.
HILL CM., LI WS., THODEN JB., HOLDEN HM., RAUSHEL FM. Enhanced degradation of chemical warfare agents through molecular engineering of the phosphotriesterase active site. J. Am. Chem. Soc. 2003, 125 : 8990-1.
JACKSON CJ., CARR PD., KIM HK., LIU JW., HERRALD P., MITIC N., SCHENK G., SMITH CA, OLLIS DL. Anomalous scattering analysis of Agrobacterium radiobacter phosphotriesterase: the prominent role of iron in the heterobinuclear active site. Biochem. J. 2006 May 11
JACKSON CJ., LIU JW., COOTE ML., OLLIS DL. The effects of substrate orientation on the mechanism of a phosphotriesterase. Org. Biomol. Chem. 2005, 3: 4343-50.
JAENICKE R. Glyceraldehyde-3-phosphate dehydrogenase from Thermotoga maritima: strategies of protein stabilization. FEMS Microbiol Rev. 1996, 18: 215-24.
LIN, Y.H., XL1, J.L., HU, J., WANG, L.H., ONG, S.L., LEADBETTER, J.R. & ZHANG, L.H. Acyl-homoserine lactone acylase from Ralstonia strain XJ12B represents a novel and pote nt class of quorum-quenching enzymes. Molecular microbiology 47, 849-860 (2003).
LO CONTE L., CHOTHIA C., JANIN J. The atomic structure of protein-protein recognition sites. J. Mol. Biol. 1999,285: 2177-98.
MERONE L., MANDRICH L., ROSSI M., MANCO G. A thermostable phosphotriesterase from the archaeon Sulfolobus solfataricus: cloning, overexpression and properties. Extremophiles. 2005, 9: 297-305.
MERONE, L., MANDRICH, L., ROSSI, M. & MANCO, G. A thermostable phosphotriesterase from the archaeon Sulfolobus solfataricus: cloning, overexpression and properties. Extremophiles 9(2005), 297-305.
MUNNECKE DM. Enzymatic hydrolysis of organophosphate insecticides, a possible pesticide disposal method. Appl. Environ. Microbiol. 1976, 32:7-13.
MUSAYEV F., SACHDEVA S., SCARSDALE JN., REYNOLDS KA., WRIGHT HT. Crystal structure of a substrate complex of Mycobacterium tuberculosis beta-ketoacyl-acyl carrier protein synthase III (FabH) with lauroyl-coenzyme A. J. Mol. Biol. 2005, 346: 1313-21.
OH, K.B., MIYAZAWA, H., NAITO, T. & MATSUOKA, H. (2001). Purification and characterization of an autoregulatory substance capable of regulating the morphological transition in Candida albicans. Proceedings of the National Academy of Sciences of the United States of America 98, 4664-4668.
PASSADOR, L., COOK, J.M., GAMBEILO, M.J., RUST, L. & IGLEWSKI, RH. Expression of Pseudomonas aeruginosa virulence genes requires cell-to-cell communication. Science (New York, N.Y) 260, 1127-1130 (1993).
PIRHONEN, M., FLEGO, D., HEIKINHEIMO, R. & PAL VA, E.T. A small diffusible signal molecule is responsible for the global control of virulence and exoenzyme production in the plant pathogen Erwinia carotovora. The EMBO journal 12 (1993), 2467-2476.
PORZIO E. MERONE L. MANDRICH L. ROSSI M. MANCO G. A new phosphotriesterase from Sulfolobus acidocaldarius and its comparison with the homologue from Sulfolobus solfataricus. Biochimie.2007
RASMUSSEN, T.B. & GIVSKOV, M. Quorum-sensing inhibitors as anti-pathogenic drugs. Int J Med Microbiol 296, 149-161 (2006).
RAVEH L., GRUNWALD J., MARCUS D., PAPIER Y., COHEN E., ASHANI Y. Human butyrylcholinesterase as a général prophylactic antidote for nerve agent toxicity. In vitro and in vivo quantitative characterization. Biochem. Pharmacol., 1993, 45:2465-74
REIMMANN, C., GINET, N., MICHEL, L., KEEL, C., MICHAUX, P., KRISHNAPILLAI, V., ZALA, M., HEURLIER, K., TRIANDAFILLU, K., HARMS, H., ET AL. Genetically programmed autoinducer destruction reduces virulence gene expression and swarming motility in Pseudomonas aeruginosa PAO1. Microbiology (Reading, England) 148, 923-932 (2002).
SAMPLES CR., HOWARD T., RAUSHEL FM., DeROSE VJ. Protonation of the binuclear metal center within the active site of phosphotriesterase. Biochemistry. 2005, 44 :11005-13.
STERNER R, LIEBL W. Thermophilic adaptation of proteins. Crit. Rev. Biochem. Mol. Biol. 2001, 36:39-106.
SZILAGYI A., ZAVODSZKY P. Structural differences between mesophilic, moderately thermophilic and extremely thermophilic protein subunits: results of a comprehensive survey. Structure. 2000, 8: 493-504.
VANHOOKE JL., BENNING MM., RAUSHEL FM., HOLDEN HM. Three-dimensional structure of the zinc-containing phosphotriesterase with the bound substrate analog diethyl 4-methylbenzylphosphonate. Biochemistry. 1996 ; 35 :6020-5.
VIEILLE C. & ZEIKUS GJ. Hyperthermophilic enzymes: sources, uses, and molecular mechanisms for thermostability. Microbiol Mol Biol Rev. 2001, 65 :1-43.
VIEILLE C., BURDETTE DS., ZEIKUS JG. Thermozymes. Biotechnol Annu Rev. 1996, 2 :1-83.
WALDEN H. TAYLOR GL., LORENTZEN E., POHL E., LILIE H., SCHRAMM A., KNURA T., STUBBE K., TJADEN B., HENSELR. Structure and function of a regulated archaeal triosephosphate isomerase adapted to high temperature. J. Mol. Biol. 2004, 342: 861-75.
WHITEHEAD, N.A., BARNARD, A.M., SLATER, H., SIMPSON, N.J. & SALMOND, A.P. Quorum-sensing in Gram-negative bacteria. FEMS microbiology reviews 25, 365-404 (2001).
WRIGHT HT. Sequence and structure determinants of the nonenzymatic deamidation of asparagine and glutamine residues in proteins. Protein Eng. 1991, 4 : 283-94.
ZHANG, L.H. Quorum quenching and proactive host defense. Trends in plant science 8, 238-244 (2003).

## Revendications

1. Phosphotriestérases (PTE) hyperthermophiles mutées possédant une activité lactonase dérivées des PTE hyperthermophiles répondant à la séquence consensus SEQ ID NO : 1, et comprenant l'une au moins des quatre mutations suivantes :
substitution de la tyrosine Y en position 98,
substitution de la tyrosine Y en position 100,
substitution de l'arginine R en position 224,
substitution de la cystéine C en position 259,
de SEQ ID NO : 1 par tout autre aminoacide naturel ou non,
lesdites phosphotriestérases (PTE) hyperthermophiles mutées possédant une activité lactonase dont l'activité est supérieure à celle des phophotriestérases (PTE) hyperthermophiles non mutées dont elles dérivent.

2. Phosphotriestérases hyperthermophiles mutées possédant une activité lactonase selon la revendication 1, dérivées de la PTE hyperthermophile de *Sulfolobus solfataricus* correspondant à la séquence SEQ ID NO : 3, ou de la PTE hyperthermophile de *Sulfolobus acidocaldarius* correspondant à la séquence SEQ ID NO : 5, lesdites séquences SEQ ID NO : 3 et SEQ ID NO : 5 appartenant à la séquence consensus SEQ ID NO : 1, l'aminoacide en position 2 dans SEQ ID NO : 1 étant manquant dans SEQ ID NO : 3.

3. Phosphotriestérases hyperthermophiles mutées possédant une activité lactonase selon la revendication 1 ou 2, comprenant au moins les quatre mutations suivantes :
substitution de la tyrosine Y en position 98,
substitution de la tyrosine Y en position 100,
substitution de l'arginine R en position 224,
substitution de la cystéine C en position 259,
de SEQ ID NO : 1 par tout autre aminoacide naturel ou non.

4. Phosphotriestérases hyperthermophiles mutées possédant une activité lactonase selon l'une des revendications 1 à 3, **caractérisées en ce qu'**elles comprennent également l'une au moins des mutations suivantes :
substitution de la valine V en position 28,
substitution de la proline P en position 68,
substitution de la thréonine T en position 69,
substitution de la leucine L en position 73,
substitution de l'aspartate D en position 142,
substitution de la glycine G en position 226,
substitution de la leucine L en position 227,
substitution de la phénylalanine F en position 230,
substitution du tryptophane W en position 264,
substitution du tryptophane W en position 279,
de SEQ ID NO : 1 par tout autre aminoacide naturel ou non.

5. Phosphotriestérases hyperthermophiles mutées possédant une activité lactonase selon l'une des revendications 1 à 4, **caractérisées en ce qu'**elles comprennent les cinq mutations suivantes :
substitution de la valine V en position 28,
substitution de la leucine L en position 73,
substitution de l'aspartate D en position 142,
substitution de la glycine G en position 226,
substitution de la leucine L en position 227,
de SEQ ID NO : 1 par tout autre aminoacide naturel ou non.

6. Phosphotriestérases hyperthermophiles mutées possédant une activité lactonase selon l'une des revendications 1 à 5, **caractérisées en ce qu'**elles comprennent les cinq mutations suivantes :
substitution de la proline P en position 68,
substitution de la thréonine T en position 69,
substitution de la phénylalanine F en position 230,
substitution du tryptophane W en position 264,
substitution du tryptophane W en position 279,
de SEQ ID NO : 1 par tout autre aminoacide naturel ou non.

7. Phosphotriestérases hyperthermophiles mutées possédant une activité lactonase selon l'une des revendications 1 à 4, dérivées des PTE hyperthermophiles répondant à la séquence consensus SEQ ID NO : 1, **caractérisées en ce qu'**elles comprennent l'une au moins des quatre mutations suivantes :
substitution de la tyrosine Y en position 98 par un tryptophane W,
substitution de la tyrosine Y en position 100 par une phénylalanine F,
substitution de l'arginine R en position 224 par une histidine H,
substitution de la cystéine C en position 259 par une leucine L,
et, le cas échéant, l'une au moins des mutations suivantes :
s ubstitution de la valine V en position 28 par une alanine A,
s ubstitution de la proline P en position 68 par une valine V,
s ubstitution de la thréonine T en position 69 par une sérine S,
s ubstitution de la leucine L en position 73 par une isoleucine I,
s ubstitution de l'aspartate D en position 142 par une thréonine T,
s ubstitution de la glycine G en position 226 par une proline P,
s ubstitution de la leucine L en position 227 par une histidine H,
s ubstitution de la phénylalanine F en position 230 par une sérine S,
s ubstitution du tryptophane W en position 264 par une alanine A,
s ubstitution du tryptophane W en position 279 par une isoleucine I.

8. Phosphotriestérases hyperthermophiles mutées possédant une activité lactonase selon l'une des revendications 1 à 7, dérivées de la PTE hyperthermophile de *Sulfolobus solfataricus* correspondant à la séquence SEQ ID NO : 3, et comprenant l'une au moins des quatre mutations suivantes :
substitution de la tyrosine Y en position 97,
substitution de la tyrosine Y en position 99,
substitution de l'arginine R en position 223,
substitution de la cystéine C en position 258,
de SEQ ID NO : 3 par tout autre aminoacide naturel ou non.

9. Phosphotriestérases hyperthermophiles mutées possédant une activité lactonase selon la revendication 8, comprenant au moins les quatre mutations suivantes :
substitution de la tyrosine Y en position 97,
substitution de la tyrosine Y en position 99,
substitution de l'arginine R en position 223,
substitution de la cystéine C en position 258,
de SEQ ID NO : 3 par tout autre aminoacide naturel ou non.

10. Phosphotriestérases hyperthermophiles mutées possédant une activité lactonase selon la revendication 8 ou 9, **caractérisées en ce qu'**elles comprennent également l'une au moins des mutations suivantes :
substitution de la valine V en position 27,
substitution de la proline P en position 67,
substitution de la thréonine T en position 68,
substitution de la leucine L en position 72,
substitution de l'aspartate D en position 141,
substitution de la glycine G en position 225,
substitution de la leucine L en position 226,
substitution de la phénylalanine F en position 229,
substitution du tryptophane W en position 263,
substitution du tryptophane W en position 278,
de SEQ ID NO : 3 par tout autre aminoacide naturel ou non.

11. Phosphotriestérases hyperthermophiles mutées possédant une activité lactonase selon l'une des revendications 8 à 10, **caractérisées en ce qu'**elles comprennent les cinq mutations suivantes :
substitution de la valine V en position 27,
substitution de la leucine L en position 72,
substitution de l'aspartate D en position 141,
substitution de la glycine G en position 225,
substitution de la leucine L en position 226,
de SEQ ID NO : 3 par tout autre aminoacide naturel ou non.

12. Phosphotriestérases hyperthermophiles mutées possédant une activité lactonase selon l'une des revendications 8 à 11, **caractérisées en ce qu'**elles comprennent les cinq mutations suivantes :
substitution de la proline P en position 67,
substitution de la thréonine T en position 68,
substitution de la phénylalanine F en position 229,
substitution du tryptophane W en position 263,
substitution du tryptophane W en position 278,
de SEQ ID NO : 3 par tout autre aminoacide naturel ou non.

13. Phosphotriestérases hyperthermophiles mutées possédant une activité lactonase selon l'une des revendications 8 à 12, **caractérisées en ce qu'**elles comprennent l'une au moins des quatre mutations suivantes :
substitution de la tyrosine Y en position 97 par un tryptophane W,
substitution de la tyrosine Y en position 99 par une phénylalanine F,
substitution de l'arginine R en position 223 par une histidine H,
substitution de la cystéine C en position 258 par une leucine L,
et, le cas échéant, l'une au moins des mutations suivantes :
s ubstitution de la valine V en position 27 par une alanine A,
s ubstitution de la proline P en position 67 par une valine V,
s ubstitution de la thréonine T en position 68 par une sérine S,
s ubstitution de la leucine L en position 72 par une isoleucine I,
s ubstitution de l'aspartate D en position 141 par une thréonine T,
s ubstitution de la glycine G en position 225 par une proline P,
s ubstitution de la leucine L en position 226 par une histidine H,
s ubstitution de la phénylalanine F en position 229 par une sérine S,
s ubstitution du tryptophane W en position 263 par une alanine A,
s ubstitution du tryptophane W en position 278 par une isoleucine I.

14. Phosphotriestérases hyperthermophiles mutées possédant une activité lactonase selon l'une des revendications 8 à 13, **caractérisées en ce qu'**elles correspondent aux séquences suivantes :
SEQ ID NO : 7 correspondant à la séquence SEQ ID NO : 3 comprenant les quatre mutations suivantes :
substitution de la tyrosine Y en position 97 par un tryptophane W,
substitution de la tyrosine Y en position 99 par une phénylalanine F,
substitution de l'arginine R en position 223 par une histidine H,
substitution de la cystéine C en position 258 par une leucine L,
SEQ ID NO : 9 correspondant à la séquence SEQ ID NO : 7 comprenant en plus les cinq mutations suivantes :
substitution de la valine V en position 27 par une alanine A,
substitution de la leucine L en position 72 par une isoleucine I,
substitution de l'aspartate D en position 141 par une thréonine T,
substitution de la glycine G en position 225 par une proline P,
substitution de la leucine L en position 226 par une histidine H,
SEQ ID NO : 11 correspondant à la séquence SEQ ID NO : 9 comprenant en plus les cinq mutations suivantes :
substitution de la proline P en position 67 par une valine V,
substitution de la thréonine T en position 68 par une sérine S,
substitution de la phénylalanine F en position 229 par une sérine S,
substitution du tryptophane W en position 263 par une alanine A,
substitution du tryptophane W en position 278 par une isoleucine I.

15. Phosphotriestérases hyperthermophiles mutées possédant une activité lactonase selon l'une des revendications 8 à 14, **caractérisées en ce qu'**elles comprennent au moins une mutation correspondant à la substitution de l'un au moins des aminoacides des couples d'aminoacides suivants dont les positions dans SEQ ID NO : 3 sont indiquées ci-après par un autre aminoacide naturel ou non : 2R/314S, 14K/12E, 26R/75D, 26R/42E, 33R/42E, 33R/45E, 55R/52E, 55R/285E, 74R/121D, 81K/42E, 81K/43D, 84K/80E, 109R/113E, 123K/162E, 147K/148D, 151K/148D, 154R/150E, 154R/187E, 154R/188E, 161K/188E, 183R/150E, 183R/187E, 183R/180E, 210K/245D, 215K/214D, 223R/256D, 223R/202D, 234K/204D, 235R/202D, 241R/245D, 245D/244K, 250K/249D, 277R/286D, 292K/298E, 310K/307E.

16. Phosphotriestérases hyperthermophiles mutées possédant une activité lactonase selon l'une des revendications 1 à 7, dérivées de la PTE hyperthermophile de *Sulfolobus acidocaldarius* correspondant à la séquence SEQ ID NO : 5, et comprenant l'une au moins des quatre mutations suivantes :
substitution de la tyrosine Y en position 98,
substitution de la tyrosine Y en position 100,
substitution de l'arginine R en position 224,
substitution de la cystéine C en position 259,
de SEQ ID NO : 5 par tout autre aminoacide naturel ou non.

17. Phosphotriestérases hyperthermophiles mutées possédant une activité lactonase selon la revendication 16, comprenant au moins les quatre mutations suivantes :
substitution de la tyrosine Y en position 98,
substitution de la tyrosine Y en position 100,
substitution de l'arginine R en position 224,
substitution de la cystéine C en position 259,
de SEQ ID NO : 5 par tout autre aminoacide naturel ou non.

18. Phosphotriestérases hyperthermophiles mutées possédant une activité lactonase selon la revendication 16 ou 17, **caractérisées en ce qu'**elles comprennent également l'une au moins des mutations suivantes :
substitution de la valine V en position 28,
substitution de la proline P en position 68,
substitution de la thréonine T en position 69,
substitution de la leucine L en position 73,
substitution de l'aspartate D en position 142,
substitution de la glycine G en position 226,
substitution de la leucine L en position 227,
substitution de la phénylalanine F en position 230,
substitution du tryptophane W en position 264,
substitution du tryptophane W en position 279,
de SEQ ID NO : 5 par tout autre aminoacide naturel ou non.

19. Phosphotriestérases hyperthermophiles mutées possédant une activité lactonase selon l'une des revendications 16 à 18, **caractérisées en ce qu'**elles comprennent les cinq mutations suivantes :
substitution de la valine V en position 28,
substitution de la leucine L en position 73,
substitution de l'aspartate D en position 142,
substitution de la glycine G en position 226,
substitution de la leucine L en position 227,
de SEQ ID NO : 5 par tout autre aminoacide naturel ou non.

20. Phosphotriestérases hyperthermophiles mutées possédant une activité lactonase selon l'une des revendications 16 à 19, **caractérisées en ce qu'**elles comprennent les cinq mutations suivantes :
substitution de la proline P en position 68,
substitution de la thréonine T en position 69,
substitution de la phénylalanine F en position 230,
substitution du tryptophane W en position 264,
substitution du tryptophane W en position 279,
de SEQ ID NO : 5 par tout autre aminoacide naturel ou non.

21. Phosphotriestérases hyperthermophiles mutées possédant une activité lactonase selon l'une des revendications 16 à 20, **caractérisées en ce qu'**elles comprennent l'une au moins des quatre mutations suivantes :
substitution de la tyrosine Y en position 98 par un tryptophane W,
substitution de la tyrosine Y en position 100 par une phénylalanine F,
substitution de l'arginine R en position 224 par une histidine H,
substitution de la cystéine C en position 259 par une leucine L,
et, le cas échéant, l'une au moins des mutations suivantes :
s ubstitution de la valine V en position 28 par une alanine A,
s ubstitution de la proline P en position 68 par une valine V,
s ubstitution de la thréonine T en position 69 par une sérine S,
s ubstitution de la Ïeucine L en position 73 par une isoleucine I,
s ubstitution de l'aspartate D en position 142 par une thréonine T,
s ubstitution de la glycine G en position 226 par une proline P,
s ubstitution de la leucine L en position 227 par une histidine H,
s ubstitution de la phénylalanine F en position 230 par une sérine S,
s ubstitution du tryptophane W en position 264 par une alanine A,
s ubstitution du tryptophane W en position 279 par une isoleucine I.

22. Phosphotriestérases hyperthermophiles mutées possédant une activité lactonase selon l'une des revendications 16 à 21, **caractérisées en ce qu'**elles correspondent aux séquences suivantes :
SEQ ID NO : 13 correspondant à la séquence SEQ ID NO : 5 comprenant les quatre mutations suivantes :
substitution de la tyrosine Y en position 98 par un tryptophane W,
substitution de la tyrosine Y en position 100 par une phénylalanine F,
substitution de l'arginine R en position 224 par une histidine H,
substitution de la cystéine C en position 259 par une leucine L,
SEQ ID NO : 15 correspondant à la séquence SEQ ID NO : 13 comprenant en plus les cinq mutations suivantes :
substitution de la valine V en position 28 par une alanine A,
substitution de la leucine L en position 73 par une isoleucine I,
substitution de l'aspartate D en position 142 par une thréonine T,
substitution de la glycine G en position 226 par une proline P,
substitution de la leucine L en position 227 par une histidine H,
SEQ ID NO : 17 correspondant à la séquence SEQ ID NO : 15 comprenant en plus les cinq mutations suivantes :
substitution de la proline P en position 68 par une valine V,
substitution de la thréonine T en position 69 par une sérine S,
substitution de la phénylalanine F en position 230 par une sérine S,
substitution du tryptophane W en position 264 par une alanine A,
substitution du tryptophane W en position 279 par une isoleucine I.

23. Phosphotriestérases hyperthermophiles mutées possédant une activité lactonase selon l'une quelconque des revendications 1 à 22, dans lesquelles au moins l'un des acides aminés impliqués dans les ponts salins est muté par addition, substitution ou délétion, de telle sorte que la température d'activation des dites phosphotriestérases hyperthermophiles mutées possédant une activité lactonase soit diminuée par rapport à la température d'activation des phosphotriestérases hyperthermophiles mutées possédant une activité lactonase dans lesquels les acides aminés impliqués dans les ponts salins sont non mutés.

24. Séquences nucléotidiques codant pour les phosphotriestérases hyperthermophiles mutées possédant une activité lactonase telles que définies dans l'une des revendications 1 à 23.

25. Vecteurs, notamment plasmides, contenant des séquences nucléotidiques codant pour les phosphotriestérases hyperthermophiles mutées possédant une activité lactonase telles que définies dans la revendication 24.

26. Cellules hôtes, notamment bactéries, transformées à l'aide d'un vecteur tel que défini dans la revendication 25, avec le proviso que les cellules hôtes ne sont pas des cellules souches embryonnaires humaines.

27. Cellules hôtes, notamment bactéries, couplées aux phosphotriestérases hyperthermophiles mutées possédant une activité lactonase telles que définies dans l'une des revendications 1 à 23, ou présantant des phosphotriestérases hyperthermophiles mutées possédant une activité lactonase telles que définies dans l'une des revendications 1 à 23, greffées sur leur surface, avec le proviso que les cellule hôtes ne sont pas des cellules souches embryonnaires humaines.

28. Organismes transgéniques, notamment mammifères, transformées à l'aide d'un vecteur tel que défini dans la revendication 25, lesdits organismes transgéniques étant résistants aux pathogènes, avec le proviso que les organismes transgéniques ne sont pas des êtres humains.

29. Utilisation de phosphotriestérases hyperthermophiles mutées possédant une activité lactonase selon l'une des revendications 1 à 23, ou de cellules hôtes selon la revendication 26 ou 27,
en tant que bio-épurateurs dans le cadre de la décontamination des surfaces des matériaux, contaminés par des composés organophosphorés, ou des bactéries,
ou pour la préparation de médicaments dans le cadre de la décontamination de la peau, ou des muqueuses, contaminés par des composés organophosphorés, ou des bactéries, ou dans le cadre de la préparation de médicaments utilisables dans le cadre de la prévention ou du traitement d'une contamination externe ou d'une intoxication interne par ingestion ou inhalation par des composés organophosphorés,
ou dans le cadre de la préparation de médicaments utilisables dans le cadre de la prévention ou du traitement d'une infection bactérienne,
ou dans le cadre de la dépollution des eaux polluées par des composés organophosphorés,
ou dans le cadre de la destruction des stocks de neurotoxiques.

30. Matériaux imprégnés de phosphotriestérases hyperthermophiles mutées possédant une activité lactonase selon l'une des revendications 1 à 23, sous forme liquide ou solide, tels que gants, vêtements divers, lingettes, mousses pulvérisables.

31. Kits pour la décontamination des surfaces des matériaux, de la peau, ou des muqueuses, contaminés par des composés organophosphorés, ou pour la dépollution des eaux polluées par des composés organophosphorés, **caractérisés en ce qu'**ils comprennent des phosphotriestérases hyperthermophiles mutées possédant une activité lactonase telles que définies dans l'une des revendications 1 à 23, ou des matériaux imprégnés de phosphotriestérases hyperthermophiles mutées possédant une activité lactonase selon la revendication 29.

32. Capteurs des composés organophosphorés comprenant des phosphotriestérases hyperthermophiles mutées possédant une activité lactonase telles que définies dans l'une des revendications 1 à 23.

33. Cartouches de décontamination externe, à l'intérieur desquelles sont greffées des phosphotriestérases hyperthermophiles mutées possédant une activité lactonase telles que définies dans l'une des revendications 1 à 23.

34. Composition pharmaceutique **caractérisée en ce qu'**elle comprend des phosphotriestérases hyperthermophiles mutées possédant une activité lactonase selon l'une des revendications 1 à 23, en association avec un véhicule pharmaceutiquement acceptable.

35. Composition pharmaceutique selon la revendication 34, **caractérisée en ce qu'**elle se présente sous une forme administrable par voie injectable, notamment en solution ou encapsidées ou peggylées, ou par voie topique, notamment sous forme de pommade, aérosol ou lingettes.

36. Utilisation de matériaux imprégnés selon la revendication 29, ou de cartouches de décontaminations externes selon la revendication 32, pour la préparation de médicaments antiseptiques pour la décontamination de l'infection bactérienne de surface.

37. Utilisation de la composition pharmaceutique selon la revendication 33 ou 34, pour la préparation d'un médicament destiné au traitement des infections bactériennes, notamment dans le sang.

## Patentansprüche

1. Eine Lactonaseaktivität aufweisende, mutierte hyperthermophile Phosphotriesterasen (PTE), die von hyperthermophilen PTEs abgeleitet sind, die der Consensus-Sequenz SEQ ID NO: 1 entsprechen und die mindestens eine der vier folgenden Mutationen umfassen:
Substitution von Tyrosin Y an Position 98,
Substitution von Tyrosin Y an Position 100,
Substitution von Arginin R an Position 224,
Substitution von Cystein C an Position 259
von SEQ ID NO: 1 mit jeder anderen natürlichen oder nicht natürlichen Aminosäure,
wobei die eine Lactonaseaktivität aufweisenden, mutierten hyperthermophilen Phosphotriesterasen (PTE) eine höhere Aktivität aufweisen als die nicht mutierten hyperthermophilen Phosphotriesterasen (PTE), von denen sie abgeleitet sind.

2. Eine Lactonaseaktivität aufweisende, mutierte hyperthermophile Phosphotriesterasen nach Anspruch 1, die von der hyperthermophilen PTE von *Sulfolobus solfataricus,* die der Sequenz SEQ ID NO: 3 entspricht, oder von der hyperthermophilen PTE von *Sulfolobus acidocaldarius,* die der Sequenz SEQ ID NO: 5 entspricht, abgeleitet sind, wobei die Sequenzen SEQ ID NO: 3 und SEQ ID NO: 5 zur Consensus-Sequenz SEQ ID NO: 1 gehören, wobei die in SEQ ID NO: 1 an Position 2 befindliche Aminosäure in SEQ ID NO: 3 fehlt.

3. Eine Lactonaseaktivität aufweisende, mutierte hyperthermophile Phosphotriesterasen nach Anspruch 1
oder 2, die mindestens die vier folgenden Mutationen umfassen:
Substitution von Tyrosin Y an Position 98,
Substitution von Tyrosin Y an Position 100,
Substitution von Arginin R an Position 224,
Substitution von Cystein C an Position 259
von SEQ ID NO: 1 mit jeder anderen natürlichen oder nicht natürlichen Aminosäure.

4. Eine Lactonaseaktivität aufweisende, mutierte, hyperthermophile Phosphotriesterasen nach einem der Ansprüche 1 bis 3, **dadurch gekennzeichnet, dass** sie mindestens auch eine der folgenden Mutationen umfassen:
Substitution von Valin V an Position 28,
Substitution von Prolin P an Position 68,
Substitution von Threonin T an Position 69,
Substitution von Leucin L an Position 73,
Substitution von Aspartat D an Position 142,
Substitution von Glycin G an Position 226,
Substitution von Leucin L an Position 227,
Substitution von Phenylalanin F an Position 230,
Substitution von Tryptophan W an Position 264,
Substitution von Tryptophan W an Position 279
von SEQ ID NO: 1 mit jeder anderen natürlichen oder nicht natürlichen Aminosäure.

5. Eine Lactonaseaktivität aufweisende, mutierte, hyperthermophile Phosphotriesterasen nach einem der Ansprüche 1 bis 4, **dadurch gekennzeichnet, dass** sie die folgenden fünf Mutationen umfassen:
Substitution von Valin V an Position 28,
Substitution von Leucin L an Position 73,
Substitution von Aspartat D an Position 142,
Substitution von Glycin G an Position 226,
Substitution von Leucin L an Position 227
von SEQ ID NO: 1 mit jeder anderen natürlichen oder nicht natürlichen Aminosäure.

6. Eine Lactonaseaktivität aufweisende, mutierte, hyperthermophile Phosphotriesterasen nach einem der Ansprüche 1 bis 5, **dadurch gekennzeichnet, dass** sie die folgenden fünf Mutationen umfassen:
Substitution von Prolin P an Position 68,
Substitution von Threonin T an Position 69,
Substitution von Phenylalanin F an Position 230,
Substitution von Tryptophan W an Position 264,
Substitution von Tryptophan W an Position 279
von SEQ ID NO: 1 mit jeder anderen natürlichen oder nicht natürlichen Aminosäure.

7. Eine Lactonaseaktivität aufweisende, mutierte hyperthermophile Phosphotriesterasen nach einem der Ansprüche 1 bis 4, die von den hyperthermophilen PTEs abgeleitet sind, die der Consensus-Sequenz SEQ ID NO: 1 entsprechen, **dadurch gekennzeichnet, dass** sie mindestens eine der vier folgenden Mutationen:
Substitution von Tyrosin Y an Position 98 durch ein Tryptophan W,
Substitution von Tyrosin Y an Position 100 durch ein Phenylalanin F,
Substitution von Arginin R an Position 224 durch ein Histidin H,
Substitution von Cystein C an Position 259 durch ein Leucin L,
und gegebenenfalls mindestens eine der folgenden Mutationen umfassen:
Substitution von Valin V an Position 28 durch ein Alanin A,
Substitution von Prolin P an Position 68 durch ein Valin V,
Substitution von Threonin T an Position 69 durch ein Serin S,
Substitution von Leucin L an Position 73 durch ein Isoleucin I,
Substitution von Aspartat D an Position 142 durch ein Threonin T,
Substitution von Glycin G an Position 226 durch ein Prolin P,
Substitution von Leucin L an Position 227 durch ein Histidin H,
Substitution von Phenylalanin F an Position 230 durch ein Serin S,
Substitution von Tryptophan W an Position 264 durch ein Alanin A,
Substitution von Tryptophan W an Position 279 durch ein Isoleucin I.

8. Eine Lactonaseaktivität aufweisende, mutierte hyperthermophile Phosphotriesterasen nach einem der Ansprüche 1 bis 7, die von der hyperthermophilen PTE von *Sulfolobus solfataricus* abgeleitet sind, die der Sequenz SEQ ID NO: 3 entspricht und mindestens eine der vier folgenden Mutationen umfasst:
Substitution von Tyrosin Y an Position 97,
Substitution von Tyrosin Y an Position 99,
Substitution von Arginin R an Position 223,
Substitution von Cystein C an Position 258
von SEQ ID NO: 3 mit jeder anderen natürlichen oder nicht natürlichen Aminosäure.

9. Eine Lactonaseaktivität aufweisende, mutierte hyperthermophile Phosphotriesterasen nach Anspruch 8, die mindestens die vier folgenden Mutationen umfassen:
Substitution von Tyrosin Y an Position 97,
Substitution von Tyrosin Y an Position 99,
Substitution von Arginin R an Position 223,
Substitution von Cystein C an Position 258
von SEQ ID NO: 3 mit jeder anderen natürlichen oder nicht natürlichen Aminosäure.

10. Eine Lactonaseaktivität aufweisende, mutierte, hyperthermophile Phosphotriesterasen nach Anspruch 8 oder 9, **dadurch gekennzeichnet, dass** sie mindestens auch eine der folgenden Mutationen umfassen:
Substitution von Valin V an Position 27,
Substitution von Prolin P an Position 67,
Substitution von Threonin T an Position 68,
Substitution von Leucin L an Position 72,
Substitution von Aspartat D an Position 141,
Substitution von Glycin G an Position 225,
Substitution von Leucin L an Position 226,
Substitution von Phenylalanin F an Position 229,
Substitution von Tryptophan W an Position 263,
Substitution von Tryptophan W an Position 278
von SEQ ID NO: 3 mit jeder anderen natürlichen oder nicht natürlichen Aminosäure.

11. Eine Lactonaseaktivität aufweisende, mutierte, hyperthermophile Phosphotriesterasen nach einem der Ansprüche 8 bis 10, **dadurch gekennzeichnet, dass** sie die folgenden fünf Mutationen umfassen:
Substitution von Valin V an Position 27,
Substitution von Leucin L an Position 72,
Substitution von Aspartat D an Position 141,
Substitution von Glycin G an Position 225,
Substitution von Leucin L an Position 226
von SEQ ID NO: 3 mit jeder anderen natürlichen oder nicht natürlichen Aminosäure.

12. Eine Lactonaseaktivität aufweisende, mutierte, hyperthermophile Phosphotriesterasen nach einem der Ansprüche 8 bis 11, **dadurch gekennzeichnet, dass** sie die folgenden fünf Mutationen umfassen:
Substitution von Prolin P an Position 67,
Substitution von Threonin T an Position 68,
Substitution von Phenylalanin F an Position 229,
Substitution von Tryptophan W an Position 263,
Substitution von Tryptophan W an Position 278
von SEQ ID NO: 3 mit jeder anderen natürlichen oder nicht natürlichen Aminosäure.

13. Eine Lactonaseaktivität aufweisende, mutierte, hyperthermophile Phosphotriesterasen nach einem der Ansprüche 8 bis 12, **dadurch gekennzeichnet, dass** sie mindestens eine der folgenden vier Mutationen:
Substitution von Tyrosin Y an Position 97 durch ein Tryptophan W,
Substitution von Tyrosin Y an Position 99 durch ein Phenylalanin F,
Substitution von Arginin R an Position 223 durch ein Histidin H,
Substitution von Cystein C an Position 258 durch ein Leucin L
und gegebenenfalls mindestens eine der folgenden Mutationen umfassen:
Substitution von Valin V an Position 27 durch ein Alanin A,
Substitution von Prolin P an Position 67 durch ein Valin V,
Substitution von Threonin T an Position 68 durch ein Serin S,
Substitution von Leucin L an Position 72 durch ein Isoleucin I,
Substitution von Aspartat D an Position 141 durch ein Threonin T,
Substitution von Glycin G an Position 225 durch ein Prolin P,
Substitution von Leucin L an Position 226 durch ein Histidin H,
Substitution von Phenylalanin F an Position 229 durch ein Serin S,
Substitution von Tryptophan W an Position 263 durch ein Alanin A,
Substitution von Tryptophan W-an Position 278 durch ein Isoleucin I.

14. Eine Lactonaseaktivität aufweisende, mutierte, hyperthermophile Phosphotriesterasen nach einem der Ansprüche 8 bis 13, **dadurch gekennzeichnet, dass** sie den folgenden Sequenzen entsprechen:
SEQ ID NO: 7, die der Sequenz SEQ ID NO: 3 entspricht, umfassend die vier folgenden Mutationen:
Substitution von Tyrosin Y an Position 97 durch ein Tryptophan W,
Substitution von Tyrosin Y an Position 99 durch ein Phenylalanin F,
Substitution von Arginin R an Position 223 durch ein Histidin H,
Substitution von Cystein C an Position 258 durch ein Leucin L,
SEQ ID NO: 9, die der Sequenz SEQ ID NO: 7 entspricht, außerdem umfassend die fünf folgenden Mutationen:
Substitution von Valin V an Position 27 durch ein Alanin A,
Substitution von Leucin L an Position 72 durch ein Isoleucin I,
Substitution von Aspartat D an Position 141 durch ein Threonin T,
Substitution von Glycin G an Position 225 durch ein Prolin P,
Substitution von Leucin L an Position 226 durch ein Histidin H,
SEQ ID NO: 11, die der Sequenz SEQ ID NO: 9 entspricht, außerdem umfassend die fünf folgenden Mutationen:
Substitution von Prolin P an Position 67 durch ein Valin V,
Substitution von Threonin T an Position 68 durch ein Serin S,
Substitution von Phenylalanin F an Position 229 durch ein Serin S,
Substitution von Tryptophan W an Position 263 durch ein Alanin A,
Substitution von Tryptophan W an Position 278 durch ein Isoleucin I.

15. Eine Lactonaseaktivität aufweisende, mutierte Phosphotriesterasen nach einem der Ansprüche 8 bis 14, **dadurch gekennzeichnet, dass** sie mindestens eine Mutation umfassen, die der Substitution mindestens einer der Aminosäuren der folgenden Aminosäurepaare entspricht, deren Positionen in SEQ ID NO: 3 im Folgenden durch eine andere natürliche oder nicht natürliche Aminosäure angegeben sind: 2R/314S, 14K/12E, 26R/75D, 26R/42E, 33R/42E, 33R/45E, 55R/52E, 55R/285E, 74R/121D, 81K/42E, 81K/43D, 84K/80E, 109R/113E, 123K/162E, 147K/148D, 151K/148D, 154R/150E, 154R/187E, 154R/188E, 161K/188E, 183R/150E, 183R/187E, 183R/180E, 210K/245D, 215K/214D, 223R/256D, 223R/202D, 234K/204D, 235R/202D, 241R/245D, 245D/244K, 250K/249D, 277R/286D, 292K/298E, 310K/307E.

16. Eine Lactonaseaktivität aufweisende, mutierte hyperthermophile Phosphotriesterasen nach einem der Ansprüche 1 bis 7, die von der hyperthermophilen PTE von *Sulfolobus acidocaldarius* abgeleitet sind, die der Sequenz SEQ ID NO: 5 entspricht und mindestens eine der vier folgenden Mutationen umfasst:
Substitution von Tyrosin Y an Position 98,
Substitution von Tyrosin Y an Position 100,
Substitution von Arginin R an Position 224,
Substitution von Cystein C an Position 259
von SEQ ID NO: 5 mit jeder anderen natürlichen oder nicht natürlichen Aminosäure.

17. Eine Lactonaseaktivität aufweisende, mutierte hyperthermophile Phosphotriesterasen nach Anspruch 16, die mindestens die vier folgenden Mutationen umfassen:
Substitution von Tyrosin Y an Position 98,
Substitution von Tyrosin Y an Position 100,
Substitution von Arginin R an Position 224,
Substitution von Cystein C an Position 259
von SEQ ID NO: 5 mit jeder anderen natürlichen oder nicht natürlichen Aminosäure.

18. Eine Lactonaseaktivität aufweisende, mutierte, hyperthermophile Phosphotriesterasen nach Anspruch 16 oder 17, **dadurch gekennzeichnet, dass** sie mindestens auch eine der folgenden Mutationen umfassen:
Substitution von Valin V an Position 28,
Substitution von Prolin P an Position 68,
Substitution von Threonin T an Position 69,
Substitution von Leucin L an Position 73,
Substitution von Aspartat D an Position 142,
Substitution von Glycin G an Position 226,
Substitution von Leucin L an Position 227,
Substitution von Phenylalanin F an Position 230,
Substitution von Tryptophan W an Position 264,
Substitution von Tryptophan W an Position 279
von SEQ ID NO: 5 mit jeder anderen natürlichen oder nicht natürlichen Aminosäure.

19. Eine Lactonaseaktivität aufweisende, mutierte, hyperthermophile Phosphotriesterasen nach einem der Ansprüche 16 bis 18, **dadurch gekennzeichnet, dass** sie die folgenden fünf Mutationen umfassen:
Substitution von Valin V an Position 28,
Substitution von Leucin L an Position 73,
Substitution von Aspartat D an Position 142,
Substitution von Glycin G an Position 226,
Substitution von Leucin L an Position 227
von SEQ ID NO: 5 mit jeder anderen natürlichen oder nicht natürlichen Aminosäure.

20. Eine Lactonaseaktivität aufweisende, mutierte, hyperthermophile Phosphotriesterasen nach einem der Ansprüche 16 bis 19, **dadurch gekennzeichnet, dass** sie die folgenden fünf Mutationen umfassen:
Substitution von Prolin P an Position 68,
Substitution von Threonin T an Position 69,
Substitution von Phenylalanin F an Position 230,
Substitution von Tryptophan W an Position 264,
Substitution von Tryptophan W an Position 279
von SEQ ID NO: 5 mit jeder anderen natürlichen oder nicht natürlichen Aminosäure.

21. Eine Lactonaseaktivität aufweisende, mutierte, hyperthermophile Phosphotriesterasen nach einem der Ansprüche 16 bis 20, **dadurch gekennzeichnet, dass** sie mindestens eine der folgenden vier Mutationen:
Substitution von Tyrosin Y an Position 98 durch ein Tryptophan W,
Substitution von Tyrosin Y an Position 100 durch ein Phenylalanin F,
Substitution von Arginin R an Position 224 durch ein Histidin H,
Substitution von Cystein C an Position 259 durch ein Leucin L
und gegebenenfalls mindestens eine der folgenden Mutationen umfassen:
Substitution von Valin V an Position 28 durch ein Alanin A,
Substitution von Prolin P an Position 68 durch ein Valin V,
Substitution von Threonin T an Position 69 durch ein Serin S,
Substitution von Leucin L an Position 73 durch ein Isoleucin I,
Substitution von Aspartat D an Position 142 durch ein Threonin T,
Substitution von Glycin G an Position 226 durch ein Prolin P,
Substitution von Leucin L an Position 227 durch ein Histidin H,
Substitution von Phenylalanin F an Position 230 durch ein Serin S,
Substitution von Tryptophan W an Position 264 durch ein Alanin A,
Substitution von Tryptophan W an Position 279 durch ein Isoleucin I.

22. Eine Lactonaseaktivität aufweisende, mutierte hyperthermophile Phosphotriesterasen nach einem der Ansprüche 16 bis 21, **dadurch gekennzeichnet, dass** sie den folgenden Sequenzen entsprechen:
SEQ ID NO: 13, die der Sequenz SEQ ID NO: 5 entspricht, umfassend die vier folgenden Mutationen:
Substitution von Tyrosin Y an Position 98 durch ein Tryptophan W,
Substitution von Tyrosin Y an Position 100 durch ein Phenylalanin F,
Substitution von Arginin R an Position 224 durch ein Histidin H,
Substitution von Cystein C an Position 259 durch ein Leucin L,
SEQ ID NO: 15, die der Sequenz SEQ ID NO: 13 entspricht, außerdem umfassend die fünf folgenden Mutationen:
Substitution von Valin V an Position 28 durch ein Alanin A,
Substitution von Leucin L an Position 73 durch ein Isoleucin I,
Substitution von Aspartat D an Position 142 durch ein Threonin T,
Substitution von Glycin G an Position 226 durch ein Prolin P,
Substitution von Leucin L an Position 227 durch ein Histidin H,
SEQ ID NO: 17, die der Sequenz SEQ ID NO: 15 entspricht, außerdem umfassend die fünf folgenden Mutationen:
Substitution von Prolin P an Position 68 durch ein Valin V,
Substitution von Threonin T an Position 69 durch ein Serin S,
Substitution von Phenylalanin F an Position 230 durch ein Serin S,
Substitution von Tryptophan W an Position 264 durch ein Alanin A,
Substitution von Tryptophan W an Position 279 durch ein Isoleucin I.

23. Ein Lactonaseaktivität aufweisende, mutierte hyperthermophile Phosphotriesterasen nach einem der Ansprüche 1 bis 22, wobei mindestens eine der an den Salzbrücken beteiligten Aminosäuren durch Substitution oder Deletion derart modifiziert ist, dass die Aktivierungstemperatur der eine Lactonaseaktivität aufweisenden, mutierten hyperthermophilen Phosphotriesterasen bezogen auf die Aktivierungstemperatur der eine Lactonaseaktivität aufweisenden, mutierten hyperthermophilen Phosphotriesterasen, bei denen die an den Salzbrücken beteiligten Aminosäuren nicht modifiziert sind, verringert ist.

24. Nukleotidsequenzen, die für eine Lactonaseaktivität aufweisende, mutierte hyperthermophile Phosphotriesterasen, wie in einem der Ansprüche 1 bis 23 definiert, kodieren.

25. Vektoren, insbesondere Plasmide, die Nukleotidsequenzen enthalten, die für eine Lactonaseaktivität aufweisende, mutierte hyperthermophile Phosphotriesterasen, wie in Anspruch 24 definiert, kodieren.

26. Wirtszellen, insbesondere Bakterien, die mithilfe eines Vektors, wie in Anspruch 25 definiert, transformiert werden, unter der Bedingung, dass die Wirtszellen keine menschlichen embryonalen Stammzellen sind.

27. Wirtzellen, insbesondere Bakterien, die mit den eine Lactonaseaktivität aufweisenden, mutierten hyperthermophilen Phosphotriesterasen, wie in einem der Ansprüche 1 bis 23 definiert, gekoppelt sind oder die eine Lactonaseaktivität aufweisende, mutierte hyperthermophile Phosphotriesterasen, wie in einem der Ansprüche 1 bis 23 definiert, auf ihre Oberfläche gepfropft aufweisen, unter der Bedingung, dass die Wirtzellen keine menschlichen embryonalen Stammzellen sind.

28. Transgene Organismen, insbesondere Säugetiere, die mithilfe eines Vektors, wie in Anspruch 25 definiert, transformiert wurden, wobei die transgenen Organismen gegen Pathogene resistent sind, unter der Bedingung, dass die transgenen Organismen keine Menschen sind.

29. Verwendung von eine Lactonaseaktivität aufweisenden, mutierten hyperthermophilen Phosphotriesterasen nach einem der Ansprüche 1 bis 23 oder von Wirtszellen nach Anspruch 26 oder 27
als biologische Reiniger im Rahmen der Dekontaminierung von Materialoberflächen, die durch phosphorhaltige organische Verbindungen oder Bakterien kontaminiert sind, oder zur Herstellung von Medikamenten im Rahmen der Kontamination der Haut oder der Schleimhäute, die durch phosphorhaltige organische Verbindungen oder Bakterien kontaminiert sind, oder im Rahmen der Herstellung von Medikamenten, die im Rahmen der Vorbeugung oder der Behandlung einer externen Kontamination oder einer internen Vergiftung durch Ingestion oder Inhalation durch phosphorhaltige organische Verbindungen verwendbar sind,
oder im Rahmen der Herstellung von Medikamenten, die im Rahmen der Vorbeugung oder der Behandlung einer bakteriellen Infektion verwendbar sind,
oder im Rahmen der Reinigung von Wasser, das mit phosphorhaltigen organischen Verbindungen verunreinigt ist,
oder im Rahmen der Zerstörung von Lagerbeständen von Nervenkampfstoffen.

30. Mit eine Lactonaseaktivität aufweisenden, mutierten hyperthermophilen Phosphotriesterasen nach einem der Ansprüche 1 bis 23 imprägnierte Materialien in flüssiger oder fester Form, wie etwa Handschuhe, verschiedenartige Kleidungsstücke, Tücher, sprühbare Schäume.

31. Kits zur Dekontaminierung von Materialoberflächen, von Haut oder Schleimhäuten, die durch phosphorhaltige organische Verbindungen kontaminiert sind, oder zur Reinigung von Wasser, das mit phosphorhaltigen organischen Verbindungen kontaminiert ist, **dadurch gekennzeichnet, dass** sie eine Lactonaseaktivität aufweisende, mutierte hyperthermophile Phosphotriesterasen, wie in einem der Ansprüche 1 bis 23 definiert, oder mit eine Lactonaseaktivität aufweisenden, mutierten, hyperthermophilen Phosphotriesterasen nach Anspruch 29 imprägnierte Materialien umfassen.

32. Sensoren der phosphorhaltigen organischen Verbindungen, die eine Lactonaseaktivität aufweisende, mutierte hyperthermophile Phosphotriesterasen, wie in einem der Ansprüche 1 bis 23 definiert, umfassen.

33. Patronen zur externen Dekontaminierung, in deren Innerem eine Lactonaseaktivität aufweisende, mutierte hyperthermophile Phosphotriesterasen, wie in einem der Ansprüche 1 bis 23 definiert, gepfropft sind.

34. Pharmazeutische Zusammensetzung, **dadurch gekennzeichnet, dass** sie eine Lactonaseaktivität aufweisende, mutierte, hyperthermophile Phosphotriesterasen nach einem der Ansprüche 1 bis 23 in Verbindung mit einem pharmazeutisch verträglichen Vehikel umfasst.

35. Pharmazeutische Zusammensetzung nach Anspruch 34, **dadurch gekennzeichnet, dass** sie eine Form aufweist, die als Injektion, insbesondere in Lösung oder verkapselt oder pegyliert, oder topisch, insbesondere in Form von Salbe, Aerosol oder Tüchern, verabreicht werden kann.

36. Verwendung von imprägnierten Materialien nach Anspruch 29 oder von Patronen zur externen Dekontaminierung nach Anspruch 32 zur Herstellung von antiseptischen Medikamenten zur Dekontaminierung von bakterieller Oberflächeninfektion.

37. Verwendung der pharmazeutischen Zusammensetzung nach Anspruch 33 oder 34 zur Herstellung eines Medikaments, das zur Behandlung von bakteriellen Infektionen, insbesondere im Blut, bestimmt ist.

## Claims

1. Mutated hyperthermophilic phosphotriesterases (PTEs) possessing a lactonase activity derived from the hyperthermophilic PTEs matching the consensus sequence SEQ ID NO: 1, and comprising at least one of the following four mutations:
substitution of the tyrosine Y in position 98,
substitution of the tyrosine Y in position 100,
substitution of the arginine R in position 224,
substitution of the cysteine C in position 259,
of SEQ ID NO: 1 by any other natural or non-natural amino acid,
said mutated hyperthermophilic phosphotriesterases (PTEs) possessing a lactonase activity having an activity greater than that of the non-mutated hyperthermophilic phosphotriesterases (PTEs) from which they are derived.

2. Mutated hyperthermophilic phosphotriesterases possessing a lactonase activity according to claim 1, derived from the hyperthermophilic PTE of *Sulfolobus solfataricus* corresponding to the sequence SEQ ID NO: 3, or from the hyperthermophilic PTE of *Sulfolobus acidocaldarius* corresponding to the sequence SEQ ID NO: 5, said sequences SEQ ID NO: 3 and SEQ ID NO: 5 belonging to the consensus sequence SEQ ID NO: 1, the amino acid in position 2 in SEQ ID NO: 1 being missing in SEQ ID NO: 3.

3. Mutated hyperthermophilic phosphotriesterases possessing a lactonase activity according to claim 1 or 2, comprising at least the following four mutations:
substitution of the tyrosine Y in position 98,
substitution of the tyrosine Y in position 100,
substitution of the arginine R in position 224,
substitution of the cysteine C in position 259,
of SEQ ID NO: 1 by any other natural or non-natural amino acid.

4. Mutated hyperthermophilic phosphotriesterases possessing a lactonase activity according to any of claims 1 to 3, **characterized in that** they also comprise at least one of the following mutations:
substitution of the valine V in position 28,
substitution of the proline P in position 68,
substitution of the threonine T in position 69,
substitution of the leucine L in position 73,
substitution of the aspartate D in position 142,
substitution of the glycine G in position 226,
substitution of the leucine L in position 227,
substitution of the phenylalanine F in position 230,
substitution of the tryptophan W in position 264,
substitution of the tryptophan W in position 279,
of SEQ ID NO: 1 by any other natural or non-natural amino acid.

5. Mutated hyperthermophilic phosphotriesterases possessing a lactonase activity according to any of claims 1 to 4, **characterized in that** they comprise the following five mutations:
substitution of the valine V in position 28,
substitution of the leucine L in position 73,
substitution of the aspartate D in position 142,
substitution of the glycine G in position 226,
substitution of the leucine L in position 227,
of SEQ ID NO: 1 by any other natural or non-natural amino acid.

6. Mutated hyperthermophilic phosphotriesterases possessing a lactonase activity according to any of claims 1 to 5, **characterized in that** they comprise the following five mutations:
substitution of the proline P in position 68,
substitution of the threonine T in position 69,
substitution of the phenylalanine F in position 230,
substitution of the tryptophan W in position 264,
substitution of the tryptophan W in position 279,
of SEQ ID NO: 1 by any other natural or non-natural amino acid.

7. Mutated hyperthermophilic phosphotriesterases possessing a lactonase activity according to any of claims 1 to 4, derived from the hyperthermophilic PTEs matching the consensus sequence SEQ ID NO: 1, **characterized in that** they comprise at least one of the following four mutations:
substitution of the tyrosine Y in position 98 by a tryptophan W,
substitution of the tyrosine Y in position 100 by a phenylalanine F,
substitution of the arginine R in position 224 by a histidine H,
substitution of the cysteine C in position 259 by a leucine L,
and, if appropriate, at least one of the following mutations:
substitution of the valine V in position 28 by an alanine A,
substitution of the proline P in position 68 by a valine V,
substitution of the threonine T in position 69 by a serine S,
substitution of the leucine L in position 73 by an isoleucine I,
substitution of the aspartate D in position 142 by a threonine T,
substitution of the glycine G in position 226 by a proline P,
substitution of the leucine L in position 227 by a histidine H,
substitution of the phenylalanine F in position 230 by a serine S,
substitution of the tryptophan W in position 264 by an alanine A,
substitution of the tryptophan W in position 279 by an isoleucine I.

8. Mutated hyperthermophilic phosphotriesterases possessing a lactonase activity according to any of claims 1 to 7, derived from the hyperthermophilic PTEs of *Sulfolobus solfataricus* corresponding to the sequence SEQ ID NO: 3, and comprising at least one of the following four mutations:
substitution of the tyrosine Y in position 97,
substitution of the tyrosine Y in position 99,
substitution of the arginine R in position 223,
substitution of the cysteine C in position 258,
of SEQ ID NO: 3 by any other natural or non-natural amino acid.

9. Mutated hyperthermophilic phosphotriesterases possessing a lactonase activity according to claim 8, comprising at least the following four mutations:
substitution of the tyrosine Y in position 97,
substitution of the tyrosine Y in position 99,
substitution of the arginine R in position 223,
substitution of the cysteine C in position 258,
of SEQ ID NO: 3 by any other natural or non-natural amino acid.

10. Mutated hyperthermophilic phosphotriesterases possessing a lactonase activity according to claim 8 or 9, **characterized in that** they also comprise at least one of the following mutations:
substitution of the valine V in position 27,
substitution of the proline P in position 67,
substitution of the threonine T in position 68,
substitution of the leucine L in position 72,
substitution of the aspartate D in position 141,
substitution of the glycine G in position 225,
substitution of the leucine L in position 226,
substitution of the phenylalanine F in position 229,
substitution of the tryptophan W in position 263,
substitution of the tryptophan W in position 278,
of SEQ ID NO: 3 by any other natural or non-natural amino acid.

11. Mutated hyperthermophilic phosphotriesterases possessing a lactonase activity according to any of claims 8 to 10, **characterized in that** they comprise the following five mutations:
substitution of the valine V in position 27,
substitution of the leucine L in position 72,
substitution of the aspartate D in position 141,
substitution of the glycine G in position 225,
substitution of the leucine L in position 226,
of SEQ ID NO: 3 by any other natural or non-natural amino acid.

12. Mutated hyperthermophilic phosphotriesterases possessing a lactonase activity according to any of claims 8 to 11, **characterized in that** they comprise the following five mutations:
substitution of the proline P in position 67,
substitution of the threonine T in position 68,
substitution of the phenylalanine F in position 229,
substitution of the tryptophan W in position 263,
substitution of the tryptophan W in position 278,
of SEQ ID NO: 3 by any other natural or non-natural amino acid.

13. Mutated hyperthermophilic phosphotriesterases possessing a lactonase activity according to any of claims 8 to 12, **characterized in that** they comprise at least one of the following four mutations:
substitution of the tyrosine Y in position 97 by a tryptophan W,
substitution of the tyrosine Y in position 99 by a phenylalanine F,
substitution of the arginine R in position 223 by a histidine H,
substitution of the cysteine C in position 258 by a leucine L,
and, if appropriate, at least one of the following mutations:
substitution of the valine V in position 27 by an alanine A,
substitution of the proline P in position 67 by a valine V,
substitution of the threonine T in position 68 by a serine S,
substitution of the leucine L in position 72 by an isoleucine I,
substitution of the aspartate D in position 141 by a threonine T,
substitution of the glycine G in position 225 by a proline P,
substitution of the leucine L in position 226 by a histidine H,
substitution of the phenylalanine F in position 229 by a serine S,
substitution of the tryptophan W in position 263 by an alanine A,
substitution of the tryptophan W in position 278 by an isoleucine I.

14. Mutated hyperthermophilic phosphotriesterases possessing a lactonase activity according to any of claims 8 to 13, **characterized in that** they correspond to the following sequences:
SEQ ID NO: 7 corresponding to the sequence SEQ ID NO: 3 comprising the following four mutations:
substitution of the tyrosine Y in position 97 by a tryptophan W,
substitution of the tyrosine Y in position 99 by a phenylalanine F,
substitution of the arginine R in position 223 by a histidine H,
substitution of the cysteine C in position 258 by a leucine L,
SEQ ID NO: 9 corresponding to the sequence SEQ ID NO: 7 additionally comprising the following five mutations:
substitution of the valine V in position 27 by an alanine A,
substitution of the leucine L in position 72 by an isoleucine I,
substitution of the aspartate D in position 141 by a threonine T,
substitution of the glycine G in position 225 by a proline P,
substitution of the leucine L in position 226 by a histidine H,
SEQ ID NO: 11 corresponding to the sequence SEQ ID NO: 9 additionally comprising the following five mutations:
substitution of the proline P in position 67 by a valine V,
substitution of the threonine T in position 68 by a serine S,
substitution of the phenylalanine F in position 229 by a serine S,
substitution of the tryptophan W in position 263 by an alanine A,
substitution of the tryptophan W in position 278 by an isoleucine I.

15. Mutated hyperthermophilic phosphotriesterases possessing a lactonase activity according to any of claims 8 to 14, **characterized in that** they comprise at least one mutation corresponding to the substitution of at least one of the amino acids of the following amino acid pairs the positions of which in SEQ ID NO: 3 are indicated hereafter, by another natural or non-natural amino acid: 2R/314S, 14K/12E, 26R/75D, 26R/42E, 33R/42E, 33R/45E, 55R/52E, 55R/285E, 74R/121D, 81K/42E, 81K/43D, 84K/80E, 109R/113E, 123K/162E, 147K/148D, 151K/148D, 154R/150E, 154R/187E, 154R/188E, 161K/188E, 183R/150E, 183R/187E, 183R/180E, 210K/245D, 215K/214D, 223R/256D, 223R/202D, 234K/204D, 235R/202D, 241R/245D, 245D/244K, 250K/249D, 277R/286D, 292K/298E, 310K/307E.

16. Mutated hyperthermophilic phosphotriesterases possessing a lactonase activity according to any of claims 1 to 7, derived from the hyperthermophilic PTEs of *Sulfolobus acidocaldarius* corresponding to the sequence SEQ ID NO: 5, and comprising at least one of the following four mutations:
substitution of the tyrosine Y in position 98,
substitution of the tyrosine Y in position 100,
substitution of the arginine R in position 224,
substitution of the cysteine C in position 259,
of SEQ ID NO: 5 by any other natural or non-natural amino acid.

17. Mutated hyperthermophilic phosphotriesterases possessing a lactonase activity according to claim 16, comprising at least the following four mutations:
substitution of the tyrosine Y in position 98,
substitution of the tyrosine Y in position 100,
substitution of the arginine R in position 224,
substitution of the cysteine C in position 259,
of SEQ ID NO: 5 by any other natural or non-natural amino acid.

18. Mutated hyperthermophilic phosphotriesterases possessing a lactonase activity according to claim 16 or 17, **characterized in that** they also comprise at least one of the following mutations:
substitution of the valine V in position 28,
substitution of the proline P in position 68,
substitution of the threonine T in position 69,
substitution of the leucine L in position 73,
substitution of the aspartate D in position 142,
substitution of the glycine G in position 226,
substitution of the leucine L in position 227,
substitution of the phenylalanine F in position 230,
substitution of the tryptophan W in position 264,
substitution of the tryptophan W in position 279,
of SEQ ID NO: 5 by any other natural or non-natural amino acid.

19. Mutated hyperthermophilic phosphotriesterases possessing a lactonase activity according to any of claims 16 to 18, **characterized in that** they comprise the following five mutations:
substitution of the valine V in position 28,
substitution of the leucine L in position 73,
substitution of the aspartate D in position 142,
substitution of the glycine G in position 226,
substitution of the leucine L in position 227,
of SEQ ID NO: 5 by any other natural or non-natural amino acid.

20. Mutated hyperthermophilic phosphotriesterases possessing a lactonase activity according to any of claims 16 to 19, **characterized in that** they comprise the following five mutations:
substitution of the proline P in position 68,
substitution of the threonine T in position 69,
substitution of the phenylalanine F in position 230,
substitution of the tryptophan W in position 264,
substitution of the tryptophan W in position 279,
of SEQ ID NO: 5 by any other natural or non-natural amino acid.

21. Mutated hyperthermophilic phosphotriesterases possessing a lactonase activity according to any of claims 16 to 20, **characterized in that** they comprise at least one of the following four mutations:
substitution of the tyrosine Y in position 98 by a tryptophan W,
substitution of the tyrosine Y in position 100 by a phenylalanine F,
substitution of the arginine R in position 224 by a histidine H,
substitution of the cysteine C in position 259 by a leucine L,
and, if appropriate, at least one of the following mutations:
substitution of the valine V in position 28 by an alanine A,
substitution of the proline P in position 68 by a valine V,
substitution of the threonine T in position 69 by a serine S,
substitution of the leucine L in position 73 by an isoleucine I,
substitution of the aspartate D in position 142 by a threonine T,
substitution of the glycine G in position 226 by a proline P,
substitution of the leucine L in position 227 by a histidine H,
substitution of the phenylalanine F in position 230 by a serine S,
substitution of the tryptophan W in position 264 by an alanine A,
substitution of the tryptophan W in position 279 by an isoleucine I.

22. Mutated hyperthermophilic phosphotriesterases possessing a lactonase activity according to any of claims 16 to 21, **characterized in that** they correspond to the following sequences:
SEQ ID NO: 13 corresponding to the sequence SEQ ID NO: 5 comprising the following four mutations:
substitution of the tyrosine Y in position 98 by a tryptophan W,
substitution of the tyrosine Y in position 100 by a phenylalanine F,
substitution of the arginine R in position 224 by a histidine H,
substitution of the cysteine C in position 259 by a leucine L,
SEQ ID NO: 15 corresponding to the sequence SEQ ID NO: 13 additionally comprising the following five mutations:
substitution of the valine V in position 28 by an alanine A,
substitution of the leucine L in position 73 by an isoleucine I,
substitution of the aspartate D in position 142 by a threonine T,
substitution of the glycine G in position 226 by a proline P,
substitution of the leucine L in position 227 by a histidine H,
SEQ ID NO: 17 corresponding to the sequence SEQ ID NO: 15 additionally comprising the following five mutations:
substitution of the proline P in position 68 by a valine V,
substitution of the threonine T in position 69 by a serine S,
substitution of the phenylalanine F in position 230 by a serine S,
substitution of the tryptophan W in position 264 by an alanine A,
substitution of the tryptophan W in position 279 by an isoleucine I.

23. Mutated hyperthermophilic phosphotriesterases possessing a lactonase activity according to any of claims 1 to 22, in which at least one of the amino acids involved in the salt bridges is mutated by addition, substitution or deletion, such that the activation temperature of said mutated hyperthermophilic phosphotriesterases possessing a lactonase activity is reduced compared with the activation temperature of the mutated hyperthermophilic phosphotriesterases possessing a lactonase activity in which the amino acids involved in the salt bridges are not mutated.

24. Nucleotide sequences encoding the mutated hyperthermophilic phosphotriesterases possessing a lactonase activity as defined in any of claims 1 to 23.

25. Vectors, in particular plasmids, containing nucleotide sequences encoding the mutated hyperthermophilic phosphotriesterases possessing a lactonase activity as defined in claim 24.

26. Host cells, in particular bacteria, transformed using a vector as defined in claim 25, provided that host cells are not human embryonic stem cells.

27. Host cells, in particular bacteria, coupled with the mutated hyperthermophilic phosphotriesterases possessing a lactonase activity as defined in any of claims 1 to 23, or having mutated hyperthermophilic phosphotriesterases possessing a lactonase activity as defined in any of claims 1 to 23, grafted to their surface,
provided that host cells are not human embryonic stem cells.

28. Transgenic organisms, in particular mammals, transformed using a vector as defined in claim 25, said transgenic organisms being resistant to pathogens, provided that transgenic organisms are not human beings.

29. Use of mutated hyperthermophilic phosphotriesterases possessing a lactonase activity as defined in any of claims 1 to 23, or host cells as defined in any of claims 26 or 27, as bio scrubbers for decontaminating surfaces of materials, contaminated with organophosphorus compounds, or bacteria,
or for the preparation of drugs for decontaminating the skin, or mucous membranes, contaminated with organophosphorus compounds, or bacteria,
or for the preparation of drugs that can be used for the prevention or treatment of an external contamination or of an internal poisoning by ingestion or inhalation by organophosphorus compounds,
or for the preparation of drugs that can be used for the prevention or treatment of a bacterial infection,
or for the pollution control of water polluted with organophosphorus compounds,
or for the destruction of stocks of neurotoxic agents.

30. Materials impregnated with mutated hyperthermophilic phosphotriesterases possessing a lactonase activity as defined in any of claims 1 to 23, in liquid or solid form, such as gloves, various garments, wipes, spray foams.

31. Kits for the decontamination of the surfaces of materials, of the skin or mucous membranes, contaminated with organophosphorus compounds, or for the pollution control of water polluted with organophosphorus compounds, **characterized in that** they comprise mutated hyperthermophilic phosphotriesterases possessing a lactonase activity as defined in any of claims 1 to 23, or materials impregnated with mutated hyperthermophilic phosphotriesterases possessing a lactonase activity according to claim 29.

32. Sensors for organophosphorus compounds comprising mutated hyperthermophilic phosphotriesterases possessing a lactonase activity as defined in any of claims 1 to 23.

33. Cartridges for external decontamination, inside which mutated hyperthermophilic phosphotriesterases possessing a lactonase activity as defined in any of claims 1 to 23 are grafted.

34. Pharmaceutical composition **characterized in that** it comprises mutated hyperthermophilic phosphotriesterases possessing a lactonase activity as defined in any of claims 1 to 23, in combination with a pharmaceutically acceptable vehicle.

35. Pharmaceutical composition according to claim 34, **characterized in that** it is presented in a form which can be administered by injectable route, in particular in solution or packaged or pegylated, or by topical route, in particular in the form of ointment, aerosol or wipes.

36. Use of materials impregnated as defined in claim 29, or cartridges for external decontaminations as defined in claim 32, for the preparation of antiseptic drugs for the decontamination of the surface bacterial infection.

37. Use of the pharmaceutical composition as defined in claim 33 or 34, for the preparation of a drug for the treatment of bacterial infections, in particular in blood.


### SEQUENCE LISTING

<110> UNIVERSITE HENRI POINCARRE-NANCY 1
   CENTRE NATIONAL DE LA RECHERCHE SCIENTIFIQUE
   Chabrière, Eric
   Helias, Mikaël
<120> PHOSPHOTRIESTERASES HYPERTHERMOPHILES MUTEES ET LEURS UTILISATIONS
<130> WOB 07 BG NAN FOLO
<160> 31
<170> PatentIn version 3.5
<210> 1
   <211> 315
   <212> PRT
   <213> sulfobacillus sp.
<220>
   <221> MISC_FEATURE
   <222> (2)..(2)
   <223> T ou blank
<220>
   <221> MISC_FEATURE
   <222> (3)..(3)
   <223> K ou R
<220>
   <221> MISC_FEATURE
   <222> (10)..(10)
   <223> G ou D
<220>
   <221> MISC_FEATURE
   <222> (11)..(11)
   <223> E ou S
<220>
   <221> MISC_FEATURE
   <222> (13)..(13)
   <223> S ou E
<220>
   <221> MISC_FEATURE
   <222> (14)..(14)
   <223> P ou S
<220>
   <221> MISC_FEATURE
   <222> (15)..(15)
   <223> G ou K
<220>
   <221> MISC_FEATURE
   <222> (16)..(16)
   <223> E ou D
<220>
   <221> MISC_FEATURE
   <222> (17)..(17)
   <223> M ou I
<220>
   <221> MISC_FEATURE
   <222> (32)..(32)
   <223> P ou A
<220>
   <221> MISC_FEATURE
   <222> (35)..(35)
   <223> Y ou Q
<220>
   <221> MISC_FEATURE
   <222> (47)..(47)
   <223> L ou F
<220>
   <221> MISC_FEATURE
   <222> (48)..(48)
   <223> K ou R
<220>
   <221> MISC_FEATURE
   <222> (56)..(56)
   <223> T ou R
<220>
   <221> MISC_FEATURE
   <222> (57)..(57)
   <223> I ou A
<220>
   <221> MISC_FEATURE
   <222> (59)..(59)
   <223> S ou Q
<220>
   <221> MISC_FEATURE
   <222> (60)..(60)
   <223> Y ou F
<220>
   <221> MISC_FEATURE
   <222> (80)..(80)
   <223> S ou M
<220>
   <221> MISC_FEATURE
   <222> (86)..(86)
   <223> E ou A
<220>
   <221> MISC_FEATURE
   <222> (91)..(91)
   <223> V ou L
<220>
   <221> MISC_FEATURE
   <222> (92)..(92)
   <223> I ou V
<220>
   <221> MISC_FEATURE
   <222> (94)..(94)
   <223> A ou G
<220>
   <221> MISC_FEATURE
   <222> (97)..(97)
   <223> L ou I
<220>
   <221> MISC_FEATURE
   <222> (99)..(99)
   <223> T ou I
<220>
   <221> MISC_FEATURE
   <222> (101)..(101)
   <223> T ou I
<220>
   <221> MISC_FEATURE
   <222> (106)..(106)
   <223> F ou Y
<220>
   <221> MISC_FEATURE
   <222> (108)..(108)
   <223> N ou L
<220>
   <221> MISC_FEATURE
   <222> (109)..(109)
   <223> G ou N
<220>
   <221> MISC_FEATURE
   <222> (112)..(112)
   <223> L ou I
<220>
   <221> MISC_FEATURE
   <222> (113)..(113)
   <223> E ou D
<220>
   <221> MISC_FEATURE
   <222> (117)..(117)
   <223> E ou D
<220>
   <221> MISC_FEATURE
   <222> (119)..(119)
   <223> L ou F
<220>
   <221> MISC_FEATURE
   <222> (125)..(125)
   <223> K ou E
<220>
   <221> MISC_FEATURE
   <222> (131)..(131)
   <223> N ou L
<220>
   <221> MISC_FEATURE
   <222> (133)..(133)
   <223> R ou K
<220>
   <221> MISC_FEATURE
   <222> (137)..(137)
   <223> I ou V
<220>
   <221> MISC_FEATURE
   <222> (138)..(138)
   <223> K ou X
<220>
   <221> MISC_FEATURE
   <222> (139)..(139)
   <223> V ou I
<220>
   <221> MISC_FEATURE
   <222> (148)..(148)
   <223> R ou K
<220>
   <221> MISC_FEATURE
   <222> (152)..(152)
   <223> R ou K
<220>
   <221> MISC_FEATURE
   <222> (153)..(153)
   <223> A ou V
<220>
   <221> MISC_FEATURE
   <222> (161)..(161)
   <223> Q ou N
<220>
   <221> MISC_FEATURE
   <222> (165)..(165)
   <223> N ou K
<220>
   <221> MISC_FEATURE
   <222> (177)..(177)
   <223> G ou N
<220>
   <221> MISC_FEATURE
   <222> (187)..(187)
   <223> M ou T
<220>
   <221> MISC_FEATURE
   <222> (195)..(195)
   <223> R ou K
<220>
   <221> MISC_FEATURE
   <222> (196)..(196)
   <223> V ou I
<220>
   <221> MISC_FEATURE
   <222> (207)..(207)
   <223> V ou I
<220>
   <221> MISC_FEATURE
   <222> (220)..(220)
   <223> V ou I
<220>
   <221> MISC_FEATURE
   <222> (233)..(233)
   <223> I ou V
<220>
   <221> MISC_FEATURE
   <222> (239)..(239)
   <223> V ou T
<220>
   <221> MISC_FEATURE
   <222> (240)..(240)
   <223> L ou T
<220>
   <221> MISC_FEATURE
   <222> (242)..(242)
   <223> K ou R
<220>
   <221> MISC_FEATURE
   <222> (249)..(249)
   <223> L ou S
<220>
   <221> MISC_FEATURE
   <222> (251)..(251)
   <223> R ou K
<220>
   <221> MISC_FEATURE
   <222> (254)..(254)
   <223> V ou I
<220>
   <221> MISC_FEATURE
   <222> (256)..(256)
   <223> Q ou H
<220>
   <221> MISC_FEATURE
   <222> (266)..(266)
   <223> I ou T
<220>
   <221> MISC_FEATURE
   <222> (278)..(278)
   <223> K ou R
<220>
   <221> MISC_FEATURE
   <222> (281)..(281)
   <223> M ou I
<220>
   <221> MISC_FEATURE
   <222> (282)..(282)
   <223> S ou T
<220>
   <221> MISC_FEATURE
   <222> (286)..(286)
   <223> T ou E
<220>
   <221> MISC_FEATURE
   <222> (288)..(288)
   <223> V ou T
<220>
   <221> MISC_FEATURE
   <222> (291)..(291)
   <223> S ou F
<220>
   <221> MISC_FEATURE
   <222> (292)..(292)
   <223> I ou L
<220>
   <221> MISC_FEATURE
   <222> (295)..(295)
   <223> A ou N
<220>
   <221> MISC_FEATURE
   <222> (298)..(298)
   <223> T ou N
<220>
   <221> MISC_FEATURE
   <222> (299)..(299)
   <223> D ou E
<220>
   <221> MISC_FEATURE
   <222> (301)..(301)
   <223> Q ou V
<220>
   <221> MISC_FEATURE
   <222> (302)..(302)
   <223> L ou I
<220>
   <221> MISC_FEATURE
   <222> (303)..(303)
   <223> H ou A
<220>
   <221> MISC_FEATURE
   <222> (304)..(304)
   <223> V ou T
<220>
   <221> MISC_FEATURE
   <222> (307)..(307)
   <223> V ou K
<220>
   <221> MISC_FEATURE
   <222> (308)..(308)
   <223> K ou E
<220>
   <221> MISC_FEATURE
   <222> (311)..(311)
   <223> A ou K
<220>
   <221> MISC_FEATURE
   <222> (312)..(312)
   <223> R ou K
<220>
   <221> MISC_FEATURE
   <222> (313)..(313)
   <223> L ou F
<400> 1
   Met Xaa Xaa Ile Pro Leu Val Gly Lys Xaa Xaa Ile Xaa Xaa Xaa Xaa
   1               5                   10                  15
   Xaa Gly Phe Thr Leu Ile His Glu His Leu Arg Val Phe Ser Glu Xaa
   20 25 30
   Val Arg Tyr Gln Trp Pro His Leu Tyr Asn Glu Asp Glu Glu Xaa Xaa
   35 40 45
   Asn Ala Val Asn Glu Val Lys Xaa Xaa Met Xaa Xaa Gly Val Lys Thr
   50 55 60
   Ile Val Asp Pro Thr Val Met Gly Leu Gly Arg Asp Ile Arg Phe Xaa
   65 70 75 80
   Glu Lys Val Val Lys Xaa Thr Gly Ile Asn Xaa Xaa Ala Xaa Thr Gly
   85 90 95
   Xaa Tyr Xaa Tyr Xaa Asp Leu Pro Phe Xaa Phe Xaa Xaa Arg Ser Xaa
   100 105 110
   Xaa Glu Ile Ala Xaa Leu Xaa Ile His Asp Ile Lys Xaa Gly Ile Gln
   115 120 125
   Gly Thr Xaa Asn Xaa Ala Gly Phe Xaa Xaa Xaa Ala Ala Asp Glu Pro
   130 135 140
   Gly Ile Thr Xaa Asp Val Glu Xaa Xaa Ile Arg Ala Ala Ala Ile Ala
   145 150 155 160
   Xaa Lys Glu Thr Xaa Val Pro Ile Ile Thr His Ser Asn Ala His Asn
   165 170 175
   Xaa Thr Gly Leu Glu Gln Gln Arg Ile Leu Xaa Glu Glu Gly Val Asp
   180 185 190
   Pro Gly Xaa Xaa Leu Ile Gly His Leu Gly Asp Thr Asp Asn Xaa Asp
   195 200 205
   Tyr Ile Lys Lys Ile Ala Asp Lys Gly Ser Phe Xaa Gly Leu Asp Arg
   210 215 220
   Tyr Gly Leu Asp Leu Phe Leu Pro Xaa Asp Lys Arg Asn Glu Xaa Xaa
   225 230 235 240
   Leu Xaa Leu Ile Lys Asp Gly Tyr Xaa Asp Xaa Ile Met Xaa Ser Xaa
   245 250 255
   Asp Tyr Cys Cys Thr Ile Asp Trp Gly Xaa Ala Lys Pro Glu Tyr Lys
   260 265 270
   Pro Lys Leu Ala Pro Xaa Trp Ser Xaa Xaa Leu Ile Phe Xaa Asp Xaa
   275 280 285
   Ile Pro Xaa Xaa Lys Arg Xaa Gly Val Xaa Xaa Glu Xaa Xaa Xaa Xaa
   290 295 300
   Ile Phe Xaa Xaa Asn Pro Xaa Xaa Xaa Phe Ser
   305 310 315
<210> 2
   <211> 942
   <212> DNA
   <213> Sulfolobus solfataricus
<220>
   <221> CDS
   <222> (1)..(942)
<400> 2
   atg aga ata cca tta gtt ggg aaa gat tca ata gaa tct aag gac ata       48
   Met Arg Ile Pro Leu Val Gly Lys Asp Ser Ile Glu Ser Lys Asp Ile
   1               5                   10                  15
   gga ttt acg cta att cat gaa cat tta aga gtt ttt agc gaa gcg gcc       96
   Gly Phe Thr Leu Ile His Glu His Leu Arg Val Phe Ser Glu Ala Ala
               20                  25                  30
   aga caa caa tgg ccc cat cta tat aac gaa gat gag gag ttc aga aac      144
   Arg Gln Gln Trp Pro His Leu Tyr Asn Glu Asp Glu Glu Phe Arg Asn
           35                  40                  45
   gct gta aat gag gtt aaa agg gca atg caa ttt gga gta aag act ata      192
   Ala Val Asn Glu Val Lys Arg Ala Met Gln Phe Gly Val Lys Thr Ile
       50                  55                  60
   gta gat ccc act gta atg gga ttg ggt agg gac atc aga ttt atg gaa      240
   Val Asp Pro Thr Val Met Gly Leu Gly Arg Asp Ile Arg Phe Met Glu
   65                  70                  75                  80
   aaa gtg gtt aag gct acc ggg ata aat tta gtt gcg ggg acg ggg att      288
   Lys Val Val Lys Ala Thr Gly Ile Asn Leu Val Ala Gly Thr Gly Ile
                   85                  90                  95
   tac ata tat atc gac tta cct ttc tat ttc tta aat agg tca att gat      336
   Tyr Ile Tyr Ile Asp Leu Pro Phe Tyr Phe Leu Asn Arg Ser Ile Asp
               100                 105                 110
   gag ata gct gac ttg ttt att cat gat ata aaa gag gga ata caa ggt      384
   Glu Ile Ala Asp Leu Phe Ile His Asp Ile Lys Glu Gly Ile Gln Gly
           115                 120                 125
   act ctc aat aaa gct ggc ttc gta aag ata gct gca gat gaa cct ggg      432
   Thr Leu Asn Lys Ala Gly Phe Val Lys Ile Ala Ala Asp Glu Pro Gly
       130                 135                 140
   atc aca aag gat gtg gag aag gta ata agg gct gct gcc ata gca aac      480
   Ile Thr Lys Asp Val Glu Lys Val Ile Arg Ala Ala Ala Ile Ala Asn
   145                 150                 155                 160
   aaa gag act aaa gta cca ata att acc cac tct aac gct cac aat aac      528
   Lys Glu Thr Lys Val Pro Ile Ile Thr His Ser Asn Ala His Asn Asn
                   165                 170                 175
   acc gga tta gaa cag caa aga ata ttg act gaa gaa ggt gtt gat cca      576
   Thr Gly Leu Glu Gln Gln Arg Ile Leu Thr Glu Glu Gly Val Asp Pro
               180                 185                 190
   ggg aaa ata tta ata ggt cat tta ggt gat aca gat aat ata gat tac      624
   Gly Lys Ile Leu Ile Gly His Leu Gly Asp Thr Asp Asn Ile Asp Tyr
           195                 200                 205
   ata aag aag ata gca gat aag gga tcc ttt att gga tta gat aga tat    672
   Ile Lys Lys Ile Ala Asp Lys Gly Ser Phe Ile Gly Leu Asp Arg Tyr
       210                 215                 220
   ggt tta gat tta ttc cta cct gtt gat aag aga aat gaa acg acc tta    720
   Gly Leu Asp Leu Phe Leu Pro Val Asp Lys Arg Asn Glu Thr Thr Leu
   225                 230                 235                 240
   aga cta atc aaa gat ggt tat tca gat aag ata atg atc tct cac gat    768
   Arg Leu Ile Lys Asp Gly Tyr Ser Asp Lys Ile Met Ile Ser His Asp
                   245                 250                 255
   tat tgt tgc aca atc gac tgg gga act gca aaa cca gaa tat aaa cct    816
   Tyr Cys Cys Thr Ile Asp Trp Gly Thr Ala Lys Pro Glu Tyr Lys Pro
               260                 265                 270
   aag ctt gct cca aga tgg agt ata act cta ata ttt gag gat acg ata    864
   Lys Leu Ala Pro Arg Trp Ser Ile Thr Leu Ile Phe Glu Asp Thr Ile
           275                 280                 285
   ccg ttc tta aag aga aat gga gtg aat gaa gag gtt ata gct aca ata    912
   Pro Phe Leu Lys Arg Asn Gly Val Asn Glu Glu Val Ile Ala Thr Ile
       290                 295                 300
   ttt aag gaa aat ccg aaa aag ttc ttc agc                            942
   Phe Lys Glu Asn Pro Lys Lys Phe Phe Ser
   305                 310
<210> 3
   <211> 314
   <212> PRT
   <213> Sulfolobus solfataricus
<400> 3
   Met Arg Ile Pro Leu Val Gly Lys Asp Ser Ile Glu Ser Lys Asp Ile
   1               5                   10                  15
   Gly Phe Thr Leu Ile His Glu His Leu Arg Val Phe Ser Glu Ala Ala
               20                  25                  30
   Arg Gln Gln Trp Pro His Leu Tyr Asn Glu Asp Glu Glu Phe Arg Asn
           35                  40                  45
   Ala Val Asn Glu Val Lys Arg Ala Met Gln Phe Gly Val Lys Thr Ile
       50                  55                  60
   Val Asp Pro Thr Val Met Gly Leu Gly Arg Asp Ile Arg Phe Met Glu
   65                  70                  75                  80
   Lys Val Val Lys Ala Thr Gly Ile Asn Leu Val Ala Gly Thr Gly Ile
                   85                  90                  95
   Tyr Ile Tyr Ile Asp Leu Pro Phe Tyr Phe Leu Asn Arg Ser Ile Asp
               100                 105                 110
   Glu Ile Ala Asp Leu Phe Ile His Asp Ile Lys Glu Gly Ile Gln Gly
           115                 120                 125
   Thr Leu Asn Lys Ala Gly Phe Val Lys Ile Ala Ala Asp Glu Pro Gly
   130 135 140
   Ile Thr Lys Asp Val Glu Lys Val Ile Arg Ala Ala Ala Ile Ala Asn
   145 150 155 160
   Lys Glu Thr Lys Val Pro Ile Ile Thr His Ser Asn Ala His Asn Asn
   165 170 175
   Thr Gly Leu Glu Gln Gln Arg Ile Leu Thr Glu Glu Gly Val Asp Pro
   180 185 190
   Gly Lys Ile Leu Ile Gly His Leu Gly Asp Thr Asp Asn Ile Asp Tyr
   195 200 205
   Ile Lys Lys Ile Ala Asp Lys Gly Ser Phe Ile Gly Leu Asp Arg Tyr
   210 215 220
   Gly Leu Asp Leu Phe Leu Pro Val Asp Lys Arg Asn Glu Thr Thr Leu
   225 230 235 240
   Arg Leu Ile Lys Asp Gly Tyr Ser Asp Lys Ile Met Ile Ser His Asp
   245 250 255
   Tyr Cys Cys Thr Ile Asp Trp Gly Thr Ala Lys Pro Glu Tyr Lys Pro
   260 265 270
   Lys Leu Ala Pro Arg Trp Ser Ile Thr Leu Ile Phe Glu Asp Thr Ile
   275 280 285
   Pro Phe Leu Lys Arg Asn Gly Val Asn Glu Glu Val Ile Ala Thr Ile
   290 295 300
   Phe Lys Glu Asn Pro Lys Lys Phe Phe Ser
   305 310
<210> 4
   <211> 945
   <212> DNA
   <213> Sulfolobus acidocaldarius
<220>
   <221> CDS
   <222> (1)..(945)
<400> 4
   atg aca aaa att cct ctt gta gga aaa ggt gaa ata tca cct gga gaa      48
   Met Thr Lys Ile Pro Leu Val Gly Lys Gly Glu Ile Ser Pro Gly Glu
   1               5                   10                  15
   atg ggt ttt act tta ata cat gag cat tta agg gtc ttc agt gaa cca      96
   Met Gly Phe Thr Leu Ile His Glu His Leu Arg Val Phe Ser Glu Pro
               20                  25                  30
   gtt aga tat caa tgg cca cat ctt tat aat gaa gat gag gag tta aaa      144
   Val Arg Tyr Gln Trp Pro His Leu Tyr Asn Glu Asp Glu Glu Leu Lys
           35                  40                  45
   aat gca gta aat gaa gta aag aca ata atg tca tat ggt gtt aag acc      192
   Asn Ala Val Asn Glu Val Lys Thr Ile Met Ser Tyr Gly Val Lys Thr
       50                  55                  60
   atc gtg gat ccc act gtc atg ggt tta ggg aga gac att aga ttc agt      240
   Ile Val Asp Pro Thr Val Met Gly Leu Gly Arg Asp Ile Arg Phe Ser
   65                  70                  75                  80
   gag aag gtc gtg aaa gaa aca ggt ata aat gtg att gca gca acg ggg      288
   Glu Lys Val Val Lys Glu Thr Gly Ile Asn Val Ile Ala Ala Thr Gly
                   85                  90                  95
   ttg tat act tac act gat tta cct ttc ttc ttc aat gga aga tca ttg      336
   Leu Tyr Thr Tyr Thr Asp Leu Pro Phe Phe Phe Asn Gly Arg Ser Leu
               100                 105                 110
   gaa gag att gca gaa tta tta ata cat gat ata aaa aag gga ata caa      384
   Glu Glu Ile Ala Glu Leu Leu Ile His Asp Ile Lys Lys Gly Ile Gln
           115                 120                 125
   ggg aca aat aat aga gca ggc ttc att aag gtt gca gca gat gag cca      432
   Gly Thr Asn Asn Arg Ala Gly Phe Ile Lys Val Ala Ala Asp Glu Pro
       130                 135                 140
   ggt ata acg agg gat gta gag agg gca ata agg gca gct gct ata gct      480
   Gly Ile Thr Arg Asp Val Glu Arg Ala Ile Arg Ala Ala Ala Ile Ala
   145                 150                 155                 160
   cag aag gag act aac gta ccc ata ata aca cat tca aat gct cat aac      528
   Gln Lys Glu Thr Asn Val Pro Ile Ile Thr His Ser Asn Ala His Asn
                   165                 170                 175
   ggg aca ggt ctt gag caa caa agg att cta atg gag gag ggt gta gac      576
   Gly Thr Gly Leu Glu Gln Gln Arg Ile Leu Met Glu Glu Gly Val Asp
               180                 185                 190
   cca ggg aga gtg cta ata ggt cac ttg ggg gac act gat aac gtg gat      624
   Pro Gly Arg Val Leu Ile Gly His Leu Gly Asp Thr Asp Asn Val Asp
           195                 200                 205
   tac ata aag aag ata gcc gat aaa ggc tcg ttt gta ggt cta gat aga      672
   Tyr Ile Lys Lys Ile Ala Asp Lys Gly Ser Phe Val Gly Leu Asp Arg
       210                 215                 220
   tac ggt cta gat cta ttc tta cct ata gat aaa agg aac gag gtg ttg      720
   Tyr Gly Leu Asp Leu Phe Leu Pro Ile Asp Lys Arg Asn Glu Val Leu
   225                 230                 235                 240
   ttg aaa tta att aaa gat gga tac ttg gac agg att atg gtg tca caa      768
   Leu Lys Leu Ile Lys Asp Gly Tyr Leu Asp Arg Ile Met Val Ser Gln
                   245                 250                 255
   gat tac tgt tgc aca att gac tgg ggg ata gca aag ccg gag tac aaa      816
   Asp Tyr Cys Cys Thr Ile Asp Trp Gly Ile Ala Lys Pro Glu Tyr Lys
               260                 265                 270
   cct aaa cta gct cca aaa tgg agt atg agt tta ata ttt aca gac gtt      864
   Pro Lys Leu Ala Pro Lys Trp Ser Met Ser Leu Ile Phe Thr Asp Val
           275                 280                 285
   ata ccc tca att aag aga gca gga gta act gat gag cag ttg cat gta      912
   Ile Pro Ser Ile Lys Arg Ala Gly Val Thr Asp Glu Gln Leu His Val
       290                 295                 300
   atc ttc gta aag aat cca gct aga cta ttt agt                          945
   Ile Phe Val Lys Asn Pro Ala Arg Leu Phe Ser
   305                 310                 315
<210> 5
   <211> 315
   <212> PRT
   <213> sulfolobus acidocaldarius
<400> 5
   Met Thr Lys Ile Pro Leu Val Gly Lys Gly Glu Ile Ser Pro Gly Glu
   1               5                   10                  15
   Met Gly Phe Thr Leu Ile His Glu His Leu Arg Val Phe Ser Glu Pro
               20                  25                  30
   Val Arg Tyr Gln Trp Pro His Leu Tyr Asn Glu Asp Glu Glu Leu Lys
           35                  40                  45
   Asn Ala Val Asn Glu Val Lys Thr Ile Met Ser Tyr Gly Val Lys Thr
       50                  55                  60
   Ile Val Asp Pro Thr Val Met Gly Leu Gly Arg Asp Ile Arg Phe Ser
   65                  70                  75                  80
   Glu Lys Val Val Lys Glu Thr Gly Ile Asn Val Ile Ala Ala Thr Gly
                   85                  90                  95
   Leu Tyr Thr Tyr Thr Asp Leu Pro Phe Phe Phe Asn Gly Arg Ser Leu
               100                 105                 110
   Glu Glu Ile Ala Glu Leu Leu Ile His Asp Ile Lys Lys Gly Ile Gln
           115                 120                 125
   Gly Thr Asn Asn Arg Ala Gly Phe Ile Lys Val Ala Ala Asp Glu Pro
       130                 135                 140
   Gly Ile Thr Arg Asp Val Glu Arg Ala Ile Arg Ala Ala Ala Ile Ala
   145                 150                 155                 160
   Gln Lys Glu Thr Asn Val Pro Ile Ile Thr His Ser Asn Ala His Asn
                   165                 170                 175
   Gly Thr Gly Leu Glu Gln Gln Arg Ile Leu Met Glu Glu Gly Val Asp
               180                 185                 190
   Pro Gly Arg Val Leu Ile Gly His Leu Gly Asp Thr Asp Asn Val Asp
           195                 200                 205
   Tyr Ile Lys Lys Ile Ala Asp Lys Gly Ser Phe Val Gly Leu Asp Arg
       210                 215                 220
   Tyr Gly Leu Asp Leu Phe Leu Pro Ile Asp Lys Arg Asn Glu Val Leu
   225                 230                 235                 240
   Leu Lys Leu Ile Lys Asp Gly Tyr Leu Asp Arg Ile Met Val Ser Gln
   245 250 255
   Asp Tyr Cys Cys Thr Ile Asp Trp Gly Ile Ala Lys Pro Glu Tyr Lys
   260 265 270
   Pro Lys Leu Ala Pro Lys Trp Ser Met Ser Leu Ile Phe Thr Asp Val
   275 280 285
   Ile Pro Ser Ile Lys Arg Ala Gly Val Thr Asp Glu Gln Leu His Val
   290 295 300
   Ile Phe Val Lys Asn Pro Ala Arg Leu Phe Ser
   305 310 315
<210> 6
   <211> 942
   <212> DNA
   <213> sulfolobus solfataricus
<220>
   <221> CDS
   <222> (1)..(942)
<400> 6
   atg aga ata cca tta gtt ggg aaa gat tca ata gaa tct aag gac ata       48
   Met Arg Ile Pro Leu Val Gly Lys Asp Ser Ile Glu Ser Lys Asp Ile
   1               5                   10                  15
   gga ttt acg cta att cat gaa cat tta aga gtt ttt agc gaa gcg gcc       96
   Gly Phe Thr Leu Ile His Glu His Leu Arg Val Phe Ser Glu Ala Ala
               20                  25                  30
   aga caa caa tgg ccc cat cta tat aac gaa gat gag gag ttc aga aac      144
   Arg Gln Gln Trp Pro His Leu Tyr Asn Glu Asp Glu Glu Phe Arg Asn
           35                  40                  45
   gct gta aat gag gtt aaa agg gca atg caa ttt gga gta aag act ata      192
   Ala Val Asn Glu Val Lys Arg Ala Met Gln Phe Gly Val Lys Thr Ile
       50                  55                  60
   gta gat ccc act gta atg gga ttg ggt agg gac atc aga ttt atg gaa      240
   Val Asp Pro Thr Val Met Gly Leu Gly Arg Asp Ile Arg Phe Met Glu
   65                  70                  75                  80
   aaa gtg gtt aag gct acc ggg ata aat tta gtt gcg ggg acg ggg att      288
   Lys Val Val Lys Ala Thr Gly Ile Asn Leu Val Ala Gly Thr Gly Ile
                   85                  90                  95
   tgg ata ttc atc gac tta cct ttc tat ttc tta aat agg tca att gat      336
   Trp Ile Phe Ile Asp Leu Pro Phe Tyr Phe Leu Asn Arg Ser Ile Asp
               100                 105                 110
   gag ata gct gac ttg ttt att cat gat ata aaa gag gga ata caa ggt      384
   Glu Ile Ala Asp Leu Phe Ile His Asp Ile Lys Glu Gly Ile Gln Gly
           115                 120                 125
   act ctc aat aaa gct ggc ttc gta aag ata gct gca gat gaa cct ggg      432
   Thr Leu Asn Lys Ala Gly Phe Val Lys Ile Ala Ala Asp Glu Pro Gly
       130                 135                 140
   atc aca aag gat gtg gag aag gta ata agg gct gct gcc ata gca aac      480
   Ile Thr Lys Asp Val Glu Lys Val Ile Arg Ala Ala Ala Ile Ala Asn
   145                 150                 155                 160
   aaa gag act aaa gta cca ata att acc cac tct aac gct cac aat aac      528
   Lys Glu Thr Lys Val Pro Ile Ile Thr His Ser Asn Ala His Asn Asn
                   165                 170                 175
   acc gga tta gaa cag caa aga ata ttg act gaa gaa ggt gtt gat cca      576
   Thr Gly Leu Glu Gln Gln Arg Ile Leu Thr Glu Glu Gly Val Asp Pro
               180                 185                 190
   ggg aaa ata tta ata ggt cat tta ggt gat aca gat aat ata gat tac      624
   Gly Lys Ile Leu Ile Gly His Leu Gly Asp Thr Asp Asn Ile Asp Tyr
           195                 200                 205
   ata aag aag ata gca gat aag gga tcc ttt att gga tta gat cat tat      672
   Ile Lys Lys Ile Ala Asp Lys Gly Ser Phe Ile Gly Leu Asp His Tyr
       210                 215                 220
   ggt tta gat tta ttc cta cct gtt gat aag aga aat gaa acg acc tta      720
   Gly Leu Asp Leu Phe Leu Pro Val Asp Lys Arg Asn Glu Thr Thr Leu
   225                 230                 235                 240
   aga cta atc aaa gat ggt tat tca gat aag ata atg atc tct cac gat      768
   Arg Leu Ile Lys Asp Gly Tyr Ser Asp Lys Ile Met Ile Ser His Asp
                   245                 250                 255
   tat tta tgc aca atc gac tgg gga act gca aaa cca gaa tat aaa cct      816
   Tyr Leu Cys Thr Ile Asp Trp Gly Thr Ala Lys Pro Glu Tyr Lys Pro
               260                 265                 270
   aag ctt gct cca aga tgg agt ata act cta ata ttt gag gat acg ata      864
   Lys Leu Ala Pro Arg Trp Ser Ile Thr Leu Ile Phe Glu Asp Thr Ile
           275                 280                 285
   ccg ttc tta aag aga aat gga gtg aat gaa gag gtt ata gct aca ata      912
   Pro Phe Leu Lys Arg Asn Gly Val Asn Glu Glu Val Ile Ala Thr Ile
       290                 295                 300
   ttt aag gaa aat ccg aaa aag ttc ttc agc                              942
   Phe Lys Glu Asn Pro Lys Lys Phe Phe Ser
   305                 310
<210> 7
   <211> 314
   <212> PRT
   <213> Sulfolobus solfataricus
<400> 7
   Met Arg Ile Pro Leu Val Gly Lys Asp Ser Ile Glu Ser Lys Asp Ile
   1               5                   10                  15
   Gly Phe Thr Leu Ile His Glu His Leu Arg Val Phe Ser Glu Ala Ala
               20                  25                  30
   Arg Gln Gln Trp Pro His Leu Tyr Asn Glu Asp Glu Glu Phe Arg Asn
           35                  40                  45
   Ala Val Asn Glu Val Lys Arg Ala Met Gln Phe Gly Val Lys Thr Ile
       50                  55                  60
   Val Asp Pro Thr Val Met Gly Leu Gly Arg Asp Ile Arg Phe Met Glu
   65                  70                  75                  80
   Lys Val Val Lys Ala Thr Gly Ile Asn Leu Val Ala Gly Thr Gly Ile
   85 90 95
   Trp Ile Phe Ile Asp Leu Pro Phe Tyr Phe Leu Asn Arg Ser Ile Asp
   100 105 110
   Glu Ile Ala Asp Leu Phe Ile His Asp Ile Lys Glu Gly Ile Gln Gly
   115 120 125
   Thr Leu Asn Lys Ala Gly Phe Val Lys Ile Ala Ala Asp Glu Pro Gly
   130 135 140
   Ile Thr Lys Asp Val Glu Lys Val Ile Arg Ala Ala Ala Ile Ala Asn
   145 150 155 160
   Lys Glu Thr Lys Val Pro Ile Ile Thr His Ser Asn Ala His Asn Asn
   165 170 175
   Thr Gly Leu Glu Gln Gln Arg Ile Leu Thr Glu Glu Gly Val Asp Pro
   180 185 190
   Gly Lys Ile Leu Ile Gly His Leu Gly Asp Thr Asp Asn Ile Asp Tyr
   195 200 205
   Ile Lys Lys Ile Ala Asp Lys Gly Ser Phe Ile Gly Leu Asp His Tyr
   210 215 220
   Gly Leu Asp Leu Phe Leu Pro Val Asp Lys Arg Asn Glu Thr Thr Leu
   225 230 235 240
   Arg Leu Ile Lys Asp Gly Tyr Ser Asp Lys Ile Met Ile Ser His Asp
   245 250 255
   Tyr Leu Cys Thr Ile Asp Trp Gly Thr Ala Lys Pro Glu Tyr Lys Pro
   260 265 270
   Lys Leu Ala Pro Arg Trp Ser Ile Thr Leu Ile Phe Glu Asp Thr Ile
   275 280 285
   Pro Phe Leu Lys Arg Asn Gly Val Asn Glu Glu Val Ile Ala Thr Ile
   290 295 300
   Phe Lys Glu Asn Pro Lys Lys Phe Phe Ser
   305 310
<210> 8
   <211> 942
   <212> DNA
   <213> Sulfolobus solfataricus
<220>
   <221> CDS
   <222> (1)..(942)
<400> 8
   atg aga ata cca tta gtt ggg aaa gat tca ata gaa tct aag gac ata    48
   Met Arg Ile Pro Leu Val Gly Lys Asp Ser Ile Glu Ser Lys Asp Ile
   1               5                   10                  15
   gga ttt acg cta att cat gaa cat tta aga gca ttt agc gaa gcg gcc    96
   Gly Phe Thr Leu Ile His Glu His Leu Arg Ala Phe Ser Glu Ala Ala
               20                  25                  30
   aga caa caa tgg ccc cat cta tat aac gaa gat gag gag ttc aga aac    144
   Arg Gln Gln Trp Pro His Leu Tyr Asn Glu Asp Glu Glu Phe Arg Asn
           35                  40                  45
   gct gta aat gag gtt aaa agg gca atg caa ttt gga gta aag act ata    192
   Ala Val Asn Glu Val Lys Arg Ala Met Gln Phe Gly Val Lys Thr Ile
       50                  55                  60
   gta gat ccc act gta atg gga ata ggt agg gac atc aga ttt atg gaa    240
   Val Asp Pro Thr Val Met Gly Ile Gly Arg Asp Ile Arg Phe Met Glu
   65                  70                  75                  80
   aaa gtg gtt aag gct acc ggg ata aat tta gtt gcg ggg acg ggg att    288
   Lys Val Val Lys Ala Thr Gly Ile Asn Leu Val Ala Gly Thr Gly Ile
                   85                  90                  95
   tgg ata ttc atc gac tta cct ttc tat ttc tta aat agg tca att gat    336
   Trp Ile Phe Ile Asp Leu Pro Phe Tyr Phe Leu Asn Arg Ser Ile Asp
               100                 105                 110
   gag ata gct gac ttg ttt att cat gat ata aaa gag gga ata caa ggt    384
   Glu Ile Ala Asp Leu Phe Ile His Asp Ile Lys Glu Gly Ile Gln Gly
           115                 120                 125
   act ctc aat aaa gct ggc ttc gta aag ata gct gca aca gaa cct ggg    432
   Thr Leu Asn Lys Ala Gly Phe Val Lys Ile Ala Ala Thr Glu Pro Gly
       130                 135                 140
   atc aca aag gat gtg gag aag gta ata agg gct gct gcc ata gca aac    480
   Ile Thr Lys Asp Val Glu Lys Val Ile Arg Ala Ala Ala Ile Ala Asn
   145                 150                 155                 160
   aaa gag act aaa gta cca ata att acc cac tct aac gct cac aat aac    528
   Lys Glu Thr Lys Val Pro Ile Ile Thr His Ser Asn Ala His Asn Asn
                   165                 170                 175
   acc gga tta gaa cag caa aga ata ttg act gaa gaa ggt gtt gat cca    576
   Thr Gly Leu Glu Gln Gln Arg Ile Leu Thr Glu Glu Gly Val Asp Pro
               180                 185                 190
   ggg aaa ata tta ata ggt cat tta ggt gat aca gat aat ata gat tac    624
   Gly Lys Ile Leu Ile Gly His Leu Gly Asp Thr Asp Asn Ile Asp Tyr
           195                 200                 205
   ata aag aag ata gca gat aag gga tcc ttt att gga tta gat cat tat    672
   Ile Lys Lys Ile Ala Asp Lys Gly Ser Phe Ile Gly Leu Asp His Tyr
       210                 215                 220
   ccg cat gat tta ttc cta cct gtt gat aag aga aat gaa acg acc tta    720
   Pro His Asp Leu Phe Leu Pro Val Asp Lys Arg Asn Glu Thr Thr Leu
   225                 230                 235                 240
   aga cta atc aaa gat ggt tat tca gat aag ata atg atc tct cac gat    768
   Arg Leu Ile Lys Asp Gly Tyr Ser Asp Lys Ile Met Ile Ser His Asp
                   245                 250                 255
   tat tta tgc aca atc gac tgg gga act gca aaa cca gaa tat aaa cct    816
   Tyr Leu Cys Thr Ile Asp Trp Gly Thr Ala Lys Pro Glu Tyr Lys Pro
               260                 265                 270
   aag ctt gct cca aga tgg agt ata act cta ata ttt gag gat acg ata        864
   Lys Leu Ala Pro Arg Trp Ser Ile Thr Leu Ile Phe Glu Asp Thr Ile
           275                 280                 285
   ccg ttc tta aag aga aat gga gtg aat gaa gag gtt ata gct aca ata        912
   Pro Phe Leu Lys Arg Asn Gly Val Asn Glu Glu Val Ile Ala Thr Ile
       290                 295                 300
   ttt aag gaa aat ccg aaa aag ttc ttc agc                                942
   Phe Lys Glu Asn Pro Lys Lys Phe Phe Ser
   305                 310
<210> 9
   <211> 314
   <212> PRT
   <213> Sulfolobus solfataricus
<400> 9
   Met Arg Ile Pro Leu Val Gly Lys Asp Ser Ile Glu Ser Lys Asp Ile
   1               5                   10                  15
   Gly Phe Thr Leu Ile His Glu His Leu Arg Ala Phe Ser Glu Ala Ala
               20                  25                  30
   Arg Gln Gln Trp Pro His Leu Tyr Asn Glu Asp Glu Glu Phe Arg Asn
           35                  40                  45
   Ala Val Asn Glu Val Lys Arg Ala Met Gln Phe Gly Val Lys Thr Ile
       50                  55                  60
   Val Asp Pro Thr Val Met Gly Ile Gly Arg Asp Ile Arg Phe Met Glu
   65                  70                  75                  80
   Lys Val Val Lys Ala Thr Gly Ile Asn Leu Val Ala Gly Thr Gly Ile
                   85                  90                  95
   Trp Ile Phe Ile Asp Leu Pro Phe Tyr Phe Leu Asn Arg Ser Ile Asp
               100                 105                 110
   Glu Ile Ala Asp Leu Phe Ile His Asp Ile Lys Glu Gly Ile Gln Gly
           115                 120                 125
   Thr Leu Asn Lys Ala Gly Phe Val Lys Ile Ala Ala Thr Glu Pro Gly
       130                 135                 140
   Ile Thr Lys Asp Val Glu Lys Val Ile Arg Ala Ala Ala Ile Ala Asn
   145                 150                 155                 160
   Lys Glu Thr Lys Val Pro Ile Ile Thr His Ser Asn Ala His Asn Asn
                   165                 170                 175
   Thr Gly Leu Glu Gln Gln Arg Ile Leu Thr Glu Glu Gly Val Asp Pro
               180                 185                 190
   Gly Lys Ile Leu Ile Gly His Leu Gly Asp Thr Asp Asn Ile Asp Tyr
           195                 200                 205
   Ile Lys Lys Ile Ala Asp Lys Gly Ser Phe Ile Gly Leu Asp His Tyr
       210                 215                 220
   Pro His Asp Leu Phe Leu Pro Val Asp Lys Arg Asn Glu Thr Thr Leu
   225                 230                 235                 240
   Arg Leu Ile Lys Asp Gly Tyr Ser Asp Lys Ile Met Ile Ser His Asp
                   245                 250                 255
   Tyr Leu Cys Thr Ile Asp Trp Gly Thr Ala Lys Pro Glu Tyr Lys Pro
               260                 265                 270
   Lys Leu Ala Pro Arg Trp Ser Ile Thr Leu Ile Phe Glu Asp Thr Ile
           275                 280                 285
   Pro Phe Leu Lys Arg Asn Gly Val Asn Glu Glu Val Ile Ala Thr Ile
       290                 295                 300
   Phe Lys Glu Asn Pro Lys Lys Phe Phe Ser
   305                 310
<210> 10
   <211> 942
   <212> DNA
   <213> Sulfolobus solfataricus
<220>
   <221> CDS
   <222> (1)..(942)
<400> 10
   atg aga ata cca tta gtt ggg aaa gat tca ata gaa tct aag gac ata      48
   Met Arg Ile Pro Leu Val Gly Lys Asp Ser Ile Glu Ser Lys Asp Ile
   1               5                   10                  15
   gga ttt acg cta att cat gaa cat tta aga gca ttt agc gaa gcg gcc      96
   Gly Phe Thr Leu Ile His Glu His Leu Arg Ala Phe Ser Glu Ala Ala
               20                  25                  30
   aga caa caa tgg ccc cat cta tat aac gaa gat gag gag ttc aga aac     144
   Arg Gln Gln Trp Pro His Leu Tyr Asn Glu Asp Glu Glu Phe Arg Asn
           35                  40                  45
   gct gta aat gag gtt aaa agg gca atg caa ttt gga gta aag act ata     192
   Ala Val Asn Glu Val Lys Arg Ala Met Gln Phe Gly Val Lys Thr Ile
       50                  55                  60
   gta gat gtt agc gta atg gga ata ggt agg gac atc aga ttt atg gaa     240
   Val Asp Val Ser Val Met Gly Ile Gly Arg Asp Ile Arg Phe Met Glu
   65                  70                  75                  80
   aaa gtg gtt aag gct acc ggg ata aat tta gtt gcg ggg acg ggg att     288
   Lys Val Val Lys Ala Thr Gly Ile Asn Leu Val Ala Gly Thr Gly Ile
                   85                  90                  95
   tgg ata ttc atc gac tta cct ttc tat ttc tta aat agg tca att gat     336
   Trp Ile Phe Ile Asp Leu Pro Phe Tyr Phe Leu Asn Arg Ser Ile Asp
                   100                 105                 110
   gag ata gct gac ttg ttt att cat gat ata aaa gag gga ata caa ggt      384
   Glu Ile Ala Asp Leu Phe Ile His Asp Ile Lys Glu Gly Ile Gln Gly
           115                 120                 125
   act ctc aat aaa gct ggc ttc gta aag ata gct gca aca gaa cct ggg      432
   Thr Leu Asn Lys Ala Gly Phe Val Lys Ile Ala Ala Thr Glu Pro Gly
       130                 135                 140
   atc aca aag gat gtg gag aag gta ata agg gct gct gcc ata gca aac      480
   Ile Thr Lys Asp Val Glu Lys Val Ile Arg Ala Ala Ala Ile Ala Asn
   145                 150                 155                 160
   aaa gag act aaa gta cca ata att acc cac tct aac gct cac aat aac      528
   Lys Glu Thr Lys Val Pro Ile Ile Thr His Ser Asn Ala His Asn Asn
                   165                 170                 175
   acc gga tta gaa cag caa aga ata ttg act gaa gaa ggt gtt gat cca      576
   Thr Gly Leu Glu Gln Gln Arg Ile Leu Thr Glu Glu Gly Val Asp Pro
               180                 185                 190
   ggg aaa ata tta ata ggt cat tta ggt gat aca gat aat ata gat tac      624
   Gly Lys Ile Leu Ile Gly His Leu Gly Asp Thr Asp Asn Ile Asp Tyr
           195                 200                 205
   ata aag aag ata gca gat aag gga tcc ttt att gga tta gat cat tat      672
   Ile Lys Lys Ile Ala Asp Lys Gly Ser Phe Ile Gly Leu Asp His Tyr
       210                 215                 220
   ccg cat gat tta tca cta cct gtt gat aag aga aat gaa acg acc tta      720
   Pro His Asp Leu Ser Leu Pro Val Asp Lys Arg Asn Glu Thr Thr Leu
   225                 230                 235                 240
   aga cta atc aaa gat ggt tat tca gat aag ata atg atc tct cac gat      768
   Arg Leu Ile Lys Asp Gly Tyr Ser Asp Lys Ile Met Ile Ser His Asp
                   245                 250                 255
   tat tta tgc aca atc gac gca gga act gca aaa cca gaa tat aaa cct      816
   Tyr Leu Cys Thr Ile Asp Ala Gly Thr Ala Lys Pro Glu Tyr Lys Pro
               260                 265                 270
   aag ctt gct cca aga atc agt ata act cta ata ttt gag gat acg ata      864
   Lys Leu Ala Pro Arg Ile Ser Ile Thr Leu Ile Phe Glu Asp Thr Ile
           275                 280                 285
   ccg ttc tta aag aga aat gga gtg aat gaa gag gtt ata gct aca ata      912
   Pro Phe Leu Lys Arg Asn Gly Val Asn Glu Glu Val Ile Ala Thr Ile
       290                 295                 300
   ttt aag gaa aat ccg aaa aag ttc ttc agc                              942
   Phe Lys Glu Asn Pro Lys Lys Phe Phe Ser
   305                 310
<210> 11
   <211> 314
   <212> PRT
   <213> sulfolobus solfataricus
<400> 11
   Met Arg Ile Pro Leu Val Gly Lys Asp Ser Ile Glu Ser Lys Asp Ile
   1 5 10 15
   Gly Phe Thr Leu Ile His Glu His Leu Arg Ala Phe Ser Glu Ala Ala
   20 25 30
   Arg Gln Gln Trp Pro His Leu Tyr Asn Glu Asp Glu Glu Phe Arg Asn
   35 40 45
   Ala Val Asn Glu Val Lys Arg Ala Met Gln Phe Gly Val Lys Thr Ile
   50 55 60
   Val Asp Val Ser Val Met Gly Ile Gly Arg Asp Ile Arg Phe Met Glu
   65 70 75 80
   Lys Val Val Lys Ala Thr Gly Ile Asn Leu Val Ala Gly Thr Gly Ile
   85 90 95
   Trp Ile Phe Ile Asp Leu Pro Phe Tyr Phe Leu Asn Arg Ser Ile Asp
   100 105 110
   Glu Ile Ala Asp Leu Phe Ile His Asp Ile Lys Glu Gly Ile Gln Gly
   115 120 125
   Thr Leu Asn Lys Ala Gly Phe Val Lys Ile Ala Ala Thr Glu Pro Gly
   130 135 140
   Ile Thr Lys Asp Val Glu Lys Val Ile Arg Ala Ala Ala Ile Ala Asn
   145 150 155 160
   Lys Glu Thr Lys Val Pro Ile Ile Thr His Ser Asn Ala His Asn Asn
   165 170 175
   Thr Gly Leu Glu Gln Gln Arg Ile Leu Thr Glu Glu Gly Val Asp Pro
   180 185 190
   Gly Lys Ile Leu Ile Gly His Leu Gly Asp Thr Asp Asn Ile Asp Tyr
   195 200 205
   Ile Lys Lys Ile Ala Asp Lys Gly Ser Phe Ile Gly Leu Asp His Tyr
   210 215 220
   Pro His Asp Leu Ser Leu Pro Val Asp Lys Arg Asn Glu Thr Thr Leu
   225 230 235 240
   Arg Leu Ile Lys Asp Gly Tyr Ser Asp Lys Ile Met Ile Ser His Asp
   245 250 255
   Tyr Leu Cys Thr Ile Asp Ala Gly Thr Ala Lys Pro Glu Tyr Lys Pro
   260 265 270
   Lys Leu Ala Pro Arg Ile Ser Ile Thr Leu Ile Phe Glu Asp Thr Ile
   275 280 285
   Pro Phe Leu Lys Arg Asn Gly Val Asn Glu Glu Val Ile Ala Thr Ile
   290 295 300
   Phe Lys Glu Asn Pro Lys Lys Phe Phe Ser
   305 310
<210> 12
   <211> 945
   <212> DNA
   <213> Sulfolobus acidocaldarius
<220>
   <221> CDS
   <222> (1)..(945)
<400> 12
   atg aca aaa att cct ctt gta gga aaa ggt gaa ata tca cct gga gaa 48
   Met Thr Lys Ile Pro Leu Val Gly Lys Gly Glu Ile Ser Pro Gly Glu
   1 5 10 15
   atg ggt ttt act tta ata cat gag cat tta agg gtc ttc agt gaa cca 96
   Met Gly Phe Thr Leu Ile His Glu His Leu Arg Val Phe Ser Glu Pro
   20 25 30
   gtt aga tat caa tgg cca cat ctt tat aat gaa gat gag gag tta aaa 144
   Val Arg Tyr Gln Trp Pro His Leu Tyr Asn Glu Asp Glu Glu Leu Lys
   35 40 45
   aat gca gta aat gaa gta aag aca ata atg tca tat ggt gtt aag acc 192
   Asn Ala Val Asn Glu Val Lys Thr Ile Met Ser Tyr Gly Val Lys Thr
   50 55 60
   atc gtg gat ccc act gtc atg ggt tta ggg aga gac att aga ttc agt 240
   Ile Val Asp Pro Thr Val Met Gly Leu Gly Arg Asp Ile Arg Phe Ser
   65 70 75 80
   gag aag gtc gtg aaa gaa aca ggt ata aat gtg att gca gca acg ggg 288
   Glu Lys Val Val Lys Glu Thr Gly Ile Asn Val Ile Ala Ala Thr Gly
   85 90 95
   ttg tgg act ttt act gat tta cct ttc ttc ttc aat gga aga tca ttg 336
   Leu Trp Thr Phe Thr Asp Leu Pro Phe Phe Phe Asn Gly Arg Ser Leu
   100 105 110
   gaa gag att gca gaa tta tta ata cat gat ata aaa aag gga ata caa 384
   Glu Glu Ile Ala Glu Leu Leu Ile His Asp Ile Lys Lys Gly Ile Gln
   115 120 125
   ggg aca aat aat aga gca ggc ttc att aag gtt gca gca gat gag cca 432
   Gly Thr Asn Asn Arg Ala Gly Phe Ile Lys Val Ala Ala Asp Glu Pro
   130 135 140
   ggt ata acg agg gat gta gag agg gca ata agg gca gct gct ata gct 480
   Gly Ile Thr Arg Asp Val Glu Arg Ala Ile Arg Ala Ala Ala Ile Ala
   145 150 155 160
   cag aag gag act aac gta ccc ata ata aca cat tca aat gct cat aac 528
   Gln Lys Glu Thr Asn Val Pro Ile Ile Thr His Ser Asn Ala His Asn
   165 170 175
   ggg aca ggt ctt gag caa caa agg att cta atg gag gag ggt gta gac 576
   Gly Thr Gly Leu Glu Gln Gln Arg Ile Leu Met Glu Glu Gly Val Asp
   180 185 190
   cca ggg aga gtg cta ata ggt cac ttg ggg gac act gat aac gtg gat 624
   Pro Gly Arg Val Leu Ile Gly His Leu Gly Asp Thr Asp Asn Val Asp
   195 200 205
   tac ata aag aag ata gcc gat aaa ggc tcg ttt gta ggt cta gat cat 672
   Tyr Ile Lys Lys Ile Ala Asp Lys Gly Ser Phe Val Gly Leu Asp His
               210                     215                     220
   tac ggt cta gat cta ttc tta cct ata gat aaa agg aac gag gtg ttg      720
   Tyr Gly Leu Asp Leu Phe Leu Pro Ile Asp Lys Arg Asn Glu Val Leu
   225                     230                     235                 240
   ttg aaa tta att aaa gat gga tac ttg gac agg att atg gtg tca caa      768
   Leu Lys Leu Ile Lys Asp Gly Tyr Leu Asp Arg Ile Met Val Ser Gln
                   245                     250                     255
   gat tac tta tgc aca att gac tgg ggg ata gca aag ccg gag tac aaa      816
   Asp Tyr Leu Cys Thr Ile Asp Trp Gly Ile Ala Lys Pro Glu Tyr Lys
               260                     265                     270
   cct aaa cta gct cca aaa tgg agt atg agt tta ata ttt aca gac gtt      864
   Pro Lys Leu Ala Pro Lys Trp Ser Met Ser Leu Ile Phe Thr Asp Val
           275                     280                     285
   ata ccc tca att aag aga gca gga gta act gat gag cag ttg cat gta      912
   Ile Pro Ser Ile Lys Arg Ala Gly Val Thr Asp Glu Gln Leu His Val
       290                     295                     300
   atc ttc gta aag aat cca gct aga cta ttt agt                          945
   Ile Phe Val Lys Asn Pro Ala Arg Leu Phe Ser
   305                     310                     315
<210> 13
   <211> 315
   <212> PRT
   <213> sulfolobus acidocaldarius
<400> 13
   Met Thr Lys Ile Pro Leu Val Gly Lys Gly Glu Ile Ser Pro Gly Glu
   1               5                   10                  15
   Met Gly Phe Thr Leu Ile His Glu His Leu Arg Val Phe Ser Glu Pro
               20                  25                  30
   Val Arg Tyr Gln Trp Pro His Leu Tyr Asn Glu Asp Glu Glu Leu Lys
           35                  40                  45
   Asn Ala Val Asn Glu Val Lys Thr Ile Met Ser Tyr Gly Val Lys Thr
       50                  55                  60
   Ile Val Asp Pro Thr Val Met Gly Leu Gly Arg Asp Ile Arg Phe Ser
   65                  70                  75                  80
   Glu Lys Val Val Lys Glu Thr Gly Ile Asn Val Ile Ala Ala Thr Gly
                   85                  90                  95
   Leu Trp Thr Phe Thr Asp Leu Pro Phe Phe Phe Asn Gly Arg Ser Leu
               100                 105                 110
   Glu Glu Ile Ala Glu Leu Leu Ile His Asp Ile Lys Lys Gly Ile Gln
           115                 120                 125
   Gly Thr Asn Asn Arg Ala Gly Phe Ile Lys Val Ala Ala Asp Glu Pro
       130                 135                 140
   Gly Ile Thr Arg Asp Val Glu Arg Ala Ile Arg Ala Ala Ala Ile Ala
   145 150 155 160
   Gln Lys Glu Thr Asn Val Pro Ile Ile Thr His Ser Asn Ala His Asn
   165 170 175
   Gly Thr Gly Leu Glu Gln Gln Arg Ile Leu Met Glu Glu Gly Val Asp
   180 185 190
   Pro Gly Arg Val Leu Ile Gly His Leu Gly Asp Thr Asp Asn Val Asp
   195 200 205
   Tyr Ile Lys Lys Ile Ala Asp Lys Gly Ser Phe Val Gly Leu Asp His
   210 215 220
   Tyr Gly Leu Asp Leu Phe Leu Pro Ile Asp Lys Arg Asn Glu Val Leu
   225 230 235 240
   Leu Lys Leu Ile Lys Asp Gly Tyr Leu Asp Arg Ile Met Val Ser Gln
   245 250 255
   Asp Tyr Leu Cys Thr Ile Asp Trp Gly Ile Ala Lys Pro Glu Tyr Lys
   260 265 270
   Pro Lys Leu Ala Pro Lys Trp Ser Met Ser Leu Ile Phe Thr Asp Val
   275 280 285
   Ile Pro Ser Ile Lys Arg Ala Gly Val Thr Asp Glu Gln Leu His Val
   290 295 300
   Ile Phe Val Lys Asn Pro Ala Arg Leu Phe Ser
   305 310 315
<210> 14
   <211> 945
   <212> DNA
   <213> sulfolobus acidocaldarius
<220>
   <221> CDS
   <222> (1)..(945)
<400> 14
   atg aca aaa att cct ctt gta gga aaa ggt gaa ata tca cct gga gaa      48
   Met Thr Lys Ile Pro Leu Val Gly Lys Gly Glu Ile Ser Pro Gly Glu
   1               5                   10                  15
   atg ggt ttt act tta ata cat gag cat tta agg gca ttc agt gaa cca      96
   Met Gly Phe Thr Leu Ile His Glu His Leu Arg Ala Phe Ser Glu Pro
               20                  25                  30
   gtt aga tat caa tgg cca cat ctt tat aat gaa gat gag gag tta aaa      144
   Val Arg Tyr Gln Trp Pro His Leu Tyr Asn Glu Asp Glu Glu Leu Lys
           35                  40                  45
   aat gca gta aat gaa gta aag aca ata atg tca tat ggt gtt aag acc      192
   Asn Ala Val Asn Glu Val Lys Thr Ile Met Ser Tyr Gly Val Lys Thr
       50                  55                  60
   atc gtg gat ccc act gtc atg ggt atc ggg aga gac att aga ttc agt      240
   Ile Val Asp Pro Thr Val Met Gly Ile Gly Arg Asp Ile Arg Phe Ser
   65                  70                  75                  80
   gag aag gtc gtg aaa gaa aca ggt ata aat gtg att gca gca acg ggg      288
   Glu Lys Val Val Lys Glu Thr Gly Ile Asn Val Ile Ala Ala Thr Gly
                   85                  90                  95
   ttg tgg act ttt act gat tta cct ttc ttc ttc aat gga aga tca ttg      336
   Leu Trp Thr Phe Thr Asp Leu Pro Phe Phe Phe Asn Gly Arg Ser Leu
               100                 105                 110
   gaa gag att gca gaa tta tta ata cat gat ata aaa aag gga ata caa      384
   Glu Glu Ile Ala Glu Leu Leu Ile His Asp Ile Lys Lys Gly Ile Gln
           115                 120                 125
   ggg aca aat aat aga gca ggc ttc att aag gtt gca gca aca gag cca      432
   Gly Thr Asn Asn Arg Ala Gly Phe Ile Lys Val Ala Ala Thr Glu Pro
       130                 135                 140
   ggt ata acg agg gat gta gag agg gca ata agg gca gct gct ata gct      480
   Gly Ile Thr Arg Asp Val Glu Arg Ala Ile Arg Ala Ala Ala Ile Ala
   145                 150                 155                 160
   cag aag gag act aac gta ccc ata ata aca cat tca aat gct cat aac      528
   Gln Lys Glu Thr Asn Val Pro Ile Ile Thr His Ser Asn Ala His Asn
                   165                 170                 175
   ggg aca ggt ctt gag caa caa agg att cta atg gag gag ggt gta gac      576
   Gly Thr Gly Leu Glu Gln Gln Arg Ile Leu Met Glu Glu Gly Val Asp
               180                 185                 190
   cca ggg aga gtg cta ata ggt cac ttg ggg gac act gat aac gtg gat      624
   Pro Gly Arg Val Leu Ile Gly His Leu Gly Asp Thr Asp Asn Val Asp
           195                 200                 205
   tac ata aag aag ata gcc gat aaa ggc tcg ttt gta ggt cta gat cat      672
   Tyr Ile Lys Lys Ile Ala Asp Lys Gly Ser Phe Val Gly Leu Asp His
       210                 215                 220
   tac cca cat gat cta ttc tta cct ata gat aaa agg aac gag gtg ttg      720
   Tyr Pro His Asp Leu Phe Leu Pro Ile Asp Lys Arg Asn Glu Val Leu
   225                 230                 235                 240
   ttg aaa tta att aaa gat gga tac ttg gac agg att atg gtg tca caa      768
   Leu Lys Leu Ile Lys Asp Gly Tyr Leu Asp Arg Ile Met Val Ser Gln
                   245                 250                 255
   gat tac tta tgc aca att gac tgg ggg ata gca aag ccg gag tac aaa      816
   Asp Tyr Leu Cys Thr Ile Asp Trp Gly Ile Ala Lys Pro Glu Tyr Lys
               260                 265                 270
   cct aaa cta gct cca aaa tgg agt atg agt tta ata ttt aca gac gtt      864
   Pro Lys Leu Ala Pro Lys Trp Ser Met Ser Leu Ile Phe Thr Asp Val
           275                 280                 285
   ata ccc tca att aag aga gca gga gta act gat gag cag ttg cat gta      912
   Ile Pro Ser Ile Lys Arg Ala Gly Val Thr Asp Glu Gln Leu His Val
       290                 295                 300
   atc ttc gta aag aat cca gct aga cta ttt agt                          945
   Ile Phe Val Lys Asn Pro Ala Arg Leu Phe Ser
   305                 310                 315
<210> 15
   <211> 315
   <212> PRT
   <213> sulfolobus acidocaldarius
<400> 15
   Met Thr Lys Ile Pro Leu Val Gly Lys Gly Glu Ile Ser Pro Gly Glu
   1               5                   10                  15
   Met Gly Phe Thr Leu Ile His Glu His Leu Arg Ala Phe Ser Glu Pro
               20                  25                  30
   Val Arg Tyr Gln Trp Pro His Leu Tyr Asn Glu Asp Glu Glu Leu Lys
           35                  40                  45
   Asn Ala Val Asn Glu Val Lys Thr Ile Met Ser Tyr Gly Val Lys Thr
       50                  55                  60
   Ile Val Asp Pro Thr Val Met Gly Ile Gly Arg Asp Ile Arg Phe Ser
   65                  70                  75                  80
   Glu Lys Val Val Lys Glu Thr Gly Ile Asn Val Ile Ala Ala Thr Gly
                   85                  90                  95
   Leu Trp Thr Phe Thr Asp Leu Pro Phe Phe Phe Asn Gly Arg Ser Leu
               100                 105                 110
   Glu Glu Ile Ala Glu Leu Leu Ile His Asp Ile Lys Lys Gly Ile Gln
           115                 120                 125
   Gly Thr Asn Asn Arg Ala Gly Phe Ile Lys Val Ala Ala Thr Glu Pro
       130                 135                 140
   Gly Ile Thr Arg Asp Val Glu Arg Ala Ile Arg Ala Ala Ala Ile Ala
   145                 150                 155                 160
   Gln Lys Glu Thr Asn Val Pro Ile Ile Thr His Ser Asn Ala His Asn
                   165                 170                 175
   Gly Thr Gly Leu Glu Gln Gln Arg Ile Leu Met Glu Glu Gly Val Asp
               180                 185                 190
   Pro Gly Arg Val Leu Ile Gly His Leu Gly Asp Thr Asp Asn Val Asp
           195                 200                 205
   Tyr Ile Lys Lys Ile Ala Asp Lys Gly Ser Phe Val Gly Leu Asp His
       210                 215                 220
   Tyr Pro His Asp Leu Phe Leu Pro Ile Asp Lys Arg Asn Glu Val Leu
   225                 230                 235                 240
   Leu Lys Leu Ile Lys Asp Gly Tyr Leu Asp Arg Ile Met Val Ser Gln
                   245                 250                 255
   Asp Tyr Leu Cys Thr Ile Asp Trp Gly Ile Ala Lys Pro Glu Tyr Lys
   260 265 270
   Pro Lys Leu Ala Pro Lys Trp Ser Met Ser Leu Ile Phe Thr Asp Val
   275 280 285
   Ile Pro Ser Ile Lys Arg Ala Gly Val Thr Asp Glu Gln Leu His Val
   290 295 300
   Ile Phe Val Lys Asn Pro Ala Arg Leu Phe Ser
   305 310 315
<210> 16
   <211> 945
   <212> DNA
   <213> Sulfolobus acidocaldarius
<220>
   <221> CDS
   <222> (1)..(945)
<400> 16
   atg aca aaa att cct ctt gta gga aaa ggt gaa ata tca cct gga gaa      48
   Met Thr Lys Ile Pro Leu Val Gly Lys Gly Glu Ile Ser Pro Gly Glu
   1               5                   10                  15
   atg ggt ttt act tta ata cat gag cat tta agg gca ttc agt gaa cca      96
   Met Gly Phe Thr Leu Ile His Glu His Leu Arg Ala Phe Ser Glu Pro
               20                  25                  30
   gtt aga tat caa tgg cca cat ctt tat aat gaa gat gag gag tta aaa      144
   Val Arg Tyr Gln Trp Pro His Leu Tyr Asn Glu Asp Glu Glu Leu Lys
           35                  40                  45
   aat gca gta aat gaa gta aag aca ata atg tca tat ggt gtt aag acc      192
   Asn Ala Val Asn Glu Val Lys Thr Ile Met Ser Tyr Gly Val Lys Thr
       50                  55                  60
   atc gtg gat gta tca gtc atg ggt atc ggg aga gac att aga ttc agt      240
   Ile Val Asp Val Ser Val Met Gly Ile Gly Arg Asp Ile Arg Phe Ser
   65                  70                  75                  80
   gag aag gtc gtg aaa gaa aca ggt ata aat gtg att gca gca acg ggg      288
   Glu Lys Val Val Lys Glu Thr Gly Ile Asn Val Ile Ala Ala Thr Gly
                   85                  90                  95
   ttg tgg act ttt act gat tta cct ttc ttc ttc aat gga aga tca ttg      336
   Leu Trp Thr Phe Thr Asp Leu Pro Phe Phe Phe Asn Gly Arg Ser Leu
               100                 105                 110
   gaa gag att gca gaa tta tta ata cat gat ata aaa aag gga ata caa      384
   Glu Glu Ile Ala Glu Leu Leu Ile His Asp Ile Lys Lys Gly Ile Gln
           115                 120                 125
   ggg aca aat aat aga gca ggc ttc att aag gtt gca gca aca gag cca      432
   Gly Thr Asn Asn Arg Ala Gly Phe Ile Lys Val Ala Ala Thr Glu Pro
       130                 135                 140
   ggt ata acg agg gat gta gag agg gca ata agg gca gct gct ata gct      480
   Gly Ile Thr Arg Asp Val Glu Arg Ala Ile Arg Ala Ala Ala Ile Ala
   145                 150                 155                 160
   cag aag gag act aac gta ccc ata ata aca cat tca aat gct cat aac      528
   Gln Lys Glu Thr Asn Val Pro Ile Ile Thr His Ser Asn Ala His Asn
                   165                 170                 175
   ggg aca ggt ctt gag caa caa agg att cta atg gag gag ggt gta gac      576
   Gly Thr Gly Leu Glu Gln Gln Arg Ile Leu Met Glu Glu Gly Val Asp
               180                 185                 190
   cca ggg aga gtg cta ata ggt cac ttg ggg gac act gat aac gtg gat      624
   Pro Gly Arg Val Leu Ile Gly His Leu Gly Asp Thr Asp Asn Val Asp
           195                 200                 205
   tac ata aag aag ata gcc gat aaa ggc tcg ttt gta ggt cta gat cat      672
   Tyr Ile Lys Lys Ile Ala Asp Lys Gly Ser Phe Val Gly Leu Asp His
       210                 215                 220
   tac cca cat gat cta tca tta cct ata gat aaa agg aac gag gtg ttg      720
   Tyr Pro His Asp Leu Ser Leu Pro Ile Asp Lys Arg Asn Glu Val Leu
   225                 230                 235                 240
   ttg aaa tta att aaa gat gga tac ttg gac agg att atg gtg tca caa      768
   Leu Lys Leu Ile Lys Asp Gly Tyr Leu Asp Arg Ile Met Val Ser Gln
                   245                 250                 255
   gat tac tta tgc aca att gac gca ggg ata gca aag ccg gag tac aaa      816
   Asp Tyr Leu Cys Thr Ile Asp Ala Gly Ile Ala Lys Pro Glu Tyr Lys
               260                 265                 270
   cct aaa cta gct cca aaa att agt atg agt tta ata ttt aca gac gtt      864
   Pro Lys Leu Ala Pro Lys Ile Ser Met Ser Leu Ile Phe Thr Asp Val
           275                 280                 285
   ata ccc tca att aag aga gca gga gta act gat gag cag ttg cat gta      912
   Ile Pro Ser Ile Lys Arg Ala Gly Val Thr Asp Glu Gln Leu His Val
       290                 295                 300
   atc ttc gta aag aat cca gct aga cta ttt agt                          945
   Ile Phe Val Lys Asn Pro Ala Arg Leu Phe Ser
   305                 310                 315
<210> 17
   <211> 315
   <212> PRT
   <213> sulfolobus acidocaldarius
<400> 17
   Met Thr Lys Ile Pro Leu Val Gly Lys Gly Glu Ile Ser Pro Gly Glu
   1               5                   10                  15
   Met Gly Phe Thr Leu Ile His Glu His Leu Arg Ala Phe Ser Glu Pro
               20                  25                  30
   Val Arg Tyr Gln Trp Pro His Leu Tyr Asn Glu Asp Glu Glu Leu Lys
           35                  40                  45
   Asn Ala Val Asn Glu Val Lys Thr Ile Met Ser Tyr Gly Val Lys Thr
       50                  55                  60
   Ile Val Asp Val Ser Val Met Gly Ile Gly Arg Asp Ile Arg Phe Ser
   65                  70                  75                  80
   Glu Lys Val Val Lys Glu Thr Gly Ile Asn Val Ile Ala Ala Thr Gly
                   85                  90                  95
   Leu Trp Thr Phe Thr Asp Leu Pro Phe Phe Phe Asn Gly Arg Ser Leu
   100 105 110
   Glu Glu Ile Ala Glu Leu Leu Ile His Asp Ile Lys Lys Gly Ile Gln
   115 120 125
   Gly Thr Asn Asn Arg Ala Gly Phe Ile Lys Val Ala Ala Thr Glu Pro
   130 135 140
   Gly Ile Thr Arg Asp Val Glu Arg Ala Ile Arg Ala Ala Ala Ile Ala
   145 150 155 160
   Gln Lys Glu Thr Asn Val Pro Ile Ile Thr His Ser Asn Ala His Asn
   165 170 175
   Gly Thr Gly Leu Glu Gln Gln Arg Ile Leu Met Glu Glu Gly Val Asp
   180 185 190
   Pro Gly Arg Val Leu Ile Gly His Leu Gly Asp Thr Asp Asn Val Asp
   195 200 205
   Tyr Ile Lys Lys Ile Ala Asp Lys Gly Ser Phe Val Gly Leu Asp His
   210 215 220
   Tyr Pro His Asp Leu Ser Leu Pro Ile Asp Lys Arg Asn Glu Val Leu
   225 230 235 240
   Leu Lys Leu Ile Lys Asp Gly Tyr Leu Asp Arg Ile Met Val Ser Gln
   245 250 255
   Asp Tyr Leu Cys Thr Ile Asp Ala Gly Ile Ala Lys Pro Glu Tyr Lys
   260 265 270
   Pro Lys Leu Ala Pro Lys Ile Ser Met Ser Leu Ile Phe Thr Asp Val
   275 280 285
   Ile Pro Ser Ile Lys Arg Ala Gly Val Thr Asp Glu Gln Leu His Val
   290 295 300
   Ile Phe Val Lys Asn Pro Ala Arg Leu Phe Ser
   305 310 315
<210> 18
   <211> 102
   <212> DNA
   <213> Sulfolobus solfataricus
<400> 18
   gatatacata tgagaatacc attagttggg aaagattcaa tagaatctaa ggacatagga 60
   tttacgctaa ttcatgaaca tttaagagct tttagcgaag cg 102
<210> 19
   <211> 92
   <212> DNA
   <213> Sulfolobus solfataricus
<400> 19
   aacctcattt acagcgtttc tgaactcctc atcttcgtta tatagatggg ccattgttgt 60
   ctgaccgctt cgctaaaagc tcttaaatgt tc 92
<210> 20
   <211> 93
   <212> DNA
   <213> Sulfolobus solfataricus
<400> 20
   gagttcagaa acgctgtaaa tgaggttaaa agggcaatgc aatttggagt aaagactata 60
   gtagatgtct ctgtaatggg aattggtagg gac 93
<210> 21
   <211> 93
   <212> DNA
   <213> Sulfolobus solfataricus
<400> 21
   cgtccccgca actaaattta tcccggtagc cttaaccact ttttccataa atctgatgtc 60
   cctaccaatt cccattacag agacatctac tat 93
<210> 22
   <211> 93
   <212> DNA
   <213> Sulfolobus solfataricus
<400> 22
   accgggataa atttagttgc ggggacgggg atttggatat ttatcgactt acctttctat 60
   ttcttaaata ggtcaattga tgagatagct gac 93
<210> 23
   <211> 93
   <212> DNA
   <213> Sulfolobus solfataricus
<400> 23
   tatctttacg aagccagctt tattgagagt accttgtatt ccctctttta tatcatgaat 60
   aaacaagtca gctatctcat caattgacct att 93
<210> 24
   <211> 93
   <212> DNA
   <213> Sulfolobus solfataricus
<400> 24
   ctcaataaag ctggcttcgt aaagatagct gcaactgaac ctgggatcac aaaggatgtg 60
   gagaaggtaa taagggctgc tgccatagca aac 93
<210> 25
   <211> 93
   <212> DNA
   <213> Sulfolobus solfataricus
<400> 25
   ttgctgttct aatccggtgt tattgtgagc gttagagtgg gtaattattg gtactttagt 60
   ctctttgttt gctatggcag cagcccttat tac 93
<210> 26
   <211> 93
   <212> DNA
   <213> Sulfolobus solfataricus
<400> 26
   cacaataaca ccggattaga acagcaaaga atattgactg aagaaggtgt tgatccaggg 60
   aaaatattaa taggtcattt aggtgataca gat 93
<210> 27
   <211> 90
   <212> DNA
   <213> Sulfolobus solfataricus
<400> 27
   aggaaaatga tctaatccaa taaaggatcc cttatctgct atcttcttta tgtaatctat 60
   attatctgta tcacctaaat gacctattaa 90
<210> 28
   <211> 99
   <212> DNA
   <213> sulfolobus solfataricus
<400> 28
   tcctttattg gattagatca ttttcctcat gatttatccc tacctgttga taagagaaat 60
   gaaacgacct taagactaat caaagatggt tattcagat 99
<210> 29
   <211> 93
   <212> DNA
   <213> sulfolobus solfataricus
<400> 29
   tttatattct ggttttgcag ttccagcgtc gaatgtgcat aaataatcgt gagagatcat 60
   tatcttatct gaataaccat ctttgattag tct 93
<210> 30
   <211> 93
   <212> DNA
   <213> Sulfolobus solfataricus
<400> 30
   gctggaactg caaaaccaga atataaacct aagcttgctc caagaattag tataactcta 60
   atatttgagg atacgatacc gttcttaaag aga 93
<210> 31
   <211> 109
   <212> DNA
   <213> Sulfolobus solfataricus
<400> 31
   caacccgaat tcttagctga agaacttttt cggattttcc ttaaatattg tagctataac 60
   ctcttcattc attcactcca tttctcttta agaacggtat cgtatcctc 109